# EUROPEAN PATENT APPLICATION

(11) **EP 4 406 973 A1**
(43) Date of publication of application: **31.07.2024**
(21) Application number: 23382072.9
(22) Date of filing: 27.01.2023
(51) Int. Cl.: C07K 16/28, A61K 39/00, A61K 47/68

(54) **ANTIBODIES AND USES THEREOF FOR THE TREATMENT OF INFECTIONS CAUSED BY ENVELOPED VIRUSES**

(71) Applicant: Fundació Privada Institut de Recerca de la SIDA-Caixa, 08916 Badalona (ES); Institució Catalana de Recerca i Estudis Avançats, 08010 Barcelona (ES)
(72) Inventor: MARTÍNEZ PICADO, Javier, E-08916 Badalona (Barcelona) (ES); RESA INFANTE, Patricia, E-08916 Badalona (Barcelona) (ES); ERQUICIA JAUREGUI, Itziar, E-08916 Badalona (Barcelona) (ES); IZQUIERDO USEROS, Nuria, E-08916 Badalona (Barcelona) (ES); CLOTET SALA, Bonaventura, E-08916 Badalona (Barcelona) (ES)
(74) Representative: ABG Intellectual Property Law, S.L.

(57) **Abstract**

The present invention relates to an antibody that specifically binds to sialic acid-binding Ig-like lectin 1 (CD169/Siglec-1), as well as the antigen-binding fragment, the kit and the pharmaceutical composition comprising said antibody. The invention also relates to the use of the antibody and the pharmaceutical composition in the prevention and/or the treatment of infection by enveloped virus and, in particular, by an infection by a virus of the filoviridae family, an infection by an Orthopneumovirus, a coronavirus infection or an arenavirus infection.

## Description

### FIELD OF THE INVENTION

The present invention is comprised within the field of biotechnology and biomedicine. It specifically relates to antibodies specific against sialic acid-binding Ig-like lectin 1 (CD169/Siglec-1), as well and the uses thereof in in the treatment and prevention of enveloped virus infections. Thus, the present invention relates to medical field, particularly, to the antiviral treatment and prevention.

### BACKGROUND OF THE INVENTION

Antigen presenting cells of myeloid lineage, such as monocytes, macrophages and dendritic cells (DCs), are essential in order to combat foreign pathogens and trigger antiviral responses. The sialic acid-binding Ig-like lectin 1 (CD169/Siglec-1) is a host membrane protein present on activated dendritic cells (DCs) and other myeloid cells such as monocytes and macrophages. Most enveloped virus contain sialylated gangliosides on their envelope as they result of their formation by budding from cellular membranes. The gangliosides present in the membrane of enveloped viruses are capable of interacting with CD169 thereby acting as attachment receptor of viruses such as the human immunodeficiency virus (HIV-1), the Ebola virus (EBOV) and the new coronavirus (SARS-CoV-2). Attachment leads to viral uptake and, ultimately leads to their dissemination throughout the host. T he outcome of these early interactions between DCs and these viruses is critical in determining host susceptibility, and may impact on progression of the infection-associated disease. Moreover, this interaction favors viral spread from the initial site of infection. Viral dispersion mediated by DCs is a common pathway co-opted by different types of viruses to evade immunity.

Accordingly, blocking the mechanisms of viral entry into DCs are potential therapeutic strategies applicable to prevent infection of certain enveloped viruses and therefore increase protection in exposed hosts.

Accordingly, there is a need in the art for agents which are capable of preventing the entry of pathogens into myeloid cells by way of the inhibition of the interaction of the CD169 membrane protein found in dendritic cells and the gangliosides present in the envelope of the viral particles.

### SUMMARY OF THE INVENTION

By means of a functional screening based on inhibition of cellular invasion assays, the inventors of the present invention have obtained antibodies which are capable of efficiently inhibiting the CD169/Siglec-1-mediated adhesion, migration and, particularly, invasive capacity of different virus. This new generation of antibodies could offer prophylaxis in forthcoming virus outbreaks, as restriction would be effective against all containing sialylated gangliosides on their envelope when they bud from cellular membranes.

In a view of the foregoing, in a first aspect the present invention relates to an antibody or an antigen-binding fragment thereof, wherein the antibody comprises one VH region and one VL region and wherein the antibody is selected from the group consisting of:
1) the 1 F5 antibody characterized in that:
   (i) the CDR1 of the VH region comprises the sequence of SEQ ID NO:1 or functionally equivalent variant thereof, the CDR2 of the VH region comprises the sequence of SEQ ID NO:2 or a functionally equivalent variant thereof and the CDR3 of the VH region comprises the sequence of SEQ ID NO: 3 or 4 or a functionally equivalent variant thereof, and
   (ii) the CDR1 of the VL region comprises the sequence of SEQ ID NO:5 or functionally equivalent variant thereof, the CDR2 of the VL region comprises the sequence XAS, wherein X Is Trp of Phe, or a functionally equivalent variant thereof and the CDR3 of the VL region comprises the sequence of SEQ ID NO: 8 or functionally equivalent variant thereof or a functionally equivalent variant thereof; and
2) the 3F1 antibody characterized in that:
   (i) the CDR1 of the VH region comprises the sequence of SEQ ID NO:40 or functionally equivalent variant thereof, the CDR2 of the VH region comprises the sequence of SEQ ID NO:41, the sequence of SEQ ID NO:42, the sequence of SEQ ID NO:43 or functionally equivalent variant thereof and the CDR3 of the VH region comprises the sequence of SEQ ID NO: 44, the sequence of SEQ ID NO:45 or functionally equivalent variants thereof, and
   (ii) the CDR1 of the VL region comprises the sequence of SEQ ID NO:46 or functionally equivalent variant thereof, the CDR2 of the VL region comprises the sequence XAS, wherein X Is Trp of Phe or a functionally equivalent variant thereof and the CDR3 of the VL region comprises the sequence of SEQ ID NO: 50 or functionally equivalent variants thereof, and
3) the 5B10 antibody comprising
   (i) the CDR1 of the VH region comprises the sequence of SEQ ID NO:88 or functionally equivalent variant thereof, the CDR2 of the VH region comprises the sequence of SEQ ID NO:89, and the CDR3 of the VH region comprises the sequence of SEQ ID NO: 90 or functionally equivalent variants thereof, and
   (ii) the CDR1 of the VL region comprises the sequence of SEQ ID NO:91, or a functionally equivalent variant thereof, the CDR2 of the VL region comprises the sequence FAS or a functionally equivalent variant thereof and the CDR3 of the VL region comprises the sequence of SEQ ID NO: 93 or a functionally equivalent variant thereof,
4) the 6G5 antibody comprising
   (i) the CDR1 of the VH region comprises the sequence of SEQ ID NO:116 or a functionally equivalent variant thereof, the CDR2 of the VH region comprises the sequence of SEQ ID NO:117 or a functionally equivalent variant thereof and the CDR3 of the VH region comprises the sequence of SEQ ID NO: 118 or a functionally equivalent variant thereof, and
   (ii) the CDR1 of the VL region comprises the sequence of SEQ ID NO:123, or a functionally equivalent variant thereof, the CDR2 of the VL region comprises the sequence of SEQ ID NO: 124 or a functionally equivalent variant thereof and the CDR3 of the VL region comprises the sequence of SEQ ID NO: 125 or a functionally equivalent variant thereof and
5) the 4E8 antibody is further characterized in that:
   (i) the CDR1 of the VH region comprises the sequence of SEQ ID NO:133 or a functionally equivalent variant thereof, the CDR2 of the VH region comprises the sequence of SEQ ID NO:134 or a functionally equivalent variant thereof and the CDR3 of the VH region comprises the sequence of SEQ ID NO: 135 or a functionally equivalent variant thereof, and
   (ii) the CDR1 of the VL region comprises the sequence of SEQ ID NO:140, or a functionally equivalent variant thereof, the CDR2 of the VL region comprises the sequence of SEQ ID NO: 141 or a functionally equivalent variant thereof and the CDR3 of the VL region comprises the sequence of SEQ ID NO: 142 or a functionally equivalent variant thereof.

In a second aspect, the invention relates to a nucleic acid encoding the antibody of the invention.

In a third aspect, the invention relates to an expression vector comprising the nucleic acid of the second aspect of the invention.

In a fourth aspect, the invention relates to a host cell comprising the nucleic acid of the second aspect of the invention or the vector of the third aspect of the invention.

In a fifth aspect, the invention relates to a pharmaceutical composition comprising any of the host cells of the fourth aspect of the invention, and/or an antibody or an antigen-binding fragment thereof according to the first aspect of the invention and at least one pharmaceutically acceptable excipient, carrier, or diluent.

In a sixth aspect, the invention relates to any of the host of the fourth aspect of the invention, antibody or an antigen-binding fragment of the first aspect of the invention, the nucleic acid of the second aspect of the invention, the expression vector of the third aspect of the invention and/or the host cell of the fourth aspect of the invention for use in medicine.

In a seventh aspect, the invention relates to any of the antibody or an antigen-binding fragment of the first aspect of the invention, the nucleic acid of the second aspect of the invention, the expression vector of the third aspect of the invention or the host cell of the fourth aspect of the invention for use in the prevention or in the treatment of an infection by an virus or as an antiviral.

In an eighth aspect, the invention relates to a conjugate comprising:
- an antibody or antigen-binding domain of the first aspect of the invention and
- an agent of interest,
wherein the agent of interest is covalently coupled to the antibody or to the antigen-binding fragment thereof.

In a ninth aspect, the invention relates to an in vitro method for the capture of particles containing a syalyllactose-containing ganglioside present in a sample which comprises:
(i) contacting the sample with a Siglec-1 protein variant that comprises the Siglec-1 V-set plus domain and three C2 Ig-like domains under conditions adequate for the binding of the particles containing a syalyllactose-containing ganglioside to the Siglec-1 protein variant and
(ii) recovering the Siglec-1 protein variants from the sample.

### DESCRIPTION OF THE FIGURES

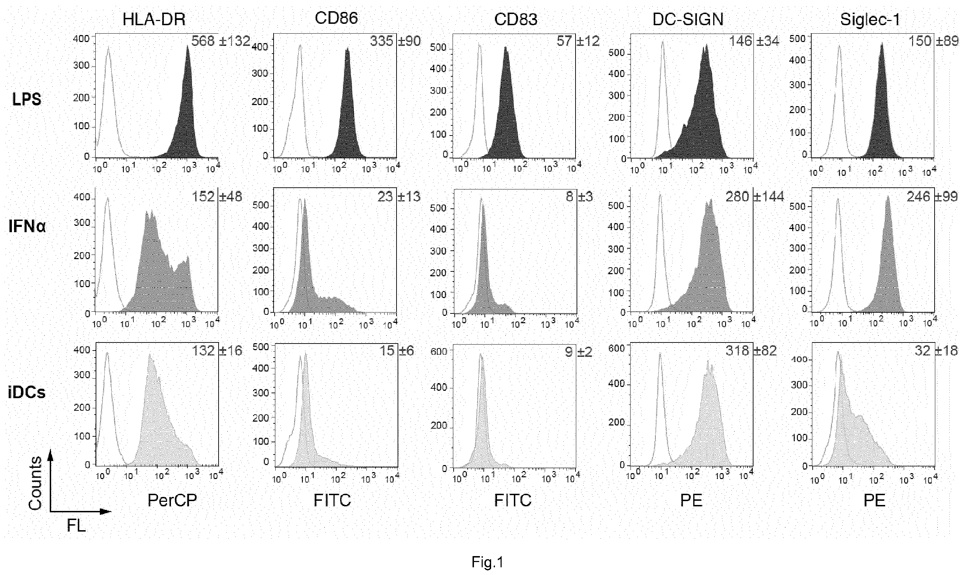

**Figure 1****.** LPS and IFNα treatment modulates HLA-DR, CD86, CD83, DC-SIGN, and Siglec-1 expression. Representative cell surface staining of distinct DC-markers analyzed by FACS. Numbers show the geometric mean fluorescence intensity and standard deviation of cells from 7 different donors and 5 experiments.

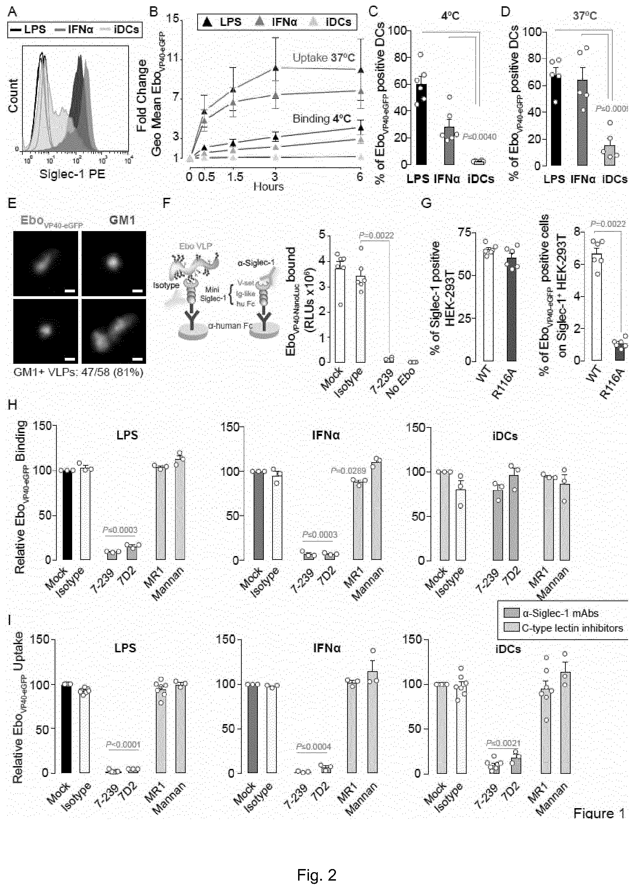

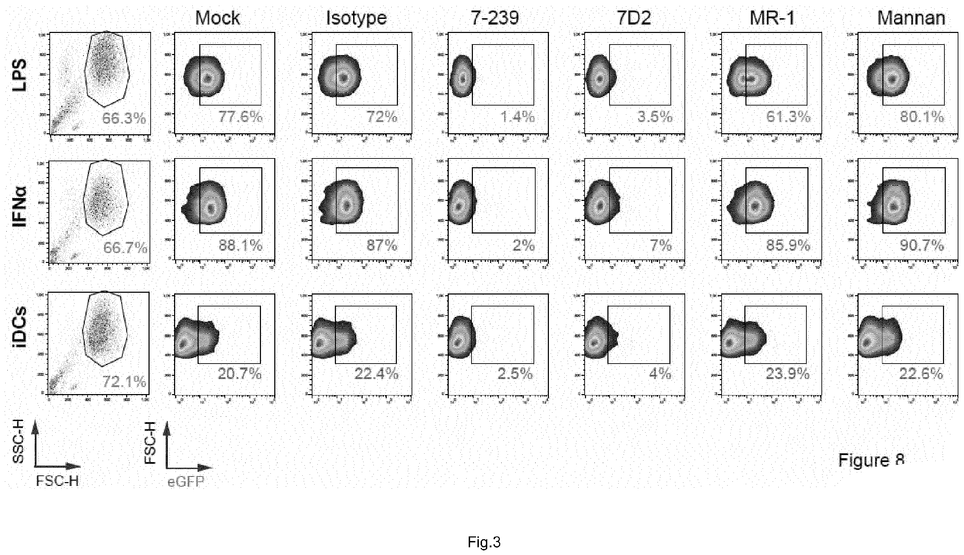

**Figure 2**.A ctivation of DCs enhances Ebola viral binding and uptake via Siglec-1 recognition of sialylated gangliosides. **A**. Representative cell surface staining of distinct DCs analyzed by FACS showing how LPS and IFN-α treatment up-regulates Siglec-1 expression. Other key markers are shown in Fig. 1. **B**.F old change in the geometric mean fluorescence intensity of distinct DCs pulsed with EboVP40-eGFP VLP at 4°C or 37°C for the indicated hours, washed and assessed by FACS, and compared to non-pulsed DCs. Data show mean values and SEM from one experiment including cells from 3 donors. **C.** Comparative DC binding at 4°C of EboVP40-eGFP VLP. DCs were pulsed for 6 h, washed and assessed by FACS. Data show mean values and SEM from two experiments including cells from 6 donors. **D**.C omparative DC uptake at 37°C of EboVP40-eGFP VLP as in C. Data show mean values and SEM from two experiments including cells from 5 donors. **E.** Confocal microscopy of EboVP40-eGFP VLPs stained with α-GM1 Abs (shown in red) and percentage of co-staining. **F.**S iglec-1-human Fc mini-proteins were left untreated or incubated with an isotype or an α-Siglec-1 mAb and then exposed to EboVP40-NanoLuc VLP. Binding of these VLPs to an ELISA plate coated with α-human Fc mAbs was detected via the attachment of the human Fc fragment of the mini-protein as illustrated, and then measured by NanoLuciferase activity in relative light units (RLUs). Graph show mean values and SEM from 2 experiments with triplicates. **G** (Left graph) Siglec-1 expression of HEK-293T cells transfected with a Siglec-1 plasmid or a R116A mutated plasmid that abrogates sialic acid recognition. (Right graph) EboVP40-eGFP VLP uptake by transfected cells that were labeled with an α-Siglec-1 PE mAb. Non-transfected cells were used as internal controls to subtract Siglec-1 independent viral uptake. **H.** Relative binding at 4°C of EboVP40-eGFP VLP by distinct DCs pre-incubated with α-Siglec-1 mAbs (orange bars), C-type lectin inhibitors (blue bars) or isotype control (yellow bars). For comparative purposes, values are normalized to the level of EboVP40-eGFP VLP uptake by mock-treated cells shown in panel B and set at 100% (dark bars). Mean values and SEM from one experiment include cells from 3 donors. **I**.R elative uptake at 37°C of EboVP40-eGFP VLP as in H.M ean values and SEM from 3 experiments include cells from 3-7 donors. Representative dot plots are shown in Fig. 3. Statistical differences in C and D were assessed with a paired t-test, in F and G with a Mann-Whitney test, and in H and I with a one-sample t-test.
**Figure 3**.R epresentative gating analysis for Ebola viral uptake. FACS analysis of EboVP40-eGFP VLP uptake by distinctly treated DCs from one donor pre-incubated with the indicated mAbs and inhibitors.

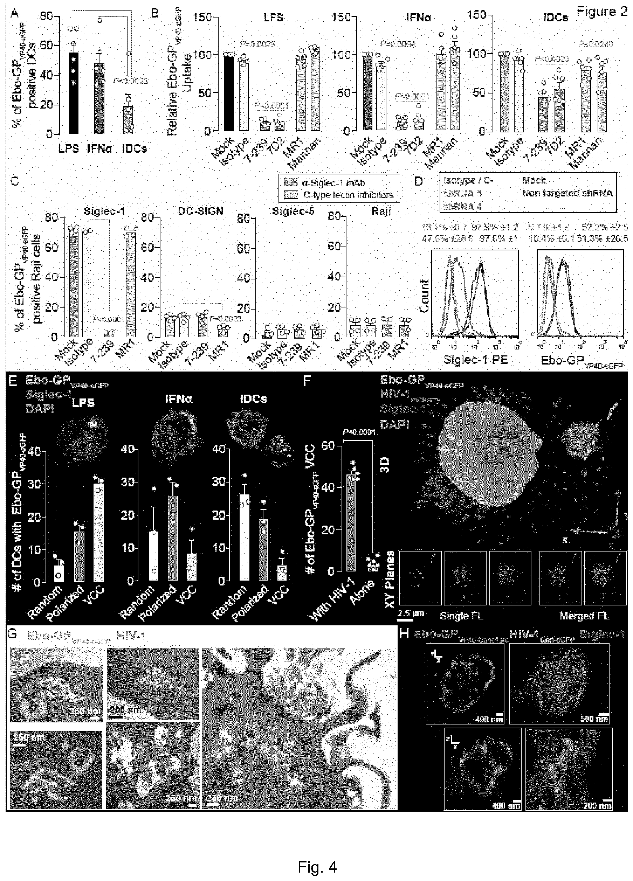

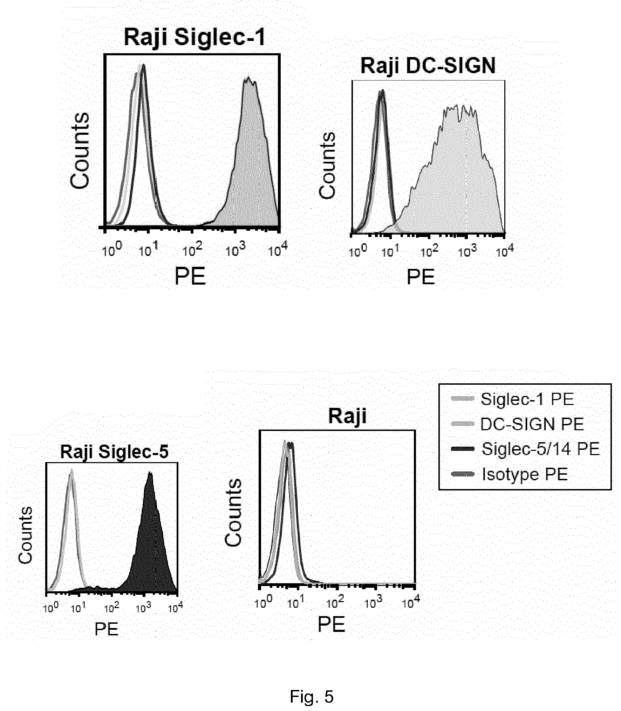

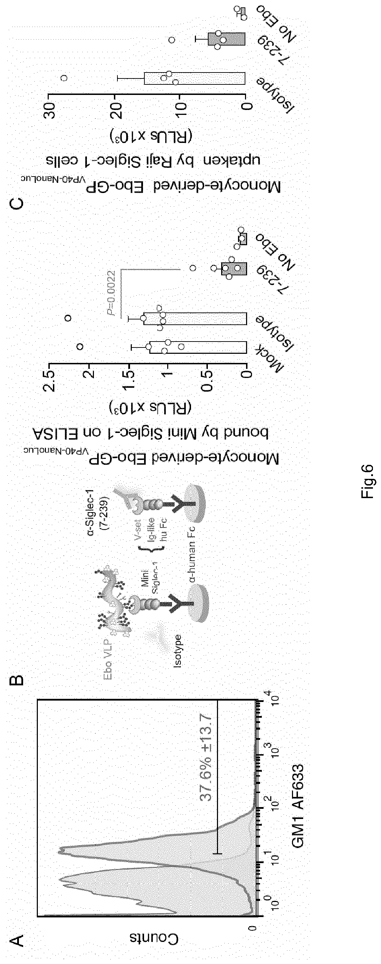

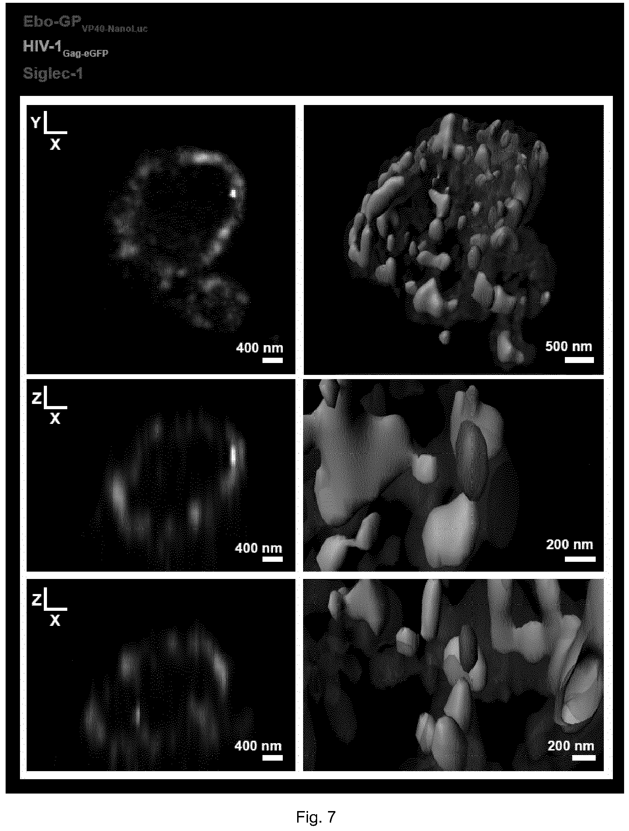

**Figure 4****.**S iglec-1 facilitates the uptake of Ebola viruses displaying envelope glycoproteins and stores these particles in the same virus-containing compartment as HIV-1. **A.** Comparative DC uptake of Ebo-GPVP40-eGFP VLP. DCs were pulsed for 3 h at 37°C, washed and assessed by FACS. Data show mean values and SEM of two experiments including cells from 6 donors. **B.**R elative uptake of Ebo-GPVP40-eGFP VLP by distinct DCs pre-incubated with the indicated mAbs and inhibitors. Values are normalized to the level of Ebo-GPVP40-eGFP VLP uptake by mock-treated cells shown in A and set at 100%. Mean values and SEM from two experiments include cells from 6 donors. **C.**C omparative uptake of Ebo-GPVP40-eGFP VLP for 3 h at 37°C by Raji B cells expressing the indicated receptors and pre-incubated with the indicated mAbs. Graphs show mean values and SEM from 2 experiments including 4 replicas. Staining of all receptors in each Raji cell line is shown in Fig. 5. Uptake of monocyte-derived Ebo-GPVP40-NanoLuc VLP by Raji Siglec-1 is shown in Fig. 6. **D.**R epresentative histograms show Siglec-1 expression (left) and Ebo-GPVP40-eGFP VLP uptake for 3 h (right) of LPS DCs transduced with Siglec-1 specific or non-targeted shRNAs. Numbers show mean % of positive cells and SD of cells from 2 donors. **E.** Confocal microscopy analysis of different DCs pulsed with Ebo-GPVP40-eGFP VLP for 4 h at 37°C and then stained with Siglec-1 PE (red) and DAPI (blue). (Images) Representative viral pattern for each kind of DC analyzed. Merge of z-planes showing maximum fluorescence intensity of the three channels. (Bar graphs) Number of DCs showing distinct viral patterns. Mean values of 50 cells from 3 different donors. **F.**C onfocal analysis of LPS DCs pulsed with Ebo-GPVP40-eGFP VLP and subsequently incubated with red fluorescent HIV-1mCherry and then stained with Siglec-1 Alexa-647 (blue) and DAPI (grey). (Bar graph) Number of Ebola virus-containing compartments (VCC) where HIV-1mCherry signal or no red signal was found along the eGFP signal. Mean values from 50 compartments in cells of 6 different donors and 2 experiments. Deconvolved reconstruction shows the fluorescence of the grey channel and maximum intensity of the other channels within a 3D volumetric x-y-z data field. XY planes show magnification of the VCC with single or merged fluorescence channels. **G.** Electron micrographs of LPS DCs exposed to Ebo-GPVP40-eGFP VLP and then to HIV-1NFN-SX. Arrows indicate distinct particles captured in VCC. **H.**S uper-resolution microscopy of VCC architecture on LPS DCs exposed to Ebo-GPVP40-NanoLuc (red) and HIV-1 Gag-eGFP mixed with HIV-1 NL4.3. Siglec-1 is shown in magenta. Left column depicts volume slices while right column shows a 3D reconstruction of a representative VCC. Fig. 7 shows a 3D reconstruction of VCC from a different donor. Statistical differences in A and F were assessed with a paired t-test, and with a one-sample t-test in B.
**Figure 5**.R epresentative surface staining of Raji cell lines. Histograms show surface phycoerythrin (PE) staining of Siglec-1, DC-SIGN or Siglec-5 in each Raji cell line.
**Figure 6**.M onocytes display GM1 on their membrane and produce Ebo VLPs recognized by Siglec-1. **A.**H istograms show surface staining of GM1 ganglioside in primary monocytes. Numbers indicate the mean percentage of positive cells and the SD from 3 independent donors. **B**.B inding of monocyte-derived Ebo-GPVP40-NanoLucVLP to a human Fc fragment capture ELISA as in Fig. 2F assessed by NanoLuciferase activity (in RLUs). Graphs show mean values and SEM from one experiment analyzing two different monocyte-derived viral stocks in triplicate. Statistical differences were assessed with a Mann-Whitney test. **C**.C omparative uptake of monocyte-derived Ebo-GPVP40-NanoLuc VLP by Raji Siglec-1 cells for 12 h at 37°C pre-incubated with the indicated mAbs. Graphs show mean values and SEM from 2 experiments analyzing two different monocyte-derived viral stocks.
**Figure 7****.** Super-resolution microscopy of VCC architecture. LPS DCs were pulsed and labeled as in Fig. 4H. Left column depicts volume slices while right column shows 3D reconstruction.

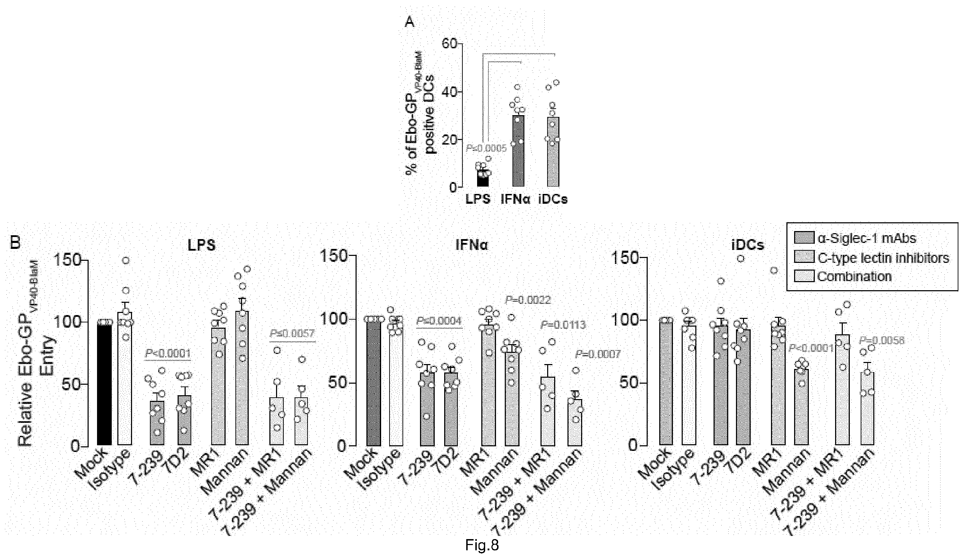

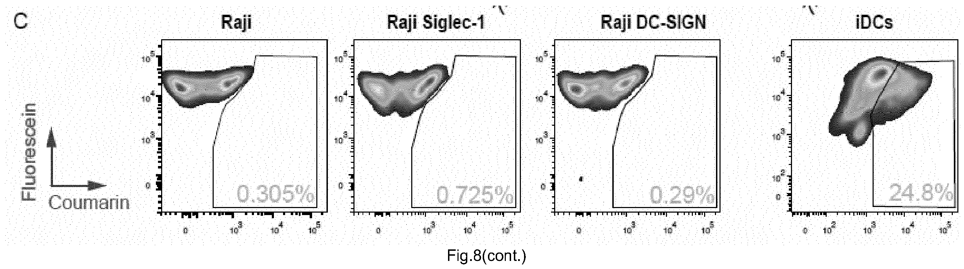

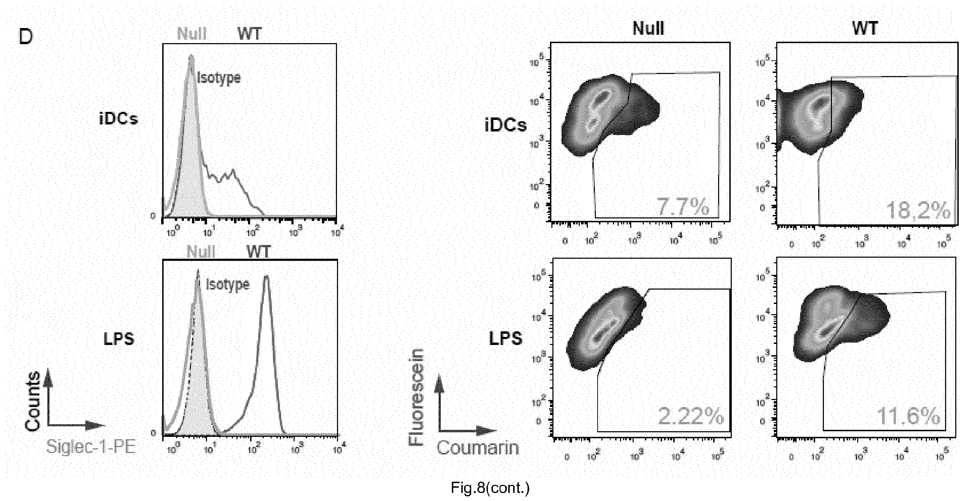

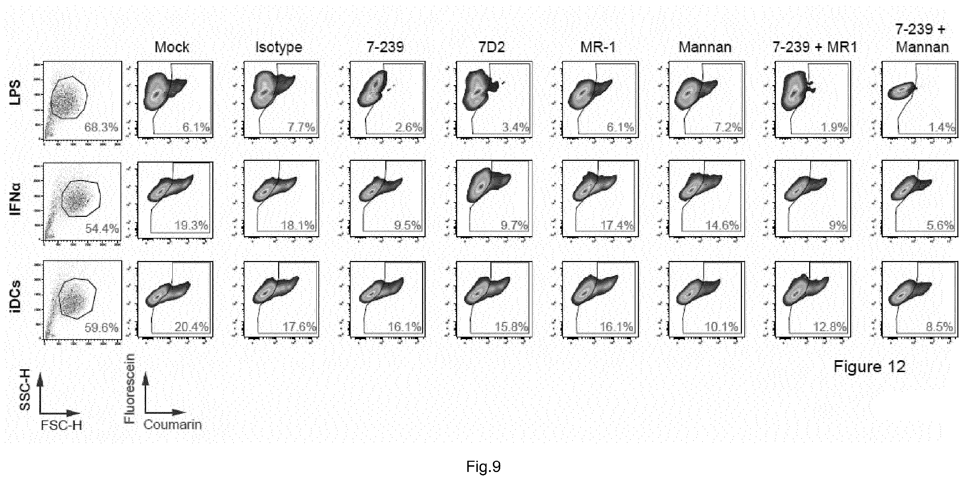

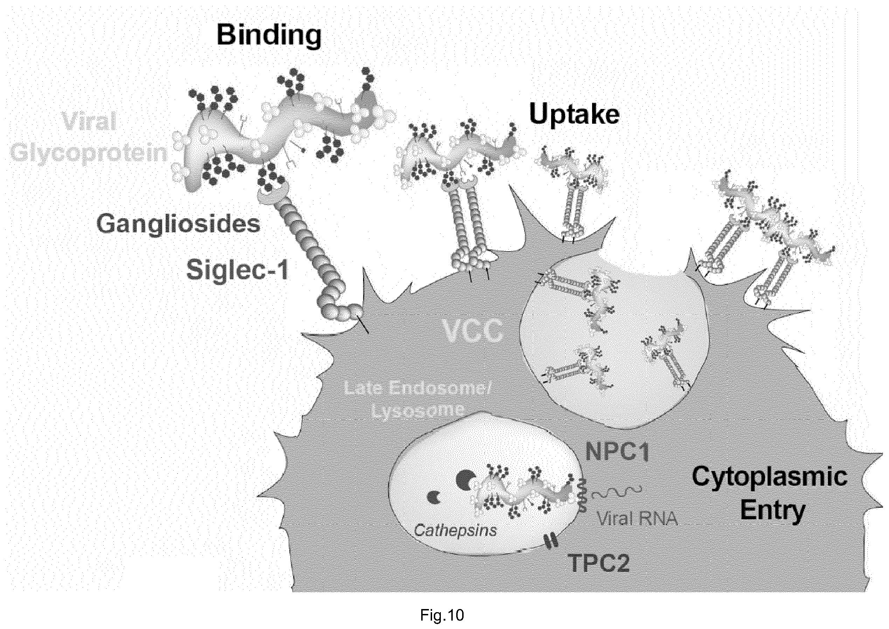

**Figure 8****.** Ebola virus uptake by Siglec-1 facilitates cytoplasmic viral entry into activated DCs. **A**.C omparative cytoplasmic entry of Ebo-GPVP40-BlaM VLP added for 12 h at 37°C to DCs and assessed by FACS with a CCF2-AM dye. Data show mean values and SEM from 3 experiments including cells from 8 donors. **B.** Relative cytoplasmic entry of Ebo-GPVP40-BlaM VLP by distinct DCs pre-incubated with the indicated mAbs and inhibitors. Values are normalized to the level of Ebo-GPVP40-BlaM entry by mock-treated cells shown in panel A (set at 100%). Mean values and SEM from 3 experiments include cells from 5-8 donors. Representative dot plots are shown in Fig. 9. **C**. Representative dot plots comparing cytoplasmic viral entry of Ebo-GPVP40-BlaM VLP as in A on distinct Raji cells and iDCs. **D**. Histograms show Siglec-1 expression on iDCs or LPS DCs derived from a Siglec-1 null individual and wild type individual. Dot plots compare cytoplasmic viral entry of Ebo-GPVP40-BlaM VLP on iDCs or LPS DCs from wild type and Siglec-1 null individuals. Fig. 10 depicts the proposed mechanism for Siglec-1 in Ebola virus uptake and subsequent cytoplasmic entry. Statistical differences in A were assessed with a paired t-test, and with a one-sample t-test in B.
**Figure 9**.R epresentative gating analysis for cytoplasmic Ebola viral entry. FACS analysis of Ebo-GPVP40-BlaM VLP entry by distinctly treated DCs from one donor pre-incubated with the indicated mAbs and inhibitors.
**Figure 10**.P roposed mechanism for Ebola virus uptake and cytoplasmic entry mediated by Siglec-1. Siglec-1 recognition of sialylated gangliosides such as GM1 on the membrane of Ebola virus (shown in red) modulates the binding, uptake and trafficking of viral particles to a sac-like virus-containing compartment (VCC) continuous with the plasma membrane. Viruses stored on VCCs can be redirected into the classical internalization pathway and facilitate viral entry into the cytoplasm. NPC1 is Niemann-Pick C1 receptor and TPC2 is two-pore channel 2.

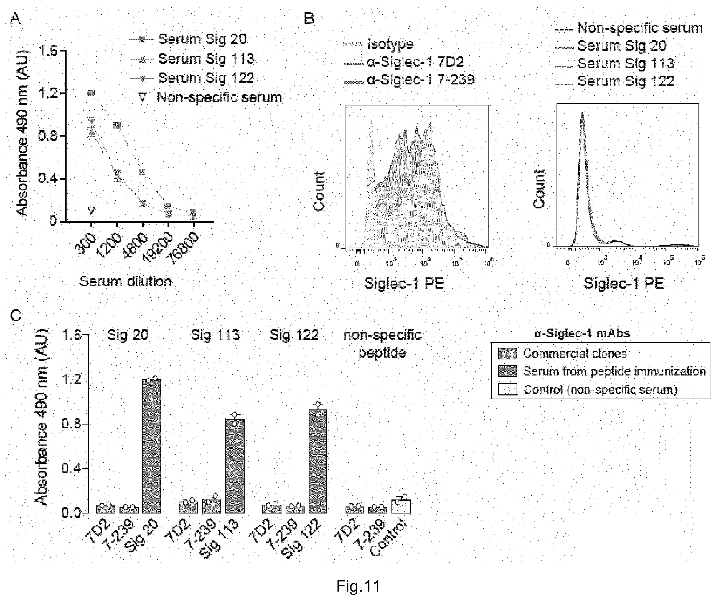

**Figure 11****.** Immunizations with small Siglec-1 linear peptides produce specific Abs that fail to recognize Siglec-1. **A**.E LISA of representative sera from immunizations with Siglec-1 linear peptides Sig20, Sig113, and Sig122. Plates coated with each peptide were probed with serial dilutions of sera from the corresponding immunization. Clear symbol shows the recognition of the Siglec-1 peptides by the serum of a non-related peptide immunization. **B.** Representative Siglec-1 staining of Raji Siglec-1 cells labeled with commercial α-Siglec-1 mAbs 7-239 and 7D2, non-specific serum, or the indicated sera from immunized mice, revealed with a secondary α-IgG polyclonal Ab labeled in PE. **C.** Lack of recognition of Siglec-1 peptides Sig20, Sig113, Sig122 or a non-specific peptide by the commercial α-Siglec-1 mAbs detected with an ELISA. Sera from immunized mice with the corresponding peptide were used as positive controls. Graph shows mean values and SEM from one experiment with two replicas.

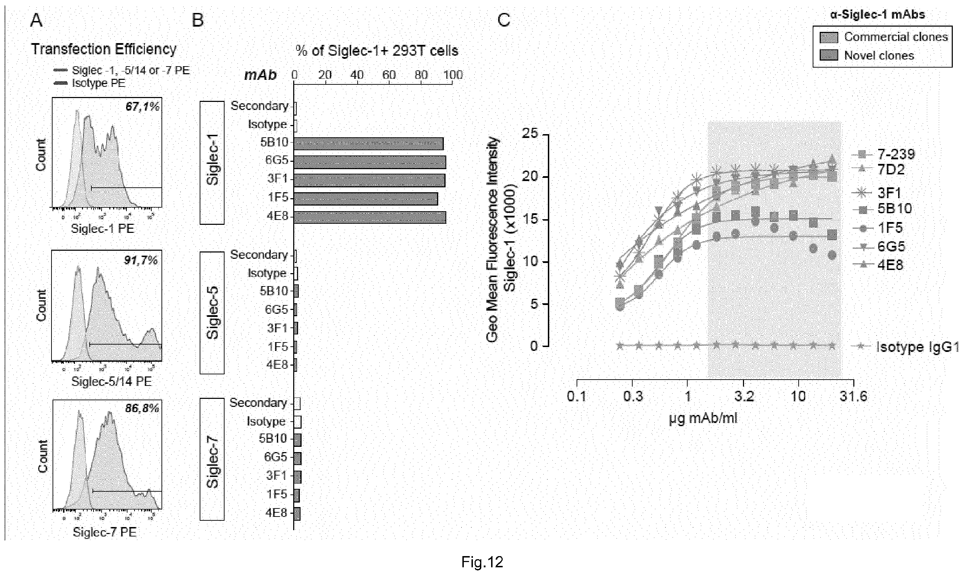

**Figure 12****.** Immunizations with Siglec-1 expressing cells produce mAbs that are able to recognize Siglec-1. **A.**T ransfection efficiencies of HEK-293T cells assessed by cell surface staining of Siglec-1, Siglec-5/14, and Siglec-7 (blue lines) compared to isotype control (grey lines). Of note, Siglec-14 shares 100% of amino acid homology with Siglec-5 in the V-set domain, so mAbs recognizing Siglec-5 cross-react with Siglec-14. **B.**F ACS staining of HEK-293T cells transfected with Siglec-1 plasmid using novel α-Siglec-1 mAbs, and lack of recognition of Siglec-5 and Siglec-7 transfected cells, revealed with a DyLight 649 secondary IgG mAb. Siglec-5 plasmid was chosen because it has the highest sequence homology to the V-set domain of Siglec-1. Graph shows values from one experiment. **C**.R epresentative Siglec-1 staining of Raji Siglec-1 cells labeled with increasing concentrations of novel α-Siglec-1 mAbs shown in grey (1F5, 6G5, 4E8, 5B10 and 3F1), commercial mAbs α-Siglec-1 shown in orange (7-239 and 7D2), and an isotype control revealed with a DyLight 649 secondary IgG mAb. Non-linear fit to a variable response curve is depicted for each mAb, and shadowed area shows saturating concentrations for all mAbs.

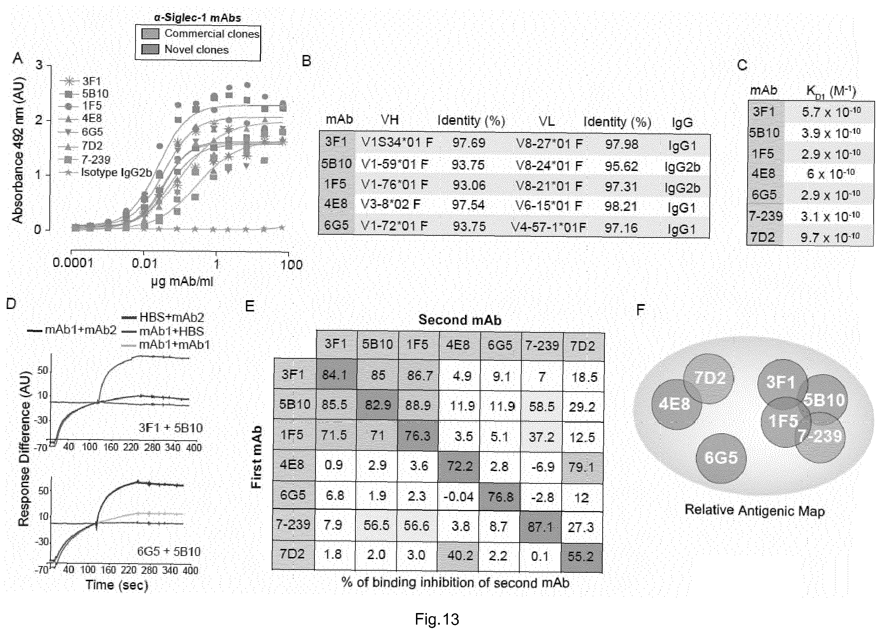

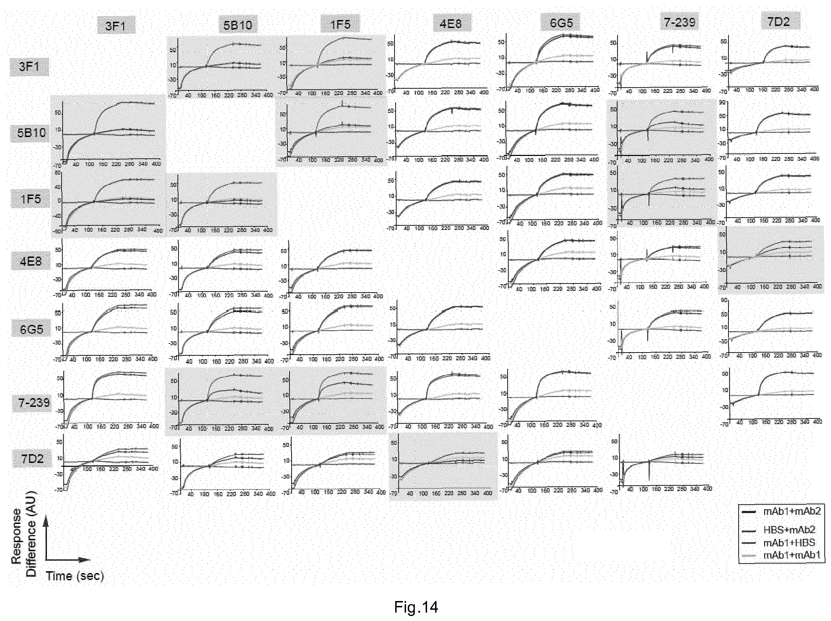

**Figure 13****.** Generation and characterization of novel α-Siglec-1 mAbs. **A.** Binding of α-Siglec-1 mAbs to immobilized Siglec-1 mini-proteins detected by ELISA. **B**.V ariable heavy (VH) and Variable light (VL) chains-gene usage, sequence analysis, and immunoglobulin G (IgG) subclass determined by ELISA. **C**.E quilibrium dissociation constants (KD1) calculated by fitting to a bivalent model the kinetic parameters obtained by surface plasmon resonance, where a recombinant human Siglec-1 protein was covalently immobilized to a sensor chip. **D.** Competitive Siglec-1 binding assays of mAb pairs analyzed by surface plasmon resonance. Graphs show response difference (arbitrary units) over time (seconds) of co-injected mAb pairs (blue lines). Top graph shows an example of competition of the second mAb with the first mAb, where we retrieved a similar signal as controls where we injected either HBS buffer followed by the first mAb or co-injected the first mAb (grey lines). An example of lack of competition is shown in the bottom graph, where signal was similar to that obtained when buffer was injected before the second mAb (red lines). Association-dissociation curves of all the individual binding experiments are shown in Fig. 14. **E.** Percentage of binding inhibition calculated for each mAb competition. Red color intensities depict the extent of competition. **F.** Relative antigenic map summarizing all combinatorial cross-competition analyses performed, where overlap represents high degree of competition between mAbs recognizing proximal epitopes.
**Figure 14****.C** ompetition between mAbs for Siglec-1 binding. Graphs show all competitions performed as in Fig. 13D. Red shadowed areas depict high degree of competition. Graphs showing competition between each mAb with itself are not shown but are included as controls in each single experiment (light grey lines).

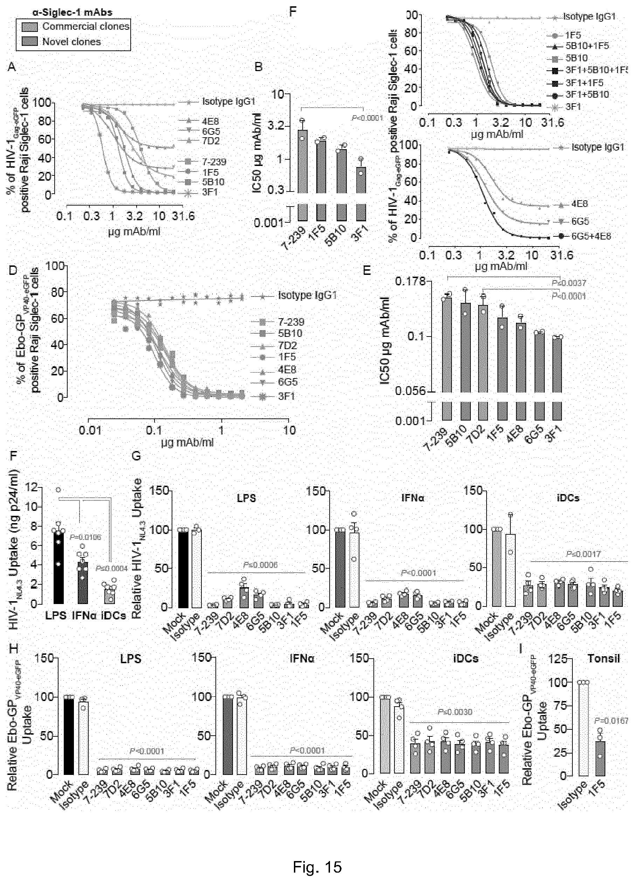

**Figure 15****.** Novel α-Siglec-1 mAbs inhibit HIV-1 and Ebola viral uptake. **A.**C ompetition for Raji Siglec-1 binding between HIV-1 Gag-eGFP VLPs and α-Siglec-1 mAbs for 1 h at 37°C. Non-linear fit to a variable response curve from one representative experiment out of two is shown. **B.** IC50 values of the particular mAbs that reached a total HIV-1Gag-eGFP VLP blocking effect. Data show mean values and SEM from two independent experiments. Associated F tests for the LogIC50 of commercial 7-239 and novel 3F1 mAbs were used to assess statistical differences. **C.**C ompetition for Raji Siglec-1 binding between HIV-1 Gag-eGFP VLP and mixes of novel α-Siglec-1 mAbs with complete (top graph) or partial (bottom graph) viral uptake inhibition as in A. **D.** Competition for Raji Siglec-1 binding between Ebo-GPVP40-eGFP VLP and α-Siglec-1 mAbs for 3 h at 37°C. **E.**I C50 values of all mAbs, which in the case of Ebo-GPVP40-eGFP VLP, reached a total blocking effect. Statistical differences were assessed as in B. **F.**C omparative DC uptake of wild type HIV-1 NL4.3. DCs were pulsed for 3 h at 37° C, washed and lysed to measure the cell-associated p24 by ELISA. Data show mean values and SEM from 3 experiments including cells from 7 donors. **G.** Relative uptake of HIV-1 by distinct DCs pre-incubated with the indicated mAbs and assessed by p24 ELISA. Values are normalized to mock-treated cells shown in panel F (set at 100%). Mean values and SEM from two experiments include cells from 2-4 donors. **H**.Relative uptake of Ebo-GPVP40-eGFP VLP for 3h at 37°C by distinct DCs pre-incubated with the indicated mAbs. Values are normalized as in G. Mean values and SEM from two experiments include cells from 4 donors. **I.** Relative uptake of Ebo-GPVP40-eGFP VLP for 12 h at 37°C by IFNα-treated BDCA1-positive myeloid cells directly isolated from lymphoid tissues. Isotype-treated cells (with a mean uptake of 15 % ± 8 SD) were set at 100%. Mean values and SEM from one experiment include cells from 3 donors. Statistical differences in F were assessed with a paired t-test, and in G-I with a one-sample t-test.

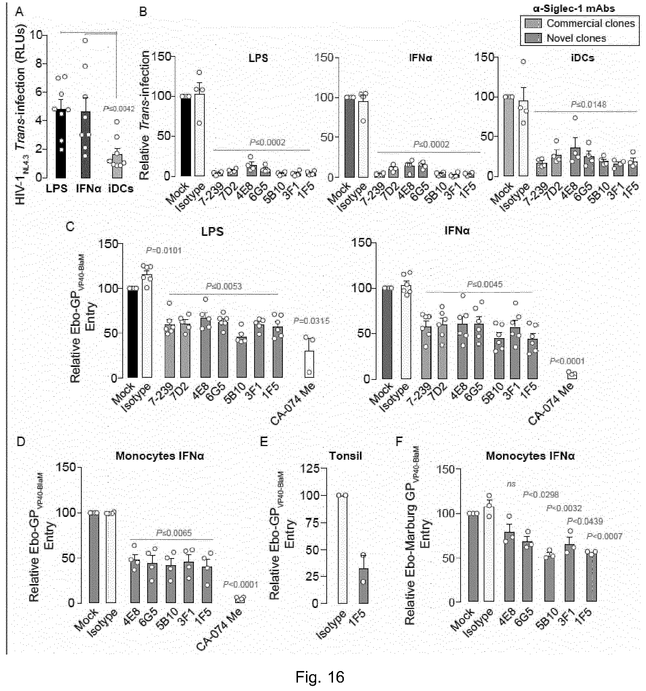

**Figure 16****.** Novel α-Siglec-1 mAbs block HIV-1 trans-infection and cytoplasmic Ebola entry. **A.** Comparative HIV-1 NL4.3 trans-infection of a luciferase reporter CD4+ cell line mediated by distinct DCs. Cells were pulsed as in 15F, washed and co-cultured with target cells for 2 days to determine the induced luciferase activity in relative light units (RLUs). Data show mean values and SEM from 3 experiments including cells from 8 donors in duplicates. **B**.R elative HIV-1 transmission from DCs pre-incubated with commercial and novel mAbs. Values are normalized to mock-treated cells shown in panel A (set at 100%). Mean values and SEM from two experiments include cells from 4 donors. **C**.R elative cytoplasmic entry of Ebo-GPVP40-BlaM VLP for 12h at 37°C by distinct DCs pre-incubated with commercial and novel mAbs. Cathepsin B inhibitor CA-074 Me was used as control. Values are normalized to mock-treated cells shown in Fig. 8A (set at 100%). Mean values and SEM from 2 experiments include cells from 3-6 donors. **D.** Relative entry of Ebo-GPVP40-BlaM VLP for 12h at 37°C by IFNα -treated monocytes pre-incubated with novel mAbs as in C. Mock-treated cells (with a mean entry of 41 % ± 20 SD) were set at 100%. Mean values and SEM from one experiment include cells from 4 donors. **E.**R elative entry of Ebo-GPVP40-BlaM VLP for 12 h at 37°C by IFNα-treated BDCA1-positive myeloid cells directly isolated from lymphoid tissues pre-incubated with novel mAb as in C. Isotype-treated cells (with a mean entry of 5 % ± 4 SD) were set at 100%. Mean values and SEM from one experiment and cells from two donors. **F.**R elative entry of Ebo-MarburgGPVP40-BlaM VLP (containing the Marburg viral glycoprotein) for 12 h at 37°C by IFNα-treated monocytes pre-incubated with novel mAb as in C. Mock-treated cells (with a mean entry of 20 % ± 5 SD) were set at 100%. Mean values and SEM from one experiment and cells from 3 donors.
   Statistical differences in A were assessed with a paired t-test, and in B-F with a one-sample t-test.

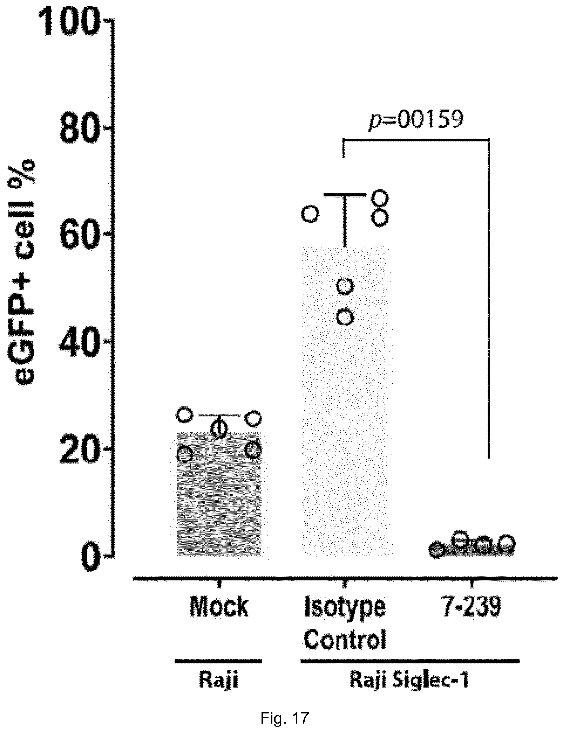

**Figure 17****.** Viral uptake of Junin VLPs by Raji Siglec-1 cells treated with the anti-Siglec-1 7-239 mAb.

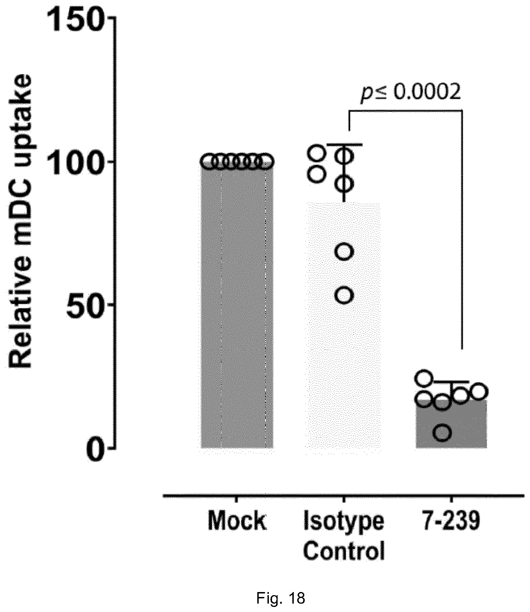

**Figure 18****.**V iral uptake of Junin VLPs by pre-incubated DCs treated with the anti-Siglec-1 7-239 mAb.

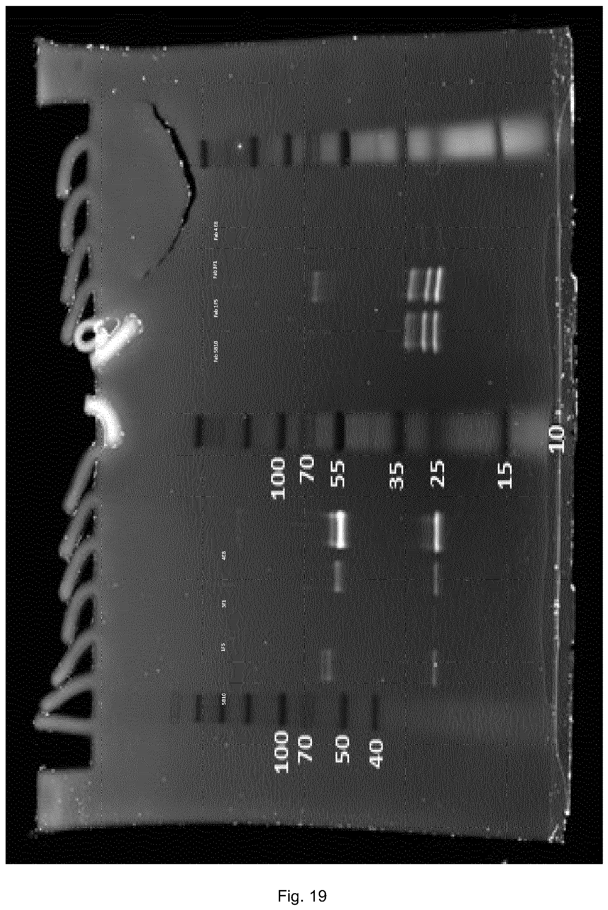

**Figure 19****.** Gel showing the cutting of the 1F5, 5B10, 3F1 and 4E8 mAbs into antigen-binding fragments (Fab).

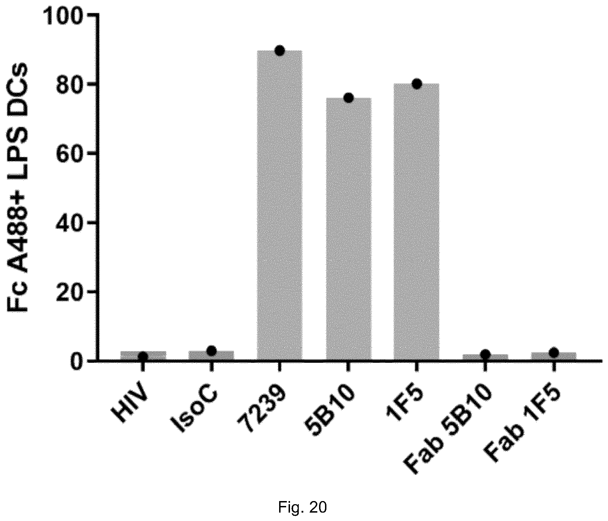

**Figure 20****.**F c region labelling of the 1F5 and 5B10 Fabs. No FC region was present in the FC labelling assay.

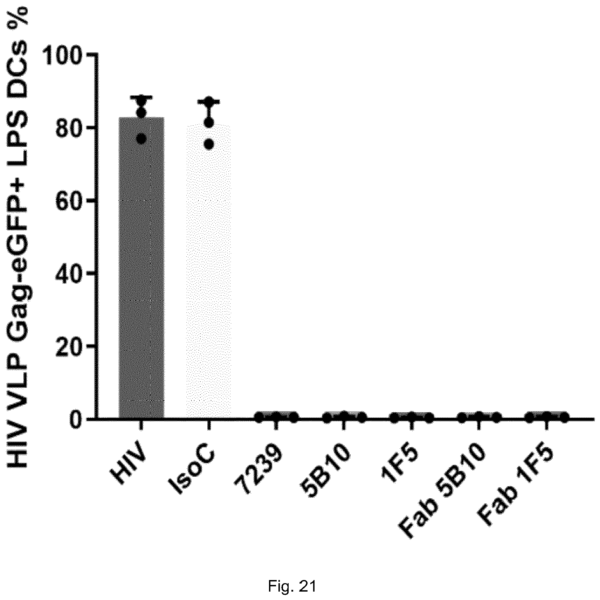

**Figure 21****.** Blocking effect of the purified 1F5 and 5B10 Fabs on Siglec-1 receptor using fluorescent HIV VLPs. The results show that the 1F5 and 5B10 Fabs have similar efficacy blocking Siglec-1 in DCs as their original mAbs or control mAbs.

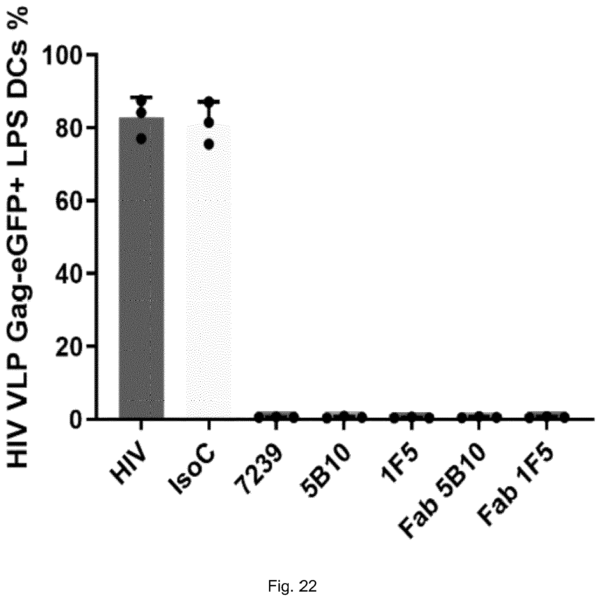

**Figure 22****.** Blocking effect of the purified 1F5 and 5B10 Fabs on Siglec-1 receptor using fluorescent WT HIV VLPs. The results show that the 1F5 and 5B10 Fabs have similar efficacy blocking Siglec-1 in DCs as their original mAbs or control mAbs.

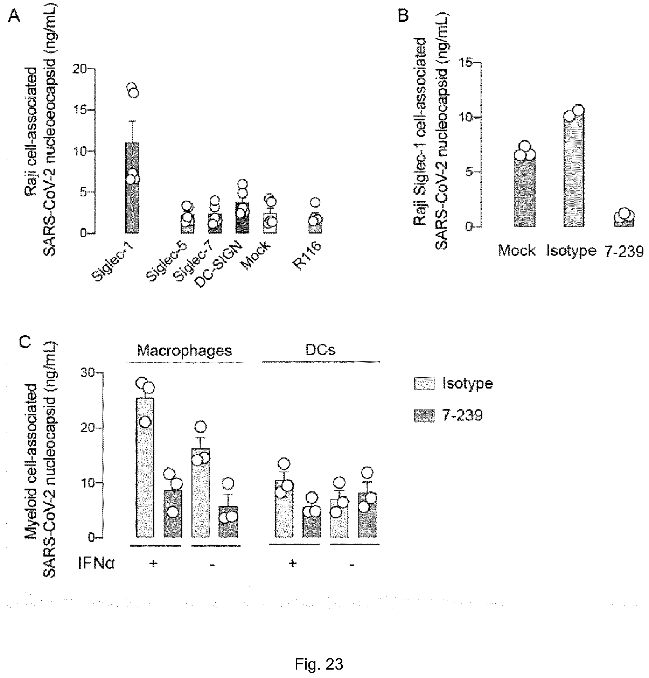

**Figure 23****. A**.C omparative uptake of SARS-CoV-2 for 2 h at 37 °C by Raji B cells. Values from five replicas and two experiments. **B**.U ptake of SARS-CoV-2 for 2 h at 37 °C by Raji B cells expressing Siglec-1 that were pre-incubated with indicated mAbs. Values from three replicas and one experiment. **C.** Comparative uptake of SARS-CoV-2 for 3 h at 37 °C by macrophages and DCs activated with IFNα or not that were pre-incubated with indicated mAbs. Values from three replicas and one experiment.

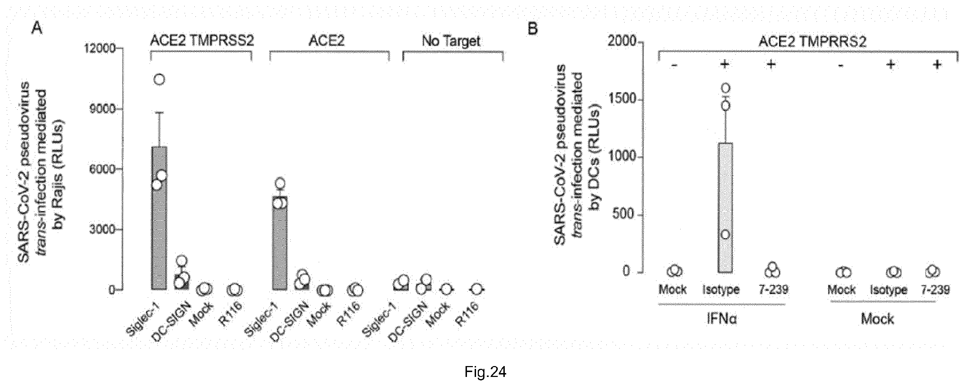

**Figure 24****. A**.T ransmission of SARS-CoV-2 pseudoviruses from distinct Raji cells to a target ACE2/TMPRSS2 expressing cell line. Raji cells were pulsed with an HIV-1 reporter virus containing luciferase that were pseudotyped with the SARS-CoV-2 spike instead of the HIV-1 envelope. Cells were then washed, and co-cultured with target cells for 48 h. Infection of target cells was determined by induced luciferase activity in relative light units (RLUs). No viral fusion was detected on Raji cells not co-cultured with target cells. Data show mean values and SEMs from one experiment including three replicates. **B.** Transmission of SARS-CoV-2 pseudoviruses from distinct DCs as in A. DCs were pre-incubated as in Figure 23B, washed, and co-cultured with target cells for 48 h. No viral fusion was detected on DCs not co-cultured with target cells. Data show mean values and SEMs from one experiment including cells from three donors.

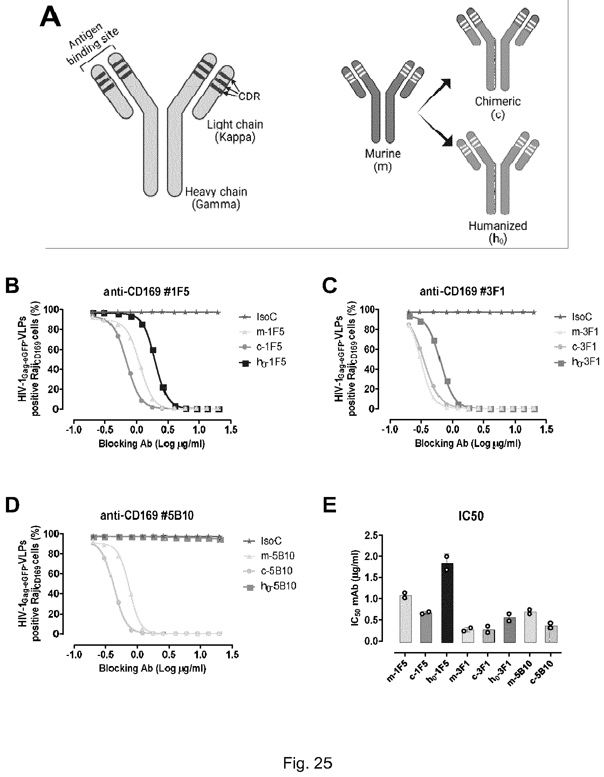

**Figure 25****. Humanization of anti-CD169-mAbs and functional validation. A**.Diagram illustrating the design of humanized anti-CD169 mAbs. **B. C. D.**C ompetition for Raji-Siglec-1 cells binding between HIV-1Gag-eGFP-VLPs and 3 different anti-CD169 mAbs for 1 h at 37 °C. As reference, antibody isotype control is used (IsoC, with grey star symbols). A comparison between original murine antibody (m-, triangle and lighter color), chimeric (c-, circle) and humanized CDR-engrafted (h0, square and darker color) is shown for each antibody clone (#1F5, B; #3F1, C; #5B10, D) corresponding to a non-linear fit to a variable response curve from one representative experiment out of two. **E.** IC50 values of individual mAbs that achieved total HIV-1Gag-eGFP-VLP blocking effect. Data from two independent experiments.

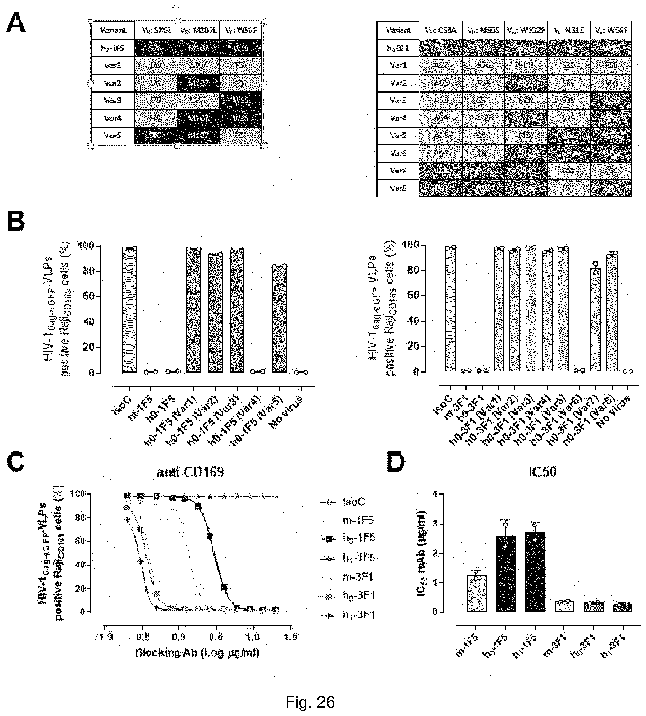

**Figure 26****. A.**P otential sequence liabilities found in CDR-engrafted humanized antibodies.**B** .C ompetition assay with RajiCD169 cells binding and HIV-1Gag-eGFP-VLPs to test anti-CD169 mAb variants to eliminate selected potential liabilities.**C** . Competition for RajiCD169 cells binding between HIV-1Gag-eGFP-VLPs and anti-CD169 mAbs. The inventors tested 3 different forms of #1F5 and #3F1 antibodies: murine (m-, triangle and lighter color), humanized (h0-, square) and stabilized (h1-, diamond). The comparison among these antibodies is shown with a non-linear fit to a variable response curve from one representative experiment out of two.**D** . IC50 values of individual mAbs were calculated with non-linear fit to a variable response curve. Data from two independent experiments.

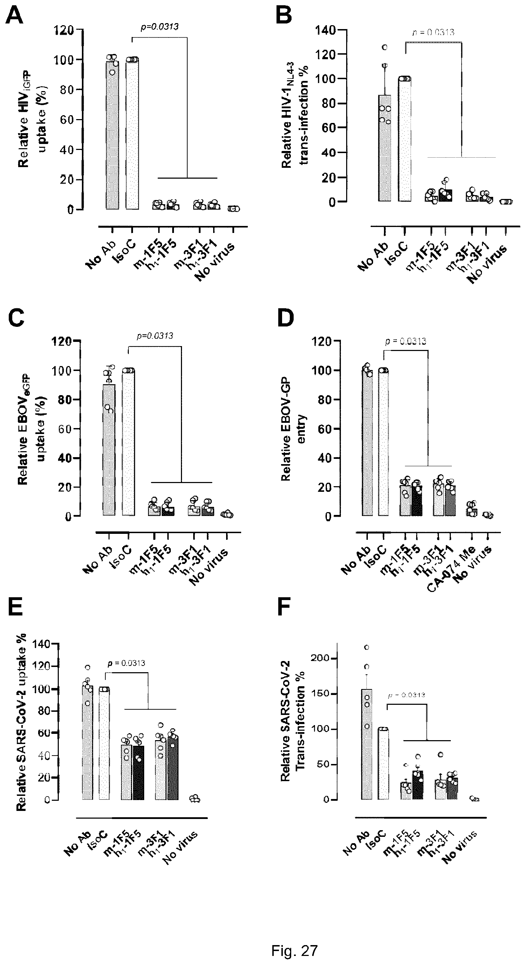

**Figure 27****. H umanized anti-CD169 mAbs block DC-mediated viral entry.** A Humanized anti-CD169 mAbs block DC-mediated HIV-1 uptake and trans-infection. A. Relative viral uptake of HIV-1 pulsed for 4 h at 37°C with LPS-treated DCs pre-incubated with humanized anti-CD169 mAbs. Values are normalized to cells with Isotype control mAb incubation, with a mean entry of 78,8 ± 9,7% (SD) and set as 100%. **B**.R elative HIV-1 trans-infection mediated by LPS-treated DCs pre-incubated with anti-CD169 mAbs. Values are normalized to cells with Isotype control mAb incubation, with a mean entry of 2,83 x106 ± 2,3 x106 RLU (SD) and set at 100%. **C**.H umanized anti-CD169 mAbs block DC-mediated Ebola VLP uptake and internalization. A. Relative viral uptake of Ebo VP40eGFP VLPs pulsed for 4 h at 37°C with LPS-treated DCs pre-incubated with humanized anti-CD169 mAbs. Values are normalized to cells with Isotype control mAb incubation, with a mean entry of 51,2 ± 18,7% (SD) and set as 100%. **D**.R elative Ebo VP40BlaM-ZaiGP internalization mediated by LPS-treated DCs pre-incubated with anti-CD169 mAbs. Values are normalized to cells with Isotype control mAb incubation, with a mean entry of 46,8 ± 14,2% (SD) and set at 100%. **E**.H umanized anti-CD169 mAbs block DC-mediated SARS-CoV-2 uptake and trans-infection. A. Relative viral uptake of SARS-CoV-2 pulsed for 3 h at 37°C with LPS-treated DCs pre-incubated with humanized anti-CD169 mAbs. Values are normalized to cells with Isotype control mAb incubation, with a mean entry of 13,65 ± 8,2 ng/mL (SD) and set as 100%. **F**. Relative SARS-CoV-2 trans-infection mediated by LPS-treated DCs pre-incubated with anti-CD169 mAbs. Values are normalized to cells with Isotype control mAb incubation, with a mean entry of 12,3 ± 2,9 ng/mL (SD) and set at 100%. Data show mean values and SD from two independent experiments and include cells from six donors. Statistical differences were assessed using a one-sample Wilcoxon test.

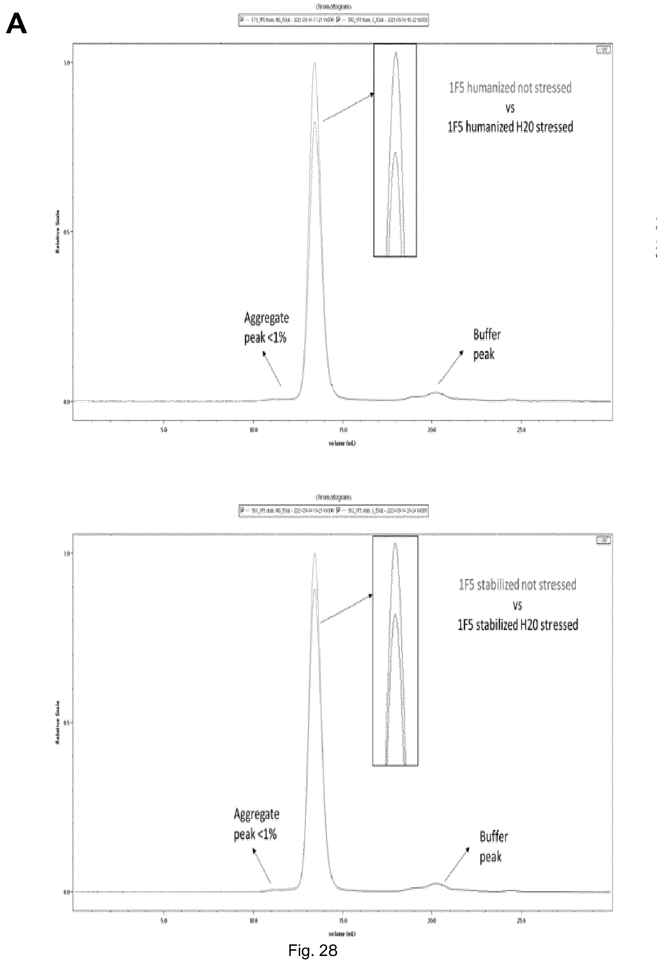

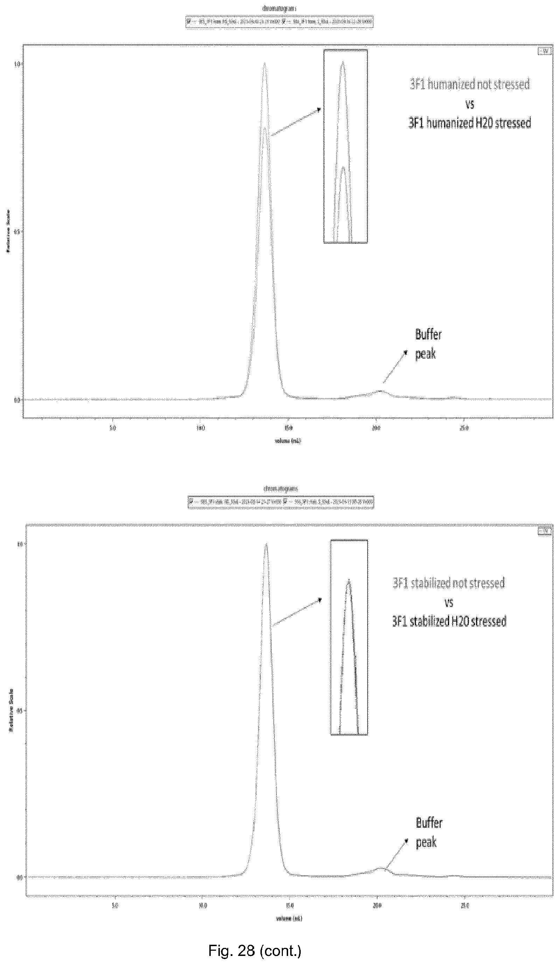

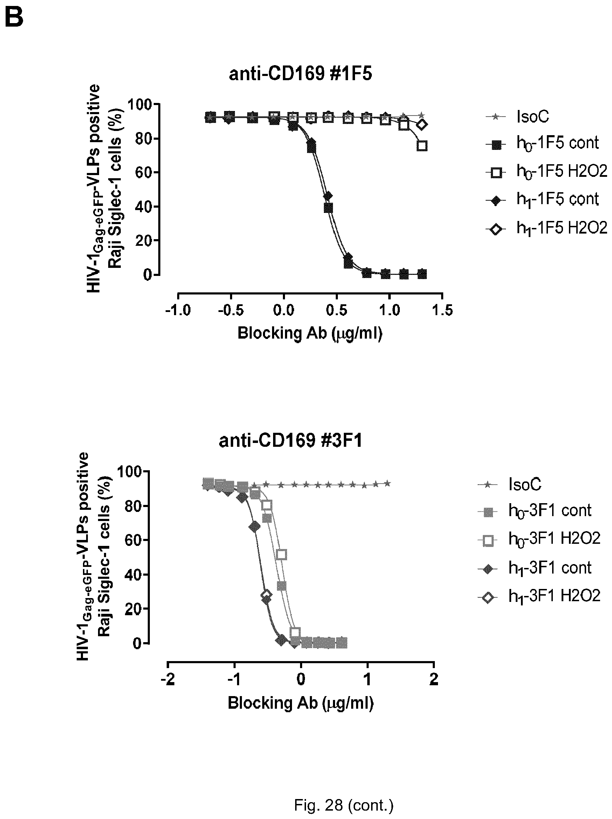

**Figure 28**.S tability analysis of humanized and stabilized anti-CD169-mAbs under oxidative stress. **A**.Chromatogram of anti-CD169-mAbs before and after H2O2 oxidative stress measured by SEC-MALS. **B**.C ompetition for RajiCD169 cells binding between HIV-1Gag-eGFP-VLPs and anti-CD169 mAbs. The inventors tested 2 different forms of #1F5 and #3F1 antibodies: humanized (h0-, square) and stabilized (h1-, diamond), before (filled symbols) and after (empty symbols) treatment with H2O2 oxidative stress. Non-linear fit to a variable response curve from one representative experiment out of two.

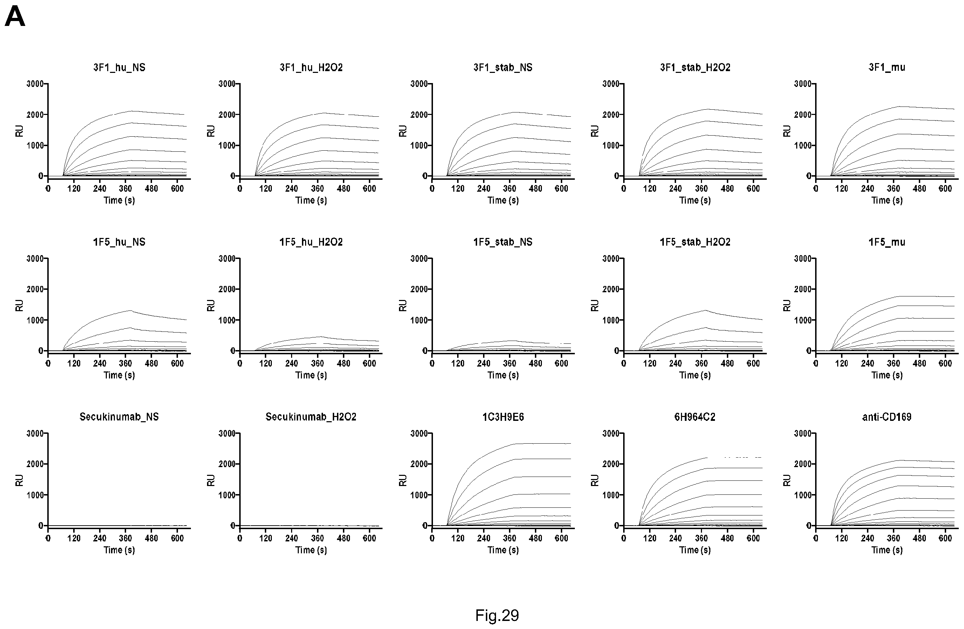

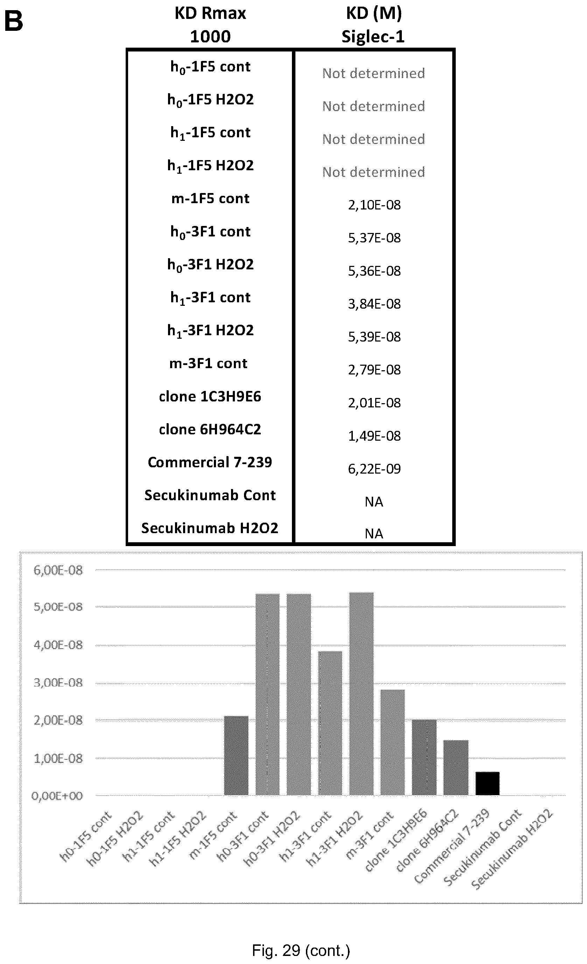

**Figure 29**.A ffinity analysis of anti-CD169-mAbs for their target CD169 receptor measured with IBIS MX96 platform. Secukinumab is included as negative control antibody that do not bind CD169. **A**.S ensograms of anti-CD169-mAbs binding to CD169 receptor before and after oxidative stress. **B**.C alculation of KD by interpolation of equilibrium fit.

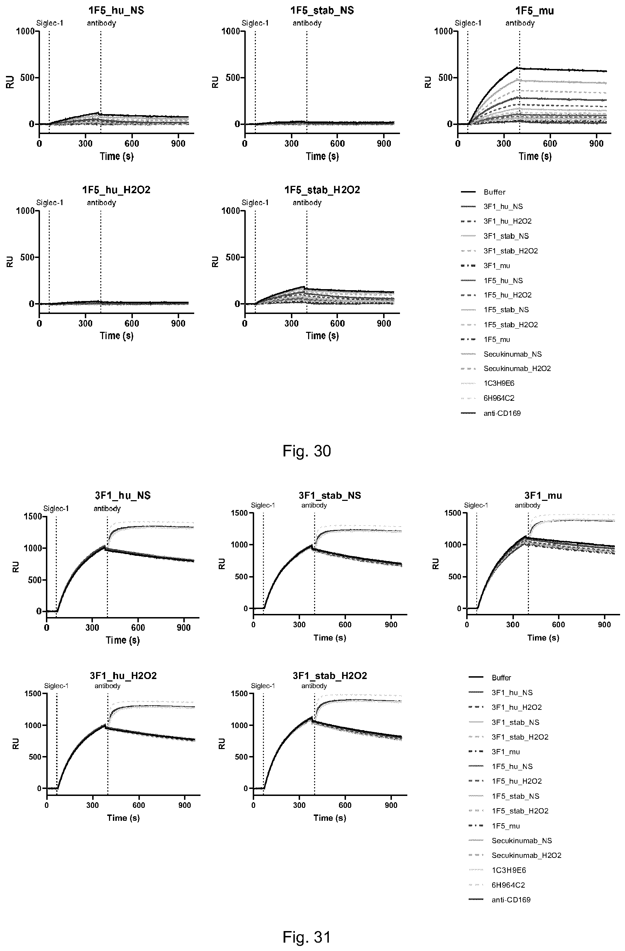

**Figure 30**.E pitope binning of anti-CD169-mAbs for their target CD169 receptor measured with IBIS MX96 platform. Antibodies indicated on each panel were spotted at different locations on an IBIS chip at 60nM. Then, recombinant CD169 protein was flowed at 20 nMo ver the sensor, followed by the antibody variant at 100 nM indicated with the colored line in the legend. CD169/Siglec-1 and antibody injection for competition are depicted with a vertical dotted line.
**Figure 31****.** Epitope binning of anti-CD169-mAbs for their target CD169 receptor measured with IBIS MX96 platform. Antibodies indicated on each panel were spotted at different locations on an IBIS chip at 60nM. Then, recombinant CD169 protein was flowed at 20 nMo ver the sensor, followed by the antibody variant at 100 nM indicated with the colored line in the legend. CD169/Siglec-1 and antibody injection for competition are depicted with a vertical dotted line.

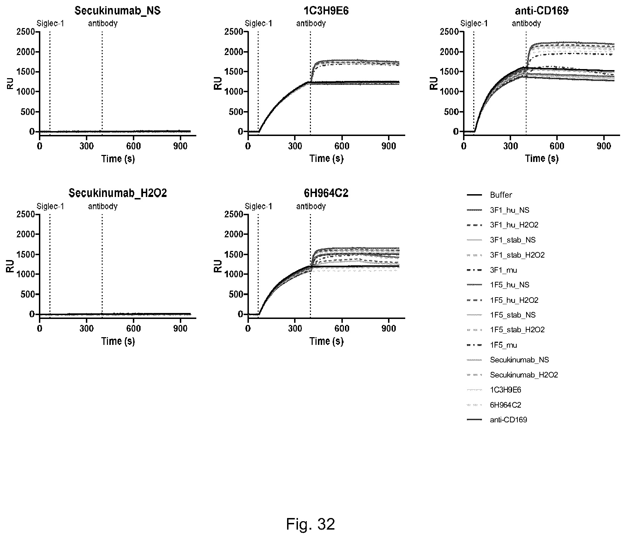

**Figure 32****.** Epitope binning of anti-CD169-mAbs for their target CD169 receptor measured with IBIS MX96 platform. Antibodies indicated on each panel were spotted at different locations on an IBIS chip at 60nM. Then, recombinant CD169 protein was flowed at 20 nMo ver the sensor, followed by the antibody variant at 100 nM indicated with the colored line in the legend. CD169/Siglec-1 and antibody injection for competition are depicted with a vertical dotted line. Secukinumab is included as negative control antibody that do not bind CD169.

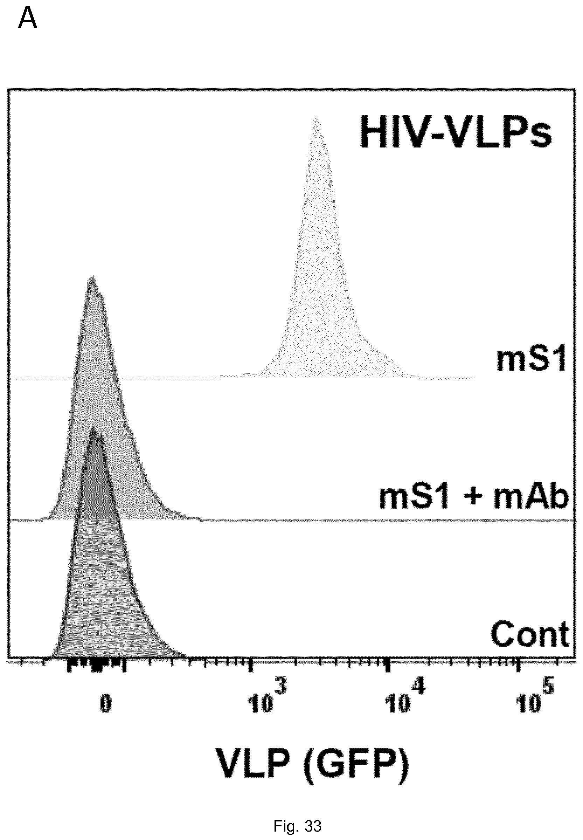

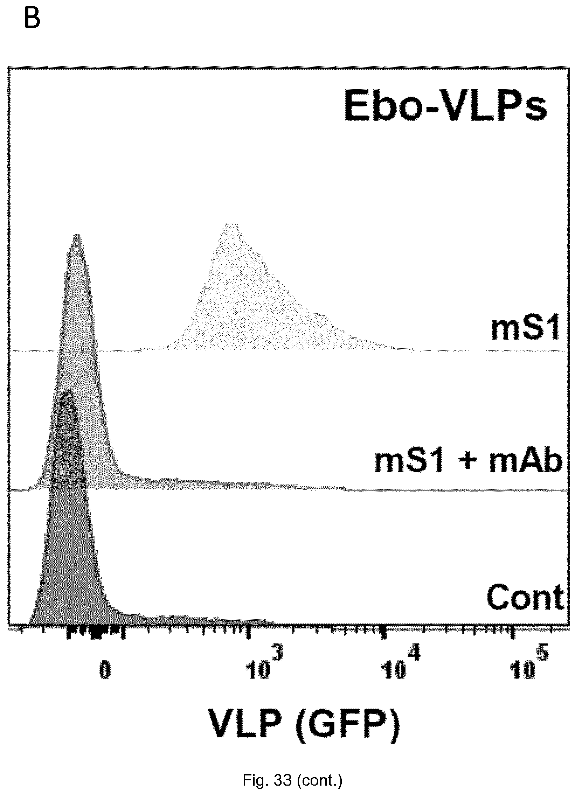

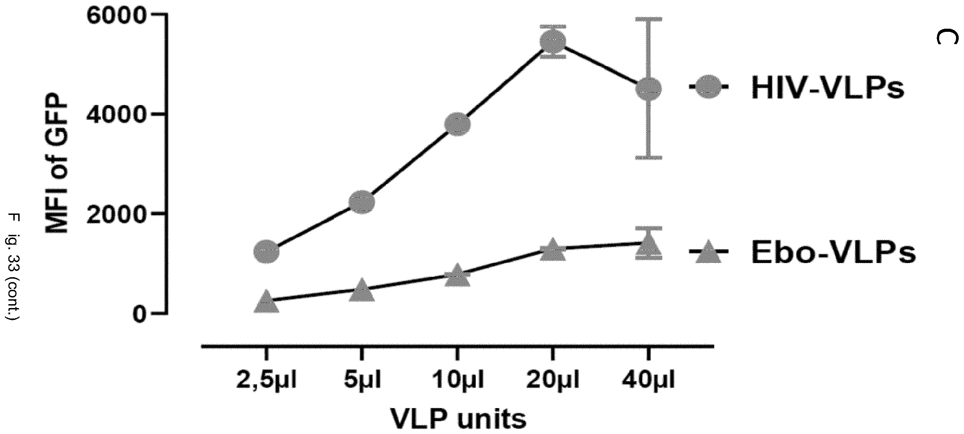

**Figure 33****.** Fig. 23. A and B. Detection of HIV and Ebola VLPs by a mS1 bead platform by flow cytometry. C. The mean fluorescence intensity (MFI) at distinct viral concentrations is also shown.

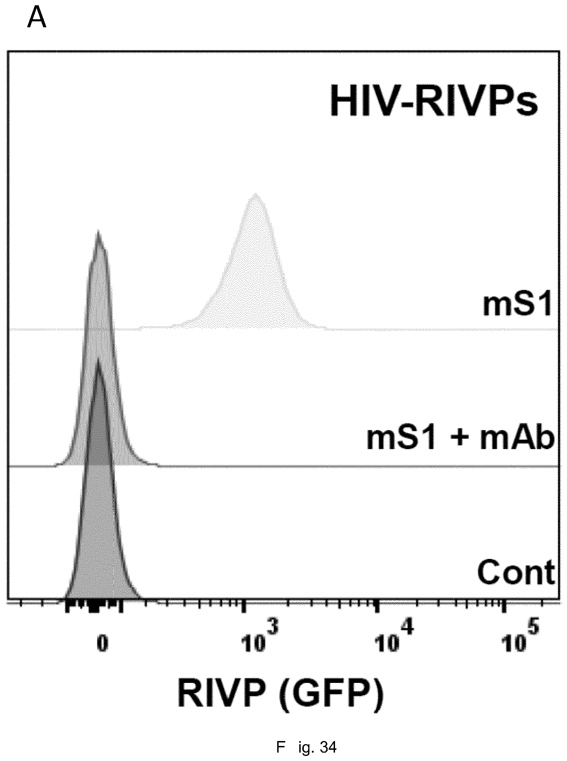

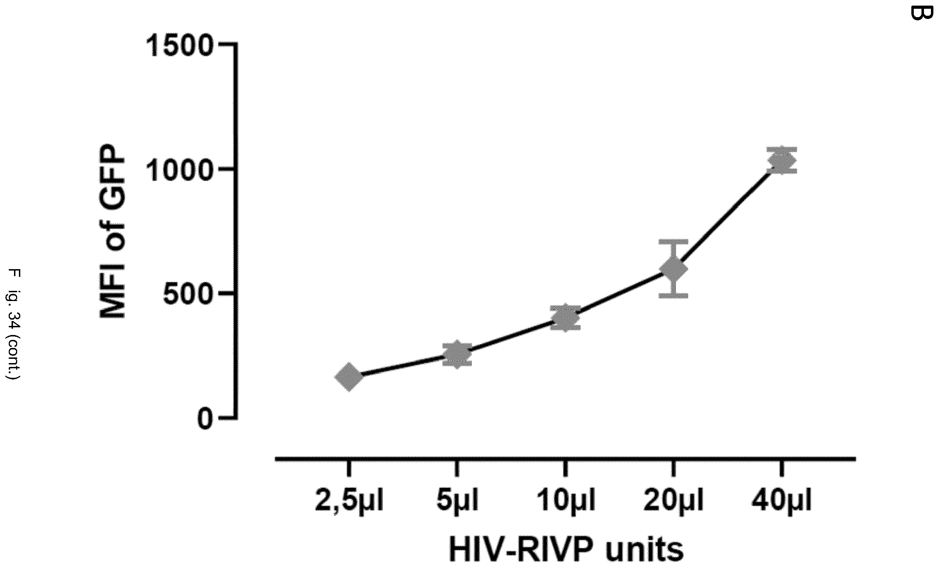

**Figure 34****.** Fig. 24. A. Detection of HIV-RIVP by a mS1 bead platform. B. The mean fluorescence intensity (MFI) at distinct viral concentrations is also shown.

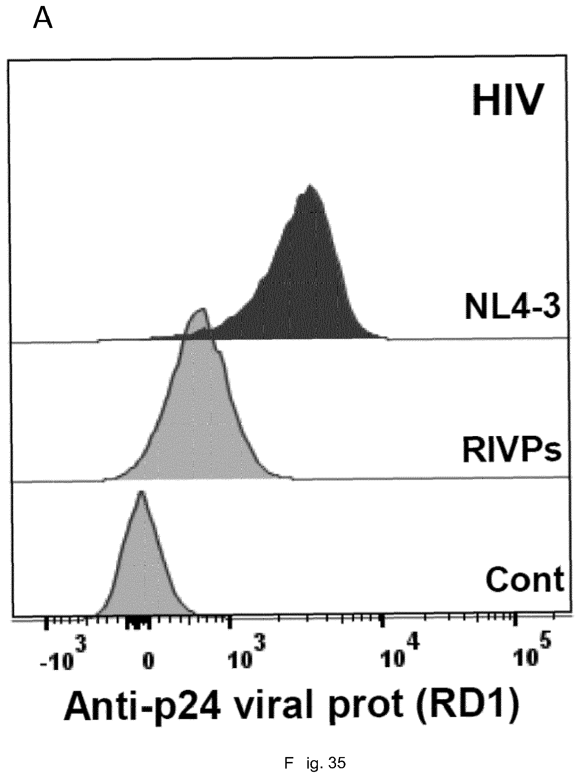

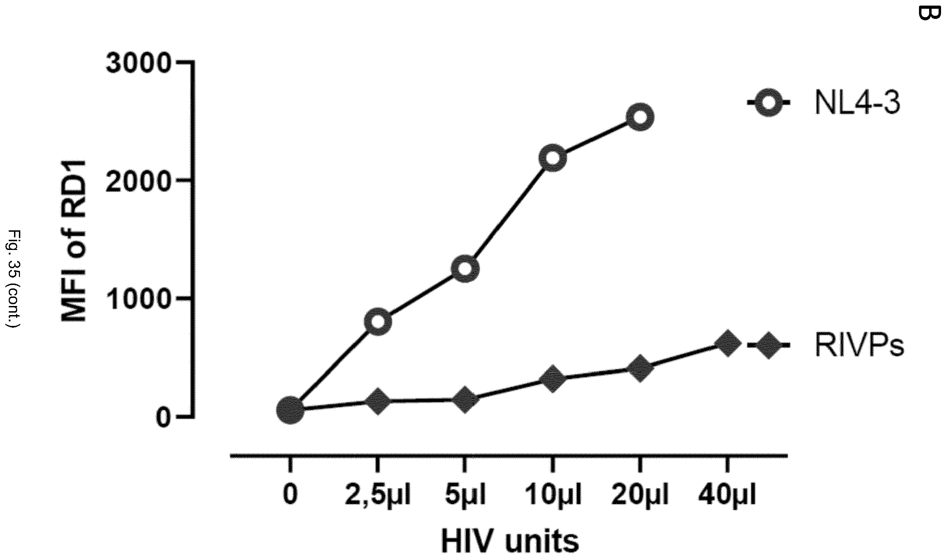

**Figure 35****.** Fig. 25. A. Detection of HIV-1_{NL4-3}b y a mS1 bead platform. B. The mean fluorescence intensity (MFI) at distinct viral concentrations is also shown.

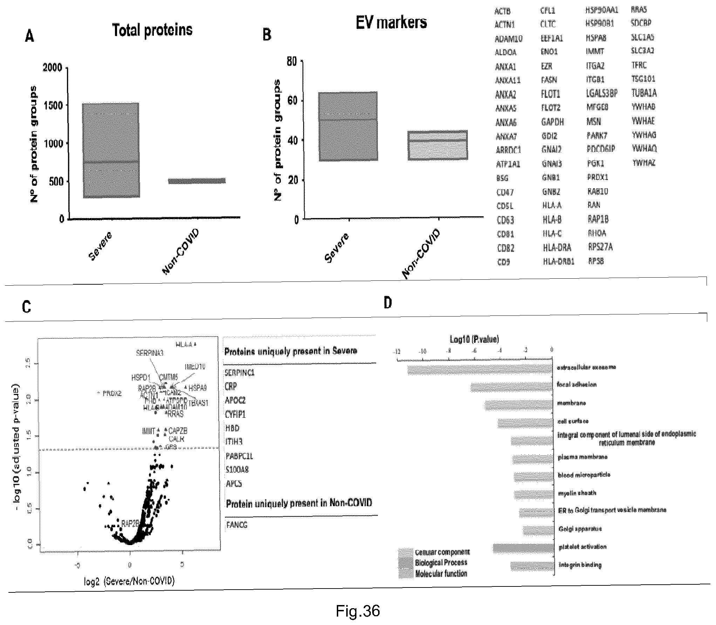

**Figure 36****.** Proteomic analysis of plasma-derived EVs from COVID-19 patients isolated by mS1 bead platform at the infection peak. Plasma from seven individuals from G1 and five from G3 at the peak of infection were used for isolation of EVs using mS1 mediated capture followed by LC-MS/MS mass spectrometry. A) Distribution of total number of proteins identified in isolated EVs. B) Distribution and identity of the EV markers in the proteome of the analyzed groups. Statistical significance was evaluated by a t-test (Mann Whitney). C) EVs protein abundance from severe COVID-19 and Non-COVID-19 individuals were compared. Volcano plot highlighting proteins found up and downregulated in Severe COVID-19 patients with statistical significance (q-val <0.05). Proteins listed aside correspond to proteins uniquely identified in each group. Proteins labeled in red were uniquely found in G1 while proteins labeled in green were uniquely detected in G3. D) Functional enrichment analysis by Gene Ontology of protein differentially present in mS1-captured EVs from Severe COVID-19 patients when compared to Non-COVID-19 individuals. Plots show the top enriched terms for Biological process, Cellular Components and Molecular Functions with an FDR <0.05.

### DETAILED DESCRIPTION OF THE INVENTION

The present invention relates to the provision of antibodies for the treatment an antiviral treatment and prevention.

Unless otherwise defined, all technical terms used herein have the same meaning as commonly understood by one of ordinary skill in the art to which this invention belongs.

All the embodiments and definitions disclosed in the context of one aspect of the invention are also applicable to the other aspects of the invention.

### Antibodies

In a first aspect, the invention relates to an antibody or an antigen-binding fragment thereof that specifically binds to sialic acid-binding Ig-like lectin 1 (CD169/Siglec-1), wherein the antibody comprises one VH region and one VL region and wherein the antibody is selected from the group consisting of:
1) the 1F5 antibody characterized in that:
   (i) the CDR1 of the VH region comprises the sequence of SEQ ID NO:1 or functionally equivalent variant thereof, the CDR2 of the VH region comprises the sequence of SEQ ID NO:2 or a functionally equivalent variant thereof and the CDR3 of the VH region comprises the sequence of SEQ ID NO: 3 or a functionally equivalent variant thereof, and
   (ii) the CDR1 of the VL region comprises the sequence of SEQ ID NO:5 or functionally equivalent variant thereof, the CDR2 of the VL region comprises the sequence WAS or a functionally equivalent variant thereof and the CDR3 of the VL region comprises the sequence of SEQ ID NO: 8 or functionally equivalent variant thereof or a functionally equivalent variant thereof;
2) the 3F1 antibody characterized in that:
   (i) the CDR1 of the VH region comprises the sequence of SEQ ID NO:40 or functionally equivalent variant thereof, the CDR2 of the VH region comprises the sequence of SEQ ID NO:41 or a functionally equivalent variant thereof and the CDR3 of the VH region comprises the sequence of SEQ ID NO: 44 or a functionally equivalent variant thereof, and
   (ii) the CDR1 of the VL region comprises the sequence of SEQ ID NO:46 or a functionally equivalent variant thereof, the CDR2 of the VL region comprises the sequence WAS or a functionally equivalent variant thereof and the CDR3 of the VL region comprises the sequence of SEQ ID NO: 50 or functionally equivalent variants thereof,
3) the 5B10 antibody comprising
   (i) the CDR1 of the VH region comprises the sequence of SEQ ID NO:88 or functionally equivalent variant thereof, the CDR2 of the VH region comprises the sequence of SEQ ID NO:89, and the CDR3 of the VH region comprises the sequence of SEQ ID NO: 90 or functionally equivalent variants thereof, and
   (ii) the CDR1 of the VL region comprises the sequence of SEQ ID NO:91, or a functionally equivalent variant thereof, the CDR2 of the VL region comprises the sequence FAS or a functionally equivalent variant thereof and the CDR3 of the VL region comprises the sequence of SEQ ID NO: 93 or a functionally equivalent variant thereof,
4) the 6G5 antibody comprising
   (i) the CDR1 of the VH region comprises the sequence of SEQ ID NO:116 or a functionally equivalent variant thereof, the CDR2 of the VH region comprises the sequence of SEQ ID NO:117 or a functionally equivalent variant thereof and the CDR3 of the VH region comprises the sequence of SEQ ID NO: 118 or a functionally equivalent variant thereof, and
   (ii) the CDR1 of the VL region comprises the sequence of SEQ ID NO:123, or a functionally equivalent variant thereof, the CDR2 of the VL region comprises the sequence of SEQ ID NO: 124 or a functionally equivalent variant thereof and the CDR3 of the VL region comprises the sequence of SEQ ID NO: 125 or a functionally equivalent variant thereof and
5) the 4E8 antibody is further characterized in that:
   (i) the CDR1 of the VH region comprises the sequence of SEQ ID NO:133 or a functionally equivalent variant thereof, the CDR2 of the VH region comprises the sequence of SEQ ID NO:134 or a functionally equivalent variant thereof and the CDR3 of the VH region comprises the sequence of SEQ ID NO: 135 or a functionally equivalent variant thereof, and
   (ii) the CDR1 of the VL region comprises the sequence of SEQ ID NO:140, or a functionally equivalent variant thereof, the CDR2 of the VL region comprises the sequence of SEQ ID NO: 141 or a functionally equivalent variant thereof and the CDR3 of the VL region comprises the sequence of SEQ ID NO: 142 or a functionally equivalent variant thereof.

As used herein, an "antibody" refers to an intact immunoglobulin of any isotype, or a fragment thereof that can compete with the intact antibody for specific binding to the target antigen, and includes, for instance, chimeric, humanized, fully human, and bispecific antibodies. An antibody is a species of an antigen binding protein. An intact antibody will generally comprise at least two full-length heavy chains and two full-length light chains, but in some instances can include fewer chains or shorted versions of the chains comprising only the variable regions. Antibodies can be derived solely from a single source, or can be "chimeric" that is, different portions of the antibody can be derived from two different antibodies as described further below. Unless otherwise indicated, the term "antibody" includes, in addition to antibodies comprising two full-length heavy chains and two full-length light chains, derivatives, variants, fragments, and muteins thereof, examples of which are described below.

The antibody according to the invention is a monoclonal antibody. The term "monoclonal antibody," as used herein, refers to an antibody that displays a single binding specificity and affinity for a particular epitope or a composition of antibodies in which all antibodies display a single binding specificity and affinity for a particular epitope. Accordingly, the term "human monoclonal antibody" refers to an antibody or antibody composition that display(s) a single binding specificity and which has variable and optional constant regions derived from human germline immunoglobulin sequences. In one embodiment, human monoclonal antibodies are produced by a hybridoma which includes a B cell obtained from a transgenic non-human animal, e.g., a transgenic mouse, having a genome comprising a human heavy chain transgene and a light chain transgene fused to an immortalized cell.

As used herein, "isotype" refers to the antibody class (e.g., IgG1, IgG2, IgG3, IgG4, IgM, IgA1, IgA2, IgD, and IgE antibody) that is encoded by the heavy chain constant region genes.

The term "antigen-binding fragment" of an antibody, as used herein, refers to one or more fragments of an antibody that retain the ability to specifically bind to an antigen (e.g., CD169/Siglec-1). Such "fragments" are, for example between about 8 and about 1500 amino acids in length, suitably between about 8 and about 745 amino acids in length, suitably about 8 to about 300, for example about 8 to about 200 amino acids, or about 10 to about 50 or 100 amino acids in length. It has been shown that the antigen-binding function of an antibody can be performed by fragments of a full-length antibody. Examples of binding fragments encompassed within the term "antigen-binding portion" of an antibody, include (i) a Fab fragment, a monovalent fragment consisting of the VL, VH, CL and CH1 domains; (ii) a F(ab')2 fragment, a bivalent fragment comprising two Fab fragments linked by a disulfide bridge at the hinge region; (iii) a Fd fragment consisting of the VH and CH1 domains; (iv) a Fv fragment consisting of the VL and VH domains of a single arm of an antibody, (v) a dAb fragment (Ward et al., (1989) Nature 341:544-546), which consists of a VH domain; and (vi) an isolated complementarity determining region (CDR) or (vii) a combination of two or more isolated CDRs which may optionally be joined by a synthetic linker. Furthermore, although the two domains of the Fv fragment, VL and VH, are coded for by separate genes, they can be joined, using recombinant methods, by a synthetic linker that enables them to be made as a single protein chain in which the VL and VH regions pair to form monovalent molecules (known as single chain Fv (scFv); see e.g., Bird et al. (1988) Science 242:423-426; and Huston et al. (1988) Proc. Natl. Acad. Sci. USA 85:5879-5883). Such single chain antibodies are also intended to be encompassed within the term "antigen-binding portion" of an antibody. These antibody fragments are obtained using conventional techniques known to those with skill in the art, and the fragments are screened for utility in the same manner as are intact antibodies. Antigen-binding portions can be produced by recombinant DNA techniques, or by enzymatic or chemical cleavage of intact immunoglobulins.

As used herein, the term "CDR" refers to the complementarity determining region within antibody variable sequences and corresponds to an antibody region having a structure that is complimentary to its target antigen or epitope. Other portions of variable domains, not interacting with antigen, are referred to as "framework regions" (FRs). A typical antibody molecule comprises a heavy chain variable region (VH) and a light chain variable region (VL), which are usually involved in antigen binding. The VH and VL regions can be further subdivided into regions of hypervariability, also known as "complementarity determining regions" ("CDR"), interspersed with regions that are more conserved, which are known as "framework regions" ("FR"). VH and VL domains have four framework regions (FRs) each positioned before, after, and between CDR regions. VH framework regions are referred to herein as FRH1, FRH2, FRH3, and FRH4 and VL framework regions are referred to herein as FRL1, FRL2, FRL3, and FRL4. FRs and CDRs of VH domains are typically in the order of FRH1-CDRH1-FRH2-CDRH2- FRH3-CDRH3-FRH4, from N- to C-terminus. FRs and CDRs of VL domains are typically in the order of FRL 1 -CDRL 1 -FRL2-CDRL2-FRL3 -CDRL3 -FRL4, from N- to C-terminus. The extent of the framework region and CDRs can be precisely identified using methodology known in the art, for example, by the Kabat definition, the IMGT definition, the Chothia definition, the AbM definition, and/or (e.g., and) the contact definition, all of which are well known in the art. See, e.g., Kabat, E.A., et al. (1991) Sequences of Proteins of Immunological Interest, Fifth Edition, U.S. Department of Health and Human Services, NIH Publication No.91-3242; IMGT(R), the international ImMunoGeneTics information system(R) www.imgt.org, Lefranc, M.-P. et al., Nucleic Acids Res., 27:209-212 (1999); Ruiz, M. et al., Nucleic Acids Res., 28:219-221 (2000); Lefranc, M.-P., Nucleic Acids Res., 29:207-209 (2001); Lefranc, M.-P., Nucleic Acids Res., 31:307-310 (2003); Lefranc, M.-P. et al., In Silico Biol., 5, 0006 (2004) [Epub], 5:45-60 (2005); Lefranc, M.-P. et al., Nucleic Acids Res., 33:D593-597 (2005); Lefranc, M.-P. et al., Nucleic Acids Res., 37:D1006-1012 (2009); Lefranc, M.-P. et al., Nucleic Acids Res., 43:D413-422 (2015); Chothia et al., (1989) Nature 342:877; Chothia, C. et al. (1987) J. Mol. Biol.196:901-917, Al-lazikani et al (1997) J. Molec. Biol.273:927-948; and Almagro, J. Mol. Recognit.17:132-143 (2004). See also bioinf.org.uk/abs. As used herein, a CDR may refer to the CDR defined by any method known in the art. Two antibodies having the same CDR means that the two antibodies have the same amino acid sequence of that CDR as determined by the same method, for example, the IMGT definition.

The expression "functionally equivalent variant", when referred to a polypeptide region, refers to: (i) a polypeptide resulting from the modification, deletion or insertion or one or more amino acids and which substantially preserves the activity of its reference polypeptide. Functionally equivalent variants contemplated in the context of the present invention, include polypeptides showing at least 60%, 70%, 80%, 85%, 90%, 92%, 94%, 96%, 98%, 99% of similarity or identity with sequences of the CDR or FR regions of the different anti-Siglec-1 antibody. The degree of identity or similarity between two polypeptides or two polynucleotides is determined by using computer-implemented algorithms and methods that are widely known in the art. The identity and similarity between two sequences of amino acids is preferably determined using the BLASTP algorithm. See Altschul S, et al., "BLAST Manual" (NCBI NLM NIH, Bethesda, MD, USA, 2001).

It will be understood that the preservation of the activity in the functionally equivalent variant will have to be determined by reference to the activity of the reference polypeptide. Accordingly, a functionally equivalent variant of a CDR will be defined as a polypeptide which, when present in an VH or an VL chain in combination with the other CDRs of the VH or VL chain and the corresponding VH or VL chain as the case may be results in an antibody which substantially preserves its ability of specifically binding to the antigen.

As used herein, the terms "specific binding," "selective binding," "selectively binds," and "specifically binds," refer to antibody binding to an epitope on a predetermined antigen with high affinity.

As used herein, the term "high affinity" for an IgG antibody refers to an antibody having a K_{D} of 10⁻⁸ M or less, more preferably 10⁻⁹ M or less and even more preferably 10⁻¹⁰ M or less for a target antigen. However, "high affinity" binding can vary for other antibody isotypes. For example, "high affinity" binding for an IgM isotype refers to an antibody having a K_{D} of 10⁻⁷ M or less, more preferably 10⁻⁸ M or less. Typically, the antibody (i) binds with an equilibrium dissociation constant (K_{D}) of approximately less than 10⁻⁷ M, such as approximately less than 10⁻⁸ M, 10⁻⁹ M or 10⁻¹⁰ M or even lower when determined by, e.g., surface plasmon resonance (SPR) technology in a BIACORE 2000 instrument using the predetermined antigen, e.g., recombinant CD169/Siglec-1 antigen, as the analyte and the antibody as the ligand, or Scatchard analysis of binding of the antibody to antigen positive cells, and (ii) binds to the predetermined antigen with an affinity that is at least two-fold greater than its affinity for binding to a non-specific antigen (e.g., BSA, casein) other than the predetermined antigen or a closely-related antigen. Accordingly, an antibody that "specifically binds to CD169/Siglec-1" refers to an antibody that binds to soluble or cell bound human CD169/Sigelc-1 with a K_{D} of 10⁻⁷ M or less, such as approximately less than 10⁻⁸ M, 10⁻⁹ M or 10⁻¹⁰ M or even lower.

The term "stabilized a ntibodies", as used herein, refer to antibodies that contain modifications within their sequences in order to improve stability, wherein said stability involves reduced oxidation, reduced deamidation, reduced isomerization and/or reduced aggregation:
Methionine residues in CDRs of antibodies can be oxidized, resulting in potential chemical degradation and consequent reduction in potency of the antibody. Accordingly, also provided are antibodies which have one or more methionine residues in the heavy and/or light chain CDRs replaced with amino acid residues which do not undergo oxidative degradation. Although methionine is the most susceptible residue to oxidation in mAbs, other residues such as tryptophan, cysteine, tyrosine and histidine have also been reported. Accordingly, in some embodiments, the antibodies according to the present invention contain a mutation in at least one CDR or in at least one FR in which an amino acid susceptible to oxidation is replaced by a different amino acid, preferably an amino acid which is not susceptible to oxidation.

Similarly, deamidation sites may be removed from antibodies described herein, particularly in the CDRs, which frequently occurs in asparagine residues. Accordingly, in some embodiments, the antibodies according to the present invention contain a mutation in at least one CDR or in at least one FR in which an amino acid susceptible to deamidation, preferably an asparagine, is replaced by a different amino acid, preferably an amino acid which is not susceptible to deamidation.

Other frequent chemical modifications of interest for mAbs are isomerization, which frequently occurs in aspartate residues Accordingly, in some embodiments, the antibodies according to the present invention contain a mutation in at least one CDR or in at least one FR in which an amino acid susceptible to isomerization, preferably an aspartate, is replaced by a different amino acid, preferably an amino acid which is not susceptible to isomerization.

On the other hand, IgGs can contain N-linked glycans in the variable domains, the so-called Fab glycans, in addition to the Fc glycans in the CH2 domains. These Fab glycans are acquired following introduction of N-glycosylation sites during somatic hypermutation and thereby constitute part of the physiological repertoire of antibodies. Furthermore, Fab glycans are usually localized close to antigen-binding sites and can influence antigen binding and contribute to affinity maturation. Conversely, some groups reported that glycans in the variable domains enhance antibody solubility and stability, while recent literature indicates that Fab glycans may trigger aggregation. Accordingly, in some embodiments, the antibodies according to the present invention contain a mutation in at least one CDR or in at least one FR in which a N-glycosylation site is replaced by an amino acid which cannot act N-glycosylation site. In other embodiments, the antibodies according to the present invention contain a mutation in at least one CDR or in at least one FR in which an N-glycosylation site is introduced by replacing an amino acid which cannot act N-glycosylation site.

In some embodiments, the 1F5 antibody according to the present invention is stabilized by replacing the methionine residue in the VH-CDR3 region of the heavy chain (SEQ ID NO:3), by a different amino acid. In an embodiment, the replacement of the methionine in the VH-CDR3 region is a methionine to leucine replacement (SEQ ID NO:4).

In some embodiments, the 1F5 antibody according to the present invention is stabilized by replacing the tryptophan residue in the VL-CDR2 region (having the sequence WAS) by a different amino acid. In an embodiment, the replacement of the tryptophan in the VL-CDR2 region is a tryptophan to phenylalanine replacement so that the VL-CDR2 region is the FAS sequence.

In some embodiments, the 3F1 antibody according to the present invention is stabilized by replacing a cysteine in the VH-CDR2 region (SEQ ID NO:41) by a different amino acid. In some embodiment, the replacement of the cysteine in the VH-CDR2 region is a cysteine to alanine (SEQ ID NO:42).

In some embodiments, the 3F1 antibody according to the present invention is stabilized by replacing the asparagine forming part of a asparagine-glycine motif having risk of deamidation in the VH-CDR2 region (SEQ ID NO: 41) by a different amino acid. In some embodiments, the replacement of the asparagine in the VH-CDR2 region is an asparagine to glycine replacement. (SEQ ID NO: 43)

In some embodiments, the 3F1 antibody according to the present invention is stabilized by replacing a tryptophan at risk of oxidation in the VH-CDR3 region (SEQ ID NO:44) by a different amino acid. In some embodiments, the replacement of the tryptophan in the VH-CDR3 region is a tryptophan to phenylalanine (SEQ ID NO:45).

In some embodiments, the 3F1 antibody according to the present invention is stabilized by replacing a tryptophan at risk of oxidation in the VL-CDR3 region (having the WAS sequence) by a different amino acid. In some embodiments, the replacement of the tryptophan in the VL-CDR3 region is a tryptophan to phenylalanine, resulting in the FAS sequence).

In another embodiment, the antibody or an antigen-binding fragment thereof according to the invention is the 1F5 antibody further characterized in that
(i) the FR1 region of the VH region comprises the sequence of SEQ ID NO:9 or functionally equivalent variant thereof, the FR2 region of the VH region comprises the sequence of SEQ ID NO:10 or functionally equivalent variant thereof, the FR3 region of the VH region comprises the sequence of SEQ ID NO:11 or functionally equivalent variant thereof and the FR4 of the VH region comprises the sequence of SEQ ID NO: 12 or functionally equivalent variants thereof, and
(ii) the FR1 region of the VL region comprises the sequence of SEQ ID NO:18 or functionally equivalent variant thereof, the FR2 region of the VH region comprises the sequence of SEQ ID NO:19 or functionally equivalent variant thereof, the FR3 region of the VH region comprises the sequence of SEQ ID NO:20 or functionally equivalent variant thereof and the FR4 of the VH region comprises the sequence of S EQ ID NO: 21 or functionally equivalent variants thereof; and

In another embodiment, the antibody or an antigen-binding fragment thereof according to the invention is the 3F1 antibody further characterized in that
(i) the FR1 region of the VH region comprises the sequence of SEQ ID NO:51 or functionally equivalent variant thereof, the FR2 region of the VH region comprises the sequence of SEQ ID NO:52 or functionally equivalent variant thereof, the FR3 region of the VH region comprises the sequence of SEQ ID NO:53 or functionally equivalent variant thereof and the FR4 of the VH region comprises the sequence of SEQ ID NO: 54 or a functionally equivalent variants thereof, and
(ii) the FR1 region of the VL region comprises the sequence of SEQ ID NO:60 or functionally equivalent variant thereof, the FR2 region of the VH region comprises the sequence of SEQ ID NO:61 or functionally equivalent variant thereof, the FR3 region of the VH region comprises the sequence of SEQ ID NO:62 or functionally equivalent variant thereof and the FR4 of the VH region comprises the sequence of SEQ ID NO: 63 or functionally equivalent variants thereof; and

In another embodiment, the antibody or an antigen-binding fragment thereof according to the invention is the 5B10 antibody further characterized in that:
(i) the FR1 region of the VH region comprises the sequence of SEQ ID NO:95 or functionally equivalent variant thereof, the FR2 region of the VH region comprises the sequence of SEQ ID NO:96 or functionally equivalent variant thereof, the FR3 region of the VH region comprises the sequence of SEQ ID NO:97 or functionally equivalent variant thereof and the FR4 of the VH region comprises the sequence of SEQ ID NO: 98 or functionally equivalent variants thereof, and
(ii) the FR1 region of the VL region comprises the sequence of SEQ ID NO:103 or functionally equivalent variant thereof, the FR2 region of the VH region comprises the sequence of SEQ ID NO:104 or functionally equivalent variant thereof, the FR3 region of the VH region comprises the sequence of SEQ ID NO:105 or functionally equivalent variant thereof and the FR4 of the VH region comprises the sequence of and SEQ ID NO: 106 or functionally equivalent variants thereof.

In another embodiment, the antibody or an antigen-binding fragment thereof according to the invention is the 6G5 antibody further characterized in that:
(i) the FR1 region of the VH region comprises the sequence of SEQ ID NO:119 or functionally equivalent variant thereof, the FR2 region of the VH region comprises the sequence of SEQ ID NO:120 or functionally equivalent variant thereof, the FR3 region of the VH region comprises the sequence of SEQ ID NO:121 or functionally equivalent variant thereof and the FR4 of the VH region comprises the sequence of SEQ ID NO: 122 or a functionally equivalent variants thereof, and
(ii) the FR1 region of the VL region comprises the sequence of SEQ ID NO:126 or functionally equivalent variant thereof, the FR2 region of the VH region comprises the sequence of SEQ ID NO:127 or functionally equivalent variant thereof, the FR3 region of the VH region comprises the sequence of SEQ ID NO:128 or functionally equivalent variant thereof and the FR4 of the VH region comprises the sequence of and SEQ ID NO: 129 or a functionally equivalent variant thereof.

In another embodiment, the antibody or an antigen-binding fragment thereof according to the invention is the 4E8 antibody further characterized in that:
(i) the FR1 region of the VH region comprises the sequence of SEQ ID NO:136 or functionally equivalent variant thereof, the FR2 region of the VH region comprises the sequence of SEQ ID NO:137 or functionally equivalent variant thereof, the FR3 region of the VH region comprises the sequence of SEQ ID NO:138 or functionally equivalent variant thereof and the FR4 of the VH region comprises the sequence of SEQ ID NO: 139 or a functionally equivalent variants thereof, and
(ii) the FR1 region of the VL region comprises the sequence of SEQ ID NO:143 or functionally equivalent variant thereof, the FR2 region of the VH region comprises the sequence of SEQ ID NO:144 or functionally equivalent variant thereof, the FR3 region of the VH region comprises the sequence of SEQ ID NO:145 or functionally equivalent variant thereof and the FR4 of the VH region comprises the sequence of and SEQ ID NO: 146 or a functionally equivalent variant thereof.

In another embodiment, the antibodies or antigen-binding fragments according to the invention are humanized.

A "humanized" antibody refers to an antibody in which some, most or all of the amino acids outside the CDR domains of a non-human antibody are replaced with corresponding amino acids derived from human immunoglobulins. In one embodiment of a humanized form of an antibody, some, most or all of the amino acids outside the CDR domains have been replaced with amino acids from human immunoglobulins, whereas some, most or all amino acids within one or more CDR regions are unchanged. Small additions, deletions, insertions, substitutions or modifications of amino acids are permissible as long as they do not abrogate the ability of the antibody to bind to a particular antigen. A "humanized" antibody retains an antigenic specificity similar to that of the original antibody. Humanized antibodies also include antibodies in which one or more residues of the human antibody are modified by one or more amino acid substitutions and/or one or more FR residues of the human protein are replaced by corresponding non-human residues. Humanized antibodies may also comprise residues which are found in neither the human antibody or in the non-human antibody. The term "humanized protein" also encompasses a super-humanized protein, e.g., as described in US7732578.

The humanized antibody may be of any isotype, and in particular is an IgG-type antibody, especially IgG1, IgG2, IgG3 or IgG4. In specific embodiments, the humanized antibody is an IgG1-type antibody.

In another embodiment, the antibody or an antigen-binding fragment thereof according to the invention is the 1F5 antibody further characterized in that:
(i) the FR1 region of the VH region comprises the sequence of SEQ ID NO:13 or functionally equivalent variant thereof, the FR2 region of the VH region comprises the sequence of SEQ ID NO:14 or functionally equivalent variant thereof, the FR3 region of the VH region comprises the sequence of SEQ ID NO:15, the sequence of SEQ ID NO:16 or functionally equivalent variant thereof and the FR4 of the VH region comprises the sequence of SEQ ID NO: 17 or a functionally equivalent variant thereof, and
(ii) the FR1 region of the VL region comprises the sequence of SEQ ID NO:22 or functionally equivalent variant thereof, the FR2 region of the VH region comprises the sequence of SEQ ID NO:23 or functionally equivalent variant thereof, the FR3 region of the VH region comprises the sequence of SEQ ID NO:24 or functionally equivalent variant thereof and the FR4 of the VH region comprises the sequence of SEQ ID NO: 25 or functionally equivalent variants thereof.

In another embodiment, the antibody or an antigen-binding fragment thereof according to the invention is the 3F1 antibody further characterized in that:
(i) the FR1 region of the VH region comprises the sequence of SEQ ID NO:55 or functionally equivalent variant thereof, the FR2 region of the VH region comprises the sequence of SEQ ID NO:56 or functionally equivalent variant thereof, the FR3 region of the VH region comprises the sequence of SEQ ID NO:57, the sequence of SEQ ID NO:58 or a functionally equivalent variant thereof and the FR4 of the VH region comprises the sequence of SEQ ID NO: 59 or a functionally equivalent variant thereof, and
(ii) the FR1 region of the VL region comprises the sequence of SEQ ID NO:64 or functionally equivalent variant thereof, the FR2 region of the VH region comprises the sequence of SEQ ID NO:65 or functionally equivalent variant thereof, the FR3 region of the VH region comprises the sequence of SEQ ID NO:66 or functionally equivalent variant thereof and the FR4 of the VH region comprises the sequence of SEQ ID NO: 67 or a functionally equivalent variant thereof.

In another embodiment, the antibody or an antigen-binding fragment thereof according to the invention is the 5B10 antibody further characterized in that:
(i) the FR1 region of the VH region comprises the sequence of SEQ ID NO:98 or functionally equivalent variant thereof, the FR2 region of the VH region comprises the sequence of SEQ ID NO:99 or functionally equivalent variant thereof, the FR3 region of the VH region comprises the sequence of SEQ ID NO:100 or functionally equivalent variant thereof and the FR4 of the VH region comprises the sequence of SEQ ID NO: 101 or a functionally equivalent variant thereof, and
(ii) the FR1 region of the VL region comprises the sequence of SEQ ID NO:106 or functionally equivalent variant thereof, the FR2 region of the VH region comprises the sequence of SEQ ID NO:107 or functionally equivalent variant thereof, the FR3 region of the VH region comprises the sequence of SEQ ID NO:108 or functionally equivalent variant thereof and the FR4 of the VH region comprises the sequence of SEQ ID NO: 109 or a functionally equivalent variant thereof.

In some embodiments, the antibodies according to the invention are engineered antibodies.

Engineered antibodies described herein include those in which modifications have been made to framework residues within V_{H} and/or V_{L}, e.g. to improve the properties of the antibody. Typically such framework modifications are made to decrease the immunogenicity of the antibody. For example, one approach is to "backmutate" one or more framework residues to the corresponding germline sequence. More specifically, an antibody that has undergone somatic mutation may contain framework residues that differ from the germline sequence from which the antibody is derived. Such residues can be identified by comparing the antibody framework sequences to the germline sequences from which the antibody is derived. To return the framework region sequences to their germline configuration, the somatic mutations can be "backmutated" to the germline sequence by, for example, site-directed mutagenesis or PCR-mediated mutagenesis. Such "backmutated" antibodies are also intended to be encompassed.

Another type of framework modification involves mutating one or more residues within the framework region, or even within one or more CDR regions, to remove T cell epitopes to thereby reduce the potential immunogenicity of the antibody. This approach is also referred to as "deimmunization" and is described in further detail in U.S. Patent Publication No. 20030153043 by Carr et al.

Another type of variable region modification is to mutate amino acid residues within the V_{H} and/or V_{L} CDR1, CDR2 and/or CDR3 regions to thereby improve one or more binding properties (e.g., affinity) of the antibody of interest. Site-directed mutagenesis or PCR-mediated mutagenesis can be performed to introduce the mutation(s) and the effect on antibody binding, or other functional property of interest, can be evaluated in in vitro or in vivo assays as described herein and provided in the Examples. Preferably conservative modifications (as discussed above) are introduced. The mutations may be amino acid substitutions, additions or deletions, but are preferably substitutions. Moreover, typically no more than one, two, three, four or five residues within a CDR region are altered.

In some embodiments, the 1F5 antibody comprises a VH region as defined in SEQ ID NO:31, 26, 27 or 28 or a VL region as defined in SEQ ID NO:29 or 30.

In some embodiments, the 3F1 antibody comprises a VH region as defined in SEQ ID NO:86, 68, 69 or 70 or a VL region as defined in SEQ ID NO:87, 77, 78, 80, 84 or 85.

In some embodiments, the 5B10 antibody comprises a VH region as defined in SEQ ID NO:113 or 111 or a VL region as defined in SEQ ID NO:114 or 112.

In some embodiments, the 6G5 antibody comprises a VH region as defined in SEQ ID NO:130 or a VL region as defined in SEQ ID NO:131.

In some embodiments, the 4E8 antibody comprises a VH region as defined in SEQ ID NO:147 or a VL region as defined in SEQ ID NO:148.

In some embodiments, the antibodies 1F5, 3F1, 5B10, 6G5 and 4E8 or antigen-binding fragments according to the invention are defined by the VH and VL regions as defined above or by a variant thereof comprising of an amino acid sequence at least 70 percent, at least 75 percent, at least 85 percent, at least 86 percent, at least 87 percent, at least 88 percent, at least 89 percent, at least 90 percent, at least 91 percent, at least 92 percent, at least 93 percent, at least 94 percent, at least 95 percent, at least 96 percent, at least 97 percent, at least 98 percent, at least 99 percent or 100 percent identical to the amino acid sequence of a light chain variable region selected from any one clone or type of Table 2; and a heavy chain variable region comprising of an amino acid sequence at least 70 percent, at least 75 percent, at least 85 percent, at least 86 percent, at least 87 percent, at least 88 percent, at least 89 percent, at least 90 percent, at least 91 percent, at least 92 percent, at least 93 percent, at least 94 percent, at least 95 percent, at least 96 percent, at least 97 percent, at least 98 percent, at least 99 percent or 100 percent identical to the amino acid sequence of a heavy chain variable region selected from any one corresponding clone or type of Table 4, wherein the antibody binds specifically to CD169/Siglec-1.

In another embodiment, the antibody or an antigen-binding fragment thereof according to the invention is the 1F5 antibody which is selected from the group consisting of:
(i) the antibody in which the VH region comprises the sequence of SEQ ID NO:26 and the VL region comprises the sequence of SEQ ID NO:29,
(ii) the antibody in which the VH region comprises the sequence of SEQ ID NO:31 and the VL region comprises the sequence of SEQ ID NO:32,
(iii) the antibody in which the VH region comprises the sequence of SEQ ID NO:27 and the VL region comprises the sequence of SEQ ID NO:30,
(iv) the antibody in which the VH region comprises the sequence of SEQ ID NO:28 and the VL region comprises the sequence of SEQ ID NO:30,
(v) the antibody in which the VH region comprises the sequence of SEQ ID NO:27 and the VL region comprises the sequence of SEQ ID NO:29
(vi) the antibody in which the VH region comprises the sequence of SEQ ID NO:28 and the VL region comprises the sequence of SEQ ID NO:29
(vii) the antibody in which the VH region comprises the sequence of SEQ ID NO:26 and the VL region comprises the sequence of SEQ ID NO:30,

In another embodiment, the antibody or an antigen-binding fragment thereof according to the invention is the 3F1 antibody which is selected from the group consisting of:
(i) the antibody in which the VH region comprises the sequence of SEQ ID NO:68 and the VL region comprises the sequence of SEQ ID NO:77
(ii) the antibody in which the VH region comprises the sequence of SEQ ID NO:86 and the VL region comprises the sequence of SEQ ID NO:87
(iii) the antibody in which the VH region comprises the sequence of SEQ ID NO:69 and the VL region comprises the sequence of SEQ ID NO:78
(iv) the antibody in which the VH region comprises the sequence of SEQ ID NO:70 and the VL region comprises the sequence of SEQ ID NO:78
(v) the antibody in which the VH region comprises the sequence of SEQ ID NO:69 and the VL region comprises the sequence of SEQ ID NO:80
(vi) the antibody in which the VH region comprises the sequence of SEQ ID NO:70 and the VL region comprises the sequence of SEQ ID NO:80
(vii) the antibody in which the VH region comprises the sequence of SEQ ID NO:69 and the VL region comprises the sequence of SEQ ID NO:77
(viii) the antibody in which the VH region comprises the sequence of SEQ ID NO:70 and the VL region comprises the sequence of SEQ ID NO:77
(ix) the antibody in which the VH region comprises the sequence of SEQ ID NO:68 and the VL region comprises the sequence of SEQ ID NO:84
(x) the antibody in which the VH region comprises the sequence of SEQ ID NO:68 and the VL region comprises the sequence of SEQ ID NO:85 or

In another embodiment, the antibody or an antigen-binding fragment thereof according to the invention is the 5B10 antibody which is selected from the group consisting of:
(i) the antibody wherein the VH region comprises the sequence SEQ ID NO:113 and the VL region comprises the sequence of SEQ ID NO: 114 or
(ii) the antibody wherein the VH region comprises the sequence SEQ ID NO: 111 and a VL region as defined in SEQ ID NO:114 or 112

In another embodiment, the antibody or an antigen-binding fragment thereof according to the invention is the 6G5 antibody wherein the VH region comprises the sequence SEQ ID NO: 130 and the VL region comprises the sequence of SEQ ID NO:131:
In another embodiment, the antibody or an antigen-binding fragment thereof according to the invention is the 4E8 antibody wherein the VH region comprises the sequence SEQ ID NO: 147 and the VL region comprises the sequence of SEQ ID NO:148:
In some embodiments, the antibodies 1F5, 3F1, 5B10, 6G5 and 4E8 or antigen-binding fragments according to the invention can also comprise at least a portion of an immunoglobulin constant region (Fc), typically that of a human immunoglobulin.

An "Fc region" (fragment crystallizable region) or "Fc domain" or "Fc" refers to the C-terminal region of the heavy chain of an antibody that mediates the binding of the immunoglobulin to host tissues or factors, including binding to Fc receptors located on various cells of the immune system (e.g., effector cells) or to the first component (Clq) of the classical complement system. Thus, an Fc region comprises the constant region of an antibody excluding the first constant region immunoglobulin domain (e.g., CH1 or CL). In IgG, IgA and IgD antibody isotypes, the Fc region comprises two identical protein fragments, derived from the second (C_{H2}) and third (C_{H3}) constant domains of the antibody's two heavy chains; IgM and IgE Fc regions comprise three heavy chain constant domains (C_{H} domains 2-4) in each polypeptide chain. For IgG, the Fe region comprises immunoglobulin domains Cgamma2 and Cgamma3 and the hinge between Cgamma1 and Cgamma2. Although the boundaries of the Fc region of an immunoglobulin heavy chain might vary, the human IgG heavy chain Fc region is usually defined to stretch from an amino acid residue at position C226 or P230 (or amino acid between these two amino acids) to the carboxy-terminus of the heavy chain, wherein the numbering is according to the EU index as in Kabat. The C_{H2} domain of a human IgG Fc region extends from about amino acid 231 to about amino acid 340, whereas the C_{H3} domain is positioned on C-terminal side of a C_{H2} domain in an Fc region, i.e., it extends from about amino acid 341 to about amino acid 447 of an IgG. As used herein, the Fc region may be a native sequence Fc, including any allotypic variant, or a variant Fc (e.g., a non-naturally occurring Fc). Fc may also refer to this region in isolation or in the context of an Fc-comprising protein polypeptide such as a "binding protein comprising an Fc region," also referred to as an "Fc fusion protein" (e.g., an antibody or immunoadhesin).

A "native sequence Fc region" or "native sequence Fc" comprises an amino acid sequence that is identical to the amino acid sequence of an Fc region found in nature. Native sequence human Fc regions include a native sequence human IgG1 Fc region; native sequence human IgG2 Fc region; native sequence human IgG3 Fc region; and native sequence human IgG4 Fc region as well as naturally occurring variants thereof. Native sequence Fc include the various allotypes of Fcs (see, e.g., Jefferis et al. (2009) mAbs 1:1).

A "hinge", "hinge domain" or "hinge region" or "antibody hinge region" refers to the domain of a heavy chain constant region that joins the CH1 domain to the CH2 domain and includes the upper, middle, and lower portions of the hinge (Roux et al. J. Immunol. 1998 161:4083). The hinge provides varying levels of flexibility between the binding and effector regions of an antibody and also provides sites for intermolecular disulfide bonding between the two heavy chain constant regions. As used herein, a hinge starts at Glu216 and ends at Gly237 for all IgG isotypes (Roux et al., 1998 J Immunol 161:4083).

The term "hinge" includes wildtype hinges as well as variants thereof (e.g., non-naturally-occurring hinges or modified hinges). For example, the term "IgG2 hinge" includes wildtype IgG2 hinge and variants having 1, 2, 3, 4, 5, 1-3, 1-5, 3-5 and/or at most 5, 4, 3, 2, or 1 mutations, e.g., substitutions, deletions or additions. Exemplary IgG2 hinge variants include IgG2 hinges in which 1, 2, 3 or all 4 cysteines (C219, C220, C226 and C229) are changed to another amino acid. In a specific embodiment, an IgG2 comprises a C219S substitution. In certain embodiments, a hinge is a hybrid hinge that comprises sequences from at least two isotypes. For example, a hinge may comprise the upper, middle or lower hinge from one isotype and the remainder of the hinge from one or more other isotypes. For example, a hinge can be an IgG2/IgG1 hinge, and may comprise, e.g., the upper and middle hinges of IgG2 and the lower hinge of IgG1. A hinge may have effector function or be deprived of effector function. For example, the lower hinge of wildtype IgG1 provides effector function.

The term "CH1 domain" refers to the heavy chain constant region linking the variable domain to the hinge in a heavy chain constant domain. As used herein, a CH1 domain starts at A118 and ends at V215. The term "CH1 domain" includes wildtype CH1 domains as well as variants thereof (e.g., non-naturally-occurring CH1 domains or modified CH1 domains). For example, the term "CH1 domain" includes wildtype CH1 domains and variants having 1, 2, 3, 4, 5, 1-3, 1-5, 3-5 and/or at most 5, 4, 3, 2, or 1 mutations, e.g., substitutions, deletions or additions. Exemplary CH1 domains include CH1 domains with mutations that modify a biological activity of an antibody, such as ADCC, CDC or half-life. Modifications to the CH1 domain that affect a biological activity of an antibody are provided herein.

The term "CH2 domain" refers to the heavy chain constant region linking the hinge to the CH3 domain in a heavy chain constant domain. As used herein, a CH2 domain starts at P238 and ends at K340. The term "CH2 domain" includes wildtype CH2 domains, as well as variants thereof (e.g., non-naturally-occurring CH2 domains or modified CH2 domains). For example, the term "CH2 domain" includes wildtype CH2 domains and variants having 1, 2, 3, 4, 5, 1-3, 1-5, 3-5 and/or at most 5, 4, 3, 2, or 1 mutations, e.g., substitutions, deletions or additions. Exemplary CH2 domains include CH2 domains with mutations that modify a biological activity of an antibody, such as ADCC, CDC or half-life. In certain embodiments, a CH2 domain comprises the substitutions A330S/P331S that reduce effector function. Other modifications to the CH2 domain that affect a biological activity of an antibody are provided herein.

The term "CH3 domain" refers to the heavy chain constant region that is C-terminal to the CH2 domain in a heavy chain constant domain. As used herein, a CH3 domain starts at G341 and ends at K447. The term "CH3 domain" includes wildtype CH3 domains, as well as variants thereof (e.g., non-naturally-occurring CH3 domains or modified CH3 domains). For example, the term "CH3 domain" includes wildtype CH3 domains and variants having 1, 2, 3, 4, 5, 1-3, 1-5, 3-5 and/or at most 5, 4, 3, 2, or 1 mutations, e.g., substitutions, deletions or additions. Exemplary CH3 domains include CH3 domains with mutations that modify a biological activity of an antibody, such as ADCC, CDC or half-life. Modifications to the CH3 domain that affect a biological activity of an antibody are provided herein.

In some embodiments, the antibody comprises an Fc domain. In an embodiment, the Fc domain has the same sequence or 99 percent or greater sequence similarity with a human IgG1 Fc domain. In an embodiment, the Fc domain has the same sequence or 99 percent or greater sequence similarity with a human IgG2 Fc domain. In an embodiment, the Fc domain has the same sequence or 99 percent or greater sequence similarity with a human IgG3 Fc domain. In an embodiment, the Fc domain has the same sequence or 99 percent or greater sequence similarity with a human IgG4 Fc domain. In an embodiment, the Fc domain is not mutated. In an embodiment, the Fc domain is mutated at the CH2-CH3 domain interface to increase the affinity of IgG for FcRn at acidic but not neutral pH (Dall'Acqua et al, 2006; Yeung et al, 2009). In an embodiment, the Fc domain has the same sequence as a human IgG1 Fc domain.

In some embodiments, the Fc region of the heavy chain is the sequence of SEQ ID NO:150. In some embodiments, the Fc region of the light chain is the sequence of SEQ ID NO:151.

### Nucleic acids encoding the antibodies of the invention, vectors and host cells containing said antibodies

In a second aspect, the invention relates to a nucleic acid encoding the antibodies or antigen binding fragments of the invention.

The terms "nucleic acid", "polynucleotide" and "nucleotide sequence", as used interchangeably herein, relate to any polymeric form of nucleotides of any length and composed of ribonucleotides or deoxyribonucleotides. The terms include both single-stranded and double-stranded polynucleotides, as well as modified polynucleotides (e.g., methylated, protected). Typically, the nucleic acid is a "coding sequence" which, as used herein, refers to a DNA sequence that is transcribed and translated into a polypeptide in a host cell when placed under the control of appropriate regulatory sequences. The boundaries of the coding sequence are determined by a start codon at the 5' (amino) terminus and a translation stop codon at the 3' (carboxy) terminus. A coding sequence can include, but is not limited to, prokaryotic sequences, cDNA from eukaryotic mRNA, genomic DNA sequences from eukaryotic (e.g., mammalian) DNA, and even synthetic DNA sequences. A transcription termination sequence will usually be located 3' to the coding sequence.

In some cases, a subject nucleic acid provides for production of an antibody of the present disclosure, e.g., in a mammalian cell. In other cases, a subject nucleic acid provides for amplification of the antibody-encoding nucleic acid.

In some embodiments, the nucleic acid according to the invention encodes the antibody or the antigen-binding fragment as a fusion protein which comprises a signal sequence located N-terminally with respect to the antibody or the antigen-binding fragment thereof.

The term "signal sequence", as used herein refers to a h ydrophobic sequence that mediates insertion of the protein through the membrane bounding the ER. Type I transmembrane proteins also comprise signal sequences. "Signal sequences," as used herein are amino-terminal hydrophobic sequences which are usually enzymatically removed following the insertion of part or all of the protein through the ER membrane into the lumen of the ER. Thus, it is known in the art that a signal precursor form of a sequence can be present as part of a precursor form of a protein, but will generally be absent from the mature form of the protein. When a protein is said to comprise a signal sequence, it is to be understood that, although a precursor form of the protein does contain the signal sequence, a mature form of the protein will likely not contain the signal sequence. Examples of signal peptides or sequences that are functional in mammalian host cells include the following: the signal sequence for interleukin-7 (IL-7) described in U.S. Pat. No. 4,965,195; the signal sequence for interleukin-2 receptor described in Cosman et al. ((1984), Nature 312:768); the interleukin-4 receptor signal peptide described in EP Patent No. 0 367 566; the type I interleukin-1 receptor signal sequence described in U.S. Pat. No. 4,968,607; the type II interleukin-1 receptor signal peptide described in EP Patent No. 0 460 846; the signal sequence of human IgG (SEQ ID NO:163); and the signal sequence of human growth hormone (SEQ ID NO:164) or the signal sequence as defined in SEQ ID NO:33 or in SEQ ID NO:115. Many other signal sequences are known in the art. In some embodiments, the signal peptide may be the naturally occurring signal peptide for a protein of interest or it may be a heterologous signal peptide.

A nucleotide sequence encoding any of the antibodies of the present invention can be operably linked to a transcriptional control element, e.g., a promoter, and enhancer, etc.

The term "operably linked", as used herein, means that the nucleotide sequence of interest is linked to the regulatory sequence(s) in a manner that allows for expression of the nucleotide sequence (e.g., in an in vitro transcription/translation system or in a host cell when the vector is introduced into the host cell). See Auer H, Nature Biotechnol. 2006; 24: 41-43.

In a preferred embodiment, a nucleic acid encoding any of the antibodies or the antigen-binding fragments is encoded as a fusion protein which comprises a signal sequence located N-terminally with respect to the antibody or the antigen-binding fragment thereof.

The terms "protein", "polypeptide" and "peptide" are used interchangeably herein to refer to a polymer of amino acid residues. The terms apply to amino acid polymers in which one or more amino acid residue is an artificial chemical mimetic of a corresponding naturally occurring amino acid, as well as to naturally occurring amino acid polymers and non-naturally occurring amino acid polymer.

In a third aspect the invention relates to an expression vector comprising the nucleic acid of the invention.

As used herein, "vector," "cloning vector," and "expression vector" refers to a nucleic acid molecule, linear or circular, that comprises a nucleic acid of the invention operably linked to additional segments that provide for its autonomous replication in a host cell or according to the expression cassette of the nucleic acid molecule. These are vehicles by which the host is transformed and expression of introduced sequences (eg, transcription and translation). Mean a vehicle in which a polynucleotide sequence (eg, a foreign gene) can be introduced into a host cell to facilitate Vectors include plasmids, phages, viruses and the like.

A nucleotide sequence encoding any of the antibodies of the invention can be present in an expression vector and/or a cloning vector. An expression vector can include a selectable marker, an origin of replication, and other features that provide for replication and/or maintenance of the vector. Suitable expression vectors include, e.g., plasmids, viral vectors, and the like.

Large numbers of suitable vectors and promoters are known to those of skill in the art; many are commercially available for generating a subject recombinant constructs. The following vectors are provided by way of example. Bacterial: pBs, phagescript, PsiX174, pBluescript SK, pBs KS, pNH8a, pNH16a, pNH18a, pNH46a (Stratagene, La Jolla, Calif., USA); pTrc99A, pKK223-3, pKK233-3, pDR540, and pRIT5 (Pharmacia, Uppsala, Sweden). Eukaryotic: pWLneo, pSV2cat, pOG44, PXR1, pSG (Stratagene) pSVK3, pBPV, pMSG and pSVL (Pharmacia).

Expression vectors generally have convenient restriction sites located near the promoter sequence to provide for the insertion of nucleic acid sequences encoding heterologous proteins. A selectable marker operative in the expression host may be present. Suitable expression vectors include, but are not limited to, viral vectors (e.g. viral vectors based on vaccinia virus; poliovirus; adenovirus; adeno-associated virus; SV40; herpes simplex virus; human immunodeficiency virus; a retroviral vector (e.g., Murine Leukemia Virus, spleen necrosis virus, and vectors derived from retroviruses such as Rous Sarcoma Virus, Harvey Sarcoma Virus, avian leukosis virus, human immunodeficiency virus, myeloproliferative sarcoma virus, and mammary tumor virus); and the like.

In a fourth aspect, the invention relates to a host cell comprising the nucleic acid of the invention or the expression vector of the invention.

The terms "host cell" means any cell of any organism that is modified, transformed, or manipulated by addition or modification of a gene, a DNA or RNA sequence, or protein or polypeptide. It also refers to the progeny of such cells. Host cells or genetically engineered cells of the present invention include isolated immune cells, such as T, NK, or NKT cells that contain the DNA or RNA sequences encoding a chimeric antigen receptor or chimeric antigen receptor complex and express the chimeric receptor on the cell surface. Isolated host cells and engineered cells may be used, for example, for enhancing an NK or NKT cell activity or a T lymphocyte activity, treatment of cancer, and treatment of infectious diseases.

### Pharmaceutical compositions

In a fifth aspect, the invention relates to a pharmaceutical composition comprising any of the host cells of the fourth aspect of the invention, and/or an antibody or an antigen-binding fragment thereof according to the first aspect of the invention and at least one pharmaceutically acceptable excipient, carrier, or diluent.

The term "pharmaceutical composition" is such a form that allows the biological activity of the active ingredient contained therein to be effective and has unacceptable toxicity for the subject to which the composition is administered. Refers to a preparation that does not contain additional ingredients.

The term "pharmaceutically acceptable carrier" refers to an ingredient of a pharmaceutical composition other than an active ingredient that is non-toxic to a subject. Pharmaceutically acceptable carriers include but are not limited to buffers, excipients, stabilizers or preservatives.

The expression "pharmaceutically acceptable excipient", as used herein, includes any and all solvents, dispersion media, coatings, antibacterial and antifungal agents, isotonic and absorption delaying agents that are physiologically compatible with the Siglec-1 inhibitors of the invention (e.g., SCE, mAbs, nucleic acids, vectors).

The term "subject", as used herein, refers to an individual, plant or animal, such as a human, a nonhuman primate (e.g., chimpanzees and other apes and monkey species); farm animals, such as birds, fish, cattle, sheep, pigs, goats and horses; domestic mammals, such as 20 dogs and cats; laboratory animals including rodents, such as mice, rats and guinea pigs. The term does not denote a particular age or sex. The term "subject" encompasses an embryo and a fetus. In a preferred embodiment, the subject is a human.

Pharmaceutical compositions and formulations as described herein can be prepared by mixing the active ingredients having the desired degree of purity with one or more optional pharmaceutically acceptable carriers (Remington's Pharmaceutical Sciences 22nd edition, 2012), in the form of lyophilized formulations or aqueous solutions. Pharmaceutically acceptable carriers are generally nontoxic to recipients at the dosages and concentrations employed, and include, but are not limited to: buffers such as phosphate, citrate, and other organic acids; antioxidants including ascorbic acid and methionine; preservatives (such as octadecyldimethylbenzyl ammonium chloride; hexamethonium chloride; benzalkonium chloride; benzethonium chloride; phenol, butyl or benzyl alcohol; alkyl parabens such as methyl or propyl paraben; catechol; resorcinol; cyclohexanol; 3-pentanol; and m-cresol); low molecular weight (less than about 10 residues) polypeptides; proteins, such as serum albumin, gelatin, or immunoglobulins; hydrophilic polymers such as polyvinylpyrrolidone; amino acids such as glycine, glutamine, asparagine, histidine, arginine, or lysine; monosaccharides, disaccharides, and other carbohydrates including glucose, mannose, or dextrins; chelating agents such as EDTA; sugars such as sucrose, mannitol, trehalose or sorbitol; salt-forming counterions such as sodium; metal complexes (e.g. Zn- protein complexes); and/or non-ionic surfactants such as polyethylene glycol (PEG). Exemplary pharmaceutically acceptable carriers herein further include insterstitial drug dispersion agents such as soluble neutral-active hyaluronidase glycoproteins (sHASEGP), for example, human soluble PH-20 hyaluronidase glycoproteins, such as rHuPH20 (HYLENEX^{®}, Baxter International, Inc.).

### Method of treatment using the antibodies and antigen-binding fragments thereof

The present invention provides methods for antiviral therapy based on the ability of the antibodies and antigen-binding fragments according to the invention to block the interaction between the gangliosides present in the vital envelopes and the CD169/Siglec1 protein found on the surface of antigen-expresing cells. comprising administering a therapeutic effective amount of host cells, antibody or antigen-binding fragment of the present invention.

Thus, in another aspect, the invention relates to the antibody of the invention or an antigen-binding fragment as well as to the nucleic acid, expression vector or host cell according to the invention for use in the prevention or in the treatment of an infection by an enveloped virus or for the treatment of a disease caused by a enveloped virus.

The terms "prevention," "preventing" and "prevent", as used herein, refer to inhibiting the inception or decreasing the occurrence of a disease in a subject. The prevention may be complete (e.g., the total absence of pathological cells in a subject). The prevention may also be partial, such as, for example, lowering the occurrence of pathological cells in a subject. Prevention also refers to a reduced susceptibility to a clinical condition. Within the context of the present invention, the terms "prevent," "preventing" and "prevention", refer specifically to averting or reducing the probability of SARS-CoV-2 infection, HIV infection, Ebola virus infection and/or Junin virus infection in a subject sustaining SARS-CoV-2, HIV, Ebola virus and/or Junin virus exposure.

As used herein, the term "treat", "treatment", or "treating" refers to the administration of the antibodies or antigen binding fragments of the invention, their compositions or combinations thereof for controlling the progression of a disease after its clinical signs have appeared. Control of the disease progression is understood to mean the beneficial or desired clinical results that include, but are not limited to, reduction of the symptoms, reduction of the duration of the disease, stabilization of pathological states (specifically to avoid additional deterioration), delaying the progression of the disease, improving the pathological state and remission (both partial and total). The control of progression of the disease also involves an extension of survival compared with the expected survival if treatment was not applied. Within the context of the present invention, the terms "treat" and "treatment" refer specifically to stopping or slowing the infection and destruction of healthy CD4+ T cells in a Coronavirus, HIV, Ebola and/or Junin virus infected subject. It also refers to the stopping and slowing of the onset of symptoms of the acquired immunodeficiency disease such as extreme low CD4+ T cell count and repeated infections by opportunistic pathogens. Beneficial or desired clinical results include, but are not limited to, an increase in absolute naive CD4+ T cell count (range 10-3520), an increase in the percentage of CD4+ T cell over total circulating immune cells (range 1-50%), or an increase in CD4+ T cell count as a percentage of normal CD4+ T cell count in an uninfected subject (range 1-161%). "Treatment" can also mean prolonging survival of the infected subject as compared to expected survival if the subject does not receive any Coronavirus, HIV, Ebola and/or Junin virus targeted treatment.

The term "enveloped virus" herein refers to a virus having a viral envelope, i.e. an outermost layer (generally a lipid bilayer) protecting the genetic material in their life-cycle when traveling between host cells. The viral envelopes are typically derived from portions of the host cell membranes (phospholipids and proteins), but include some viral glycoproteins. The glycoproteins may help viruses avoid the host immune system. Glycoproteins on the surface of the envelope serve to identify and bind to receptor sites on the host's membrane. The viral envelope then fuses with the host's membrane, allowing the capsid and viral genome to enter and infect the host. Generally, enveloped viruses also have a capsid (another protein layer), between the envelope and the genome. The lipid bilayer envelope is relatively sensitive to desiccation, heat, and amphiphiles such as soap and detergents, making enveloped viruses generally easier to sterilize than non-enveloped viruses, and having limited survival outside host environments. Enveloped viruses possess great adaptability and can change in a short time in order to evade the immune system. Enveloped viruses can cause persistent infections.

Enveloped viruses include:
- DNA viruses (such as Herpesviruses (belonging to the Herpesviridae family, which is a large family of DNA viruses comprising more than 130 known herpesviruses, some of them from mammals (among which nine herpesvirus types are known to primarily infect humans, including herpes simplex viruses 1 and 2 (HSV-1 and HSV-2), varicella zoster virus (or HHV-3), Epstein-Barr virus (EBV or HHV-4), and human cytomegalovirus (HCMV or HHV-5), birds, fish, reptiles, amphibians, and mollusks); Poxviruses (belonging to the Poxviridae family, comprising four genera that may infect humans: orthopoxvirus, parapoxvirus, yatapoxvirus, molluscipoxvirus); Hepadnaviruses (belonging to the family of Hepadanviridae, comprising Hepatitis B virus); Asfarviridae (a family of double-stranded DNA viruses comprising African swine fever virus (ASFV)); etc.);
- RNA viruses (such as Flaviviruses (belonging to the Flaviviridae family, which is a family of enveloped positive-strand RNA viruses which mainly infect mammals and birds, comprising flavivirus); Alphaviruses (belonging to the Togaviridae family and comprising e.g. Venezuelan equine encephalitis virus (VEEV), chikungunya virus (CHIKV), Ross River virus (RRV), o'nyong- nyong virus (ONNV), Mayaro virus (MAYV), eastern and western equine encephalitis, Sindbis viruses, etc.); Arenaviruses (belonging to the Arenaviridae family and comprising Gairo virus, Gbagroube virus, Ippy virus, Kodoko virus, Lassa virus, Lujo virus, Luna virus, Lunk virus, Lymphocytic choriomeningitis virus, Merino Walk virus, Menekre virus, Mobala virus, Morogoro virus, Mopeia virus, Wenzhou virus, Tacaribe virus, Amapari virus, Chapare virus, Flexal virus, Guanarito virus, Junin virus, Latino virus, Machupo virus, Oliveros virus, Parana virus, Patawa virus, Pichinde virus, Pirital virus, Sabia virus, Tacaribe virus, Tamiami virus, Whitewater Arroyo virus, etc.);; Coronaviruses (belonging to the Coronaviridae family); Hepatitis D; Orthomyxoviruses (belonging to the Orthomyxoviridae family, which is a family of negative-sense RNA viruses including seven genera: Alphainfluenzavirus, Betainfluenzavirus, Deltainfluenzavirus, Gammainfluenzavirus, Isavirus, Thogotovirus, and Quaranjavirus); Paramyxoviruses (belonging to the Paramyxoviridae family, which is a family of negative-strand RNA viruses); Rhabdovirus (belonging to the Rhabdoviridae family, which is a family of negative-strand RNA viruses); Bunyaviruses (belonging to the order of Bunyavirales, which is an order of segmented negative- strand RNA viruses); Filoviruses (belonging to the Filoviridae family); etc.) and
- Retroviruses (belonging to the Retroviridae family, which is a family of viruses that transcribes back its RNA genome into DNA with a reverse transcriptase).

In an embodiment, the enveloped virus is a virus that contains syalyllactose-containing gangliosides on its envelope. In some embodiments, the enveloped virus is a lentivirus, a virus of the filoviridae family, an Orthopneumovirus, a coronavirus or an arenavirus.

As used herein, the term "lentivirus" refers to a group (or genus) of complex retroviruses. Illustrative lentiviruses include, but are not limited to: HIV (human immunodeficiency virus; including HIV type 1, and HIV type 2); visna-maedi virus (VMV) virus; the caprine arthritis-encephalitis virus (CAEV); equine infectious anemia virus (EIAV); feline immunodeficiency virus (FIV); bovine immune deficiency virus (BIV); and simian immunodeficiency virus (SIV). In one embodiment, HIV based vector backbones (i.e., HIV cis-acting sequence elements) are preferred. In an embodiment, the lentivirus is the human immunodeficiency virus (HIV-1) and the disease caused by the virus infection is AIDS.

The term "HIV", as used herein, include HIV-1 and HIV-2, SHIV and SIV. "HIV- 1" means the human immunodeficiency virus type-1. HIV-1 includes, but is not limited to, extracellular virus particles and the forms of HIV-1 associated with HIV-1 infected cells. The HIV-1 virus may represent any of the known major subtypes (Classes A, B, C, D E, F, G and H) or outlying subtype (Group O) including laboratory strains and primary isolates. "HIV-2" means the human immunodeficiency virus type-2. HIV-2 includes, but is not limited to, extracellular virus particles and the forms of HIV-2 associated with HIV-2 infected cells. The term "SIV" refers to simian immunodeficiency virus which is an HIV-like virus that infects monkeys, chimpanzees, and other nonhuman primates. SIV includes, but is not limited to, extracellular virus particles and the forms of SIV associated with SIV infected cells.

The term "HIV infection", as used herein, refers to indications of the presence of the HIV virus in an individual including asymptomatic seropositivity, AIDS-related complex (ARC), and acquired immunodeficiency syndrome (AIDS).

The term "AIDS", as used herein, refers to the symptomatic phase of HIV infection, and includes both Acquired Immune Deficiency Syndrome (commonly known as AIDS) and "ARC," or AIDS-Related Complex. See Adler M, et al., Brit. Med. J. 1987; 294: 1145-1147. The immunological and clinical manifestations of AIDS are well known in the art and include, for example, opportunistic infections and cancers resulting from immune deficiency.

The term "filoviridae family" refers to a family of viruses with a single-stranded, unsegmented (-) sense RNA genome. Filoviruses can cause severe hemorrhagic fever in humans and nonhuman primates. So far, only two genuses of this virus family have been identified: Marburg and Ebola. Four species of Ebola virus have been identified: Cote d'Ivoire (CI), Sudan (S), Zaire (Z), and Reston (R). in an embodiment, the virus of the filoviridae family is the Ebola virus (EBOV),)

The term "Ebola virus", as used herein, refers to a linear, single-stranded, negative-sense RNA, comprising from 18,959 to 18,961 nucleotides in length and the causing agent of the Ebola virus disease (EVD). The Ebola virus is one of five known viruses within the genus Ebolavirus. The complete nucleotide sequence of the Ebola virus has the GenBank accession number KJ660346.2 (December 18, 2014).

In one embodiment, the diseases to be treated is an Ebola virus is Ebola virus infection

The term "Ebola virus infection", as used herein, refers to indications of the presence of the Ebola virus in an individual including asymptomatic seropositivity and EVD.

The term "Ebola virus disease" or "EVD", as used herein, refers to a severe, often fatal illness in humans caused by the Ebola virus. The virus is transmitted to people from wild animals and spreads in the human population through human-to-human transmission. The average EVD case fatality rate is around 50%.

The term "Orthopneumovirus", ", known by the common name of "Respiratory syncytial virus" or "RSV" are negative-sense, single-stranded RNA viruses. RSV is a member of the Pneumoviridae family. RSV primarily infects respiratory epithelial cells. There is a single RSV serotype with two major antigenic subgroups, A and B as outlined in Borchers et al (2013). The subtypes can be determined based on the reactivity of the F and G surface proteins to monoclonal antibodies. RSV infections cause can cause symptoms in primates, humans, rats, mice, cows, guinea pigs, ferrets, and hamsters. In one embodiment, the orthopneumovirus is the Respiratory syncytial virus (RSV)

The terms "Coronavirus", as used herein, refers to any member of the Coronaviridae viral family. The Coronaviridae family include single-stranded RNA viruses, about 120 nanometers in diameter. The family is divided in two subfamilies: Letovirinae and Coronavirinae. The Coronavirinae subfamily comprises the Alphacoronavirus (e.g., human coronavirus 229E (HCoV-229E), Betacoronavirus (e.g., human coronavirus HKU1, human coronavirus NL63 (HCoV-NL63, New Haven coronavirus), human coronavirus OC43 (HCoV-OC43), Middle East respiratory syndrome-related coronavirus (MERS-CoV or HCoV-EMC, the causative agent of MERS), severe acute respiratory syndrome coronavirus (SARS-CoV, the causative agent of SARS), severe acute respiratory syndrome coronavirus 2 (SARS-CoV-2, the causative agent of COVID-19), Deltacoronavirus, and Gammacoronavirus genus. Coronaviruses can also infect non-human subjects such as, for example, cattle (e.g., bovine coronavirus (BCV), cats (e.g., feline coronavirus (FCoV), dogs (e.g., canine coronavirus (CCoV), pigs (e.g., porcine coronavirus HKU15, porcine epidemic diarrhea virus (PED or PEDV), rabbits (e.g., rabbit enteric coronavirus), and birds (e.g., infectious bronchitis virus (IBV), turkey coronavirus (TCV)). There are more than 40 species of Coronaviruses. In an embodiment, the coronavirus is SARS-CoV-2.

In some embodiments, the infection is a SARS-CoV-2 infection.

The term "arenavirus" refers to members of the genus Arenavirius, a family of viruses whose members are generally associated with rodent-transmitted disease in humans, including Lymphocytic choriomeningitis virus (LCMV), Lassa virus, Junin virus, which causes Argentine hemorrhagic fever, Machupo virus, which causes Bolivian hemorrhagic fever, Guanarito virus, which causes Venezuelan hemorrhagic fever, and Sabia, which causes Brazilian hemorrhagic fever.

The terms "therapy" or "therapeutic", a used herein, refer to the use of the antibodies, antigen-binding fragments thaereof combinations and pharmaceutical compositions of the invention for either the prevention, inhibition of the progression or treatment of a disease including, but not limited to a lentivirus infection, an infection by a virus of the filoviridae family, an infection by an Orthopneumovirus, a coronavirus infection or an arenavirus infection.

### Conjugates and its uses

In an eighth aspect, the invention relates to a conjugate comprising:
- an antibody or antigen-binding domain as defined in any of the previous aspects and
- an agent of interest,
wherein the agent of interest is covalently coupled to the antibody or to the antigen-binding fragment thereof.

In a particular embodiment, the compound of interest from the conjugate is a detectable moiety or a compound with therapeutic activity.

The terms "detectable moiety", "label", "detectable label" or "detectable marker" are used interchangeably herein and refer to a detectable compound or composition which is conjugated directly or indirectly associated with the antibody so as to generate a "labeled" antibody. The label may be detectable by itself (e.g., radioisotope labels or fluorescent labels) or, in the case of an enzymatic label, may catalyze chemical alteration of a substrate compound or composition which is detectable.

Suitable optical reporters include, but are not limited to, fluorescent reporters and chemiluminescent groups, echogenic gases and radionuclides. A wide variety of fluorescent reporter dyes are known in the art.

Typically, the fluorophore is an aromatic or heteroaromatic compound and can be a pyrene, anthracene, naphthalene, acridine, stilbene, indole, benzindole, oxazole, thiazole, benzothiazole, cyanine, carbocyanine, salicylate, anthranilate, coumarin, fluorescein, rhodamine or other like compound. Other exemplary detectable labels include luminescent and bioluminescent markers (e.g., biotin, luciferase (e.g., bacterial, firefly, click beetle and the like), luciferin, and aequorin), radiolabels (e.g., 3H, 1251, 35S, 14C, or 32P), enzymes (e.g., galactosidases, glucorinidases, phosphatases (e.g., alkaline phosphatase), peroxidases (e.g., horseradish peroxidase), and cholinesterases), and calorimetric labels such as colloidal gold or colored glass or plastic (e.g., polystyrene, polypropylene, and latex) beads.

Suitable echogenic gases include, but are not limited to, a sulfur hexafluoride or perfluorocarbon gas, such as perfluoromethane, perfluoroethane, perfluoropropane, perfluorobutane, perfluorocyclobutane, perfluropentane, or perfluorohexane. Suitable non-metallic isotopes include, but are not limited to, ^{U}C, ¹⁴C, ¹³N, ¹⁸F, ¹²³I, ¹²⁴I, and ¹²⁵I. Suitable radioisotopes include, but are not limited to, "mTc, ⁹⁵Tc, ^{U1}In, ⁶²Cu, ⁶⁴C u, Ga, ⁶⁸Ga, and ¹⁵³Gd. Suitable paramagnetic metal ions include, but are not limited to, Gd(III), Dy(III), Fe(III), and Mn(II). Suitable X-ray absorbers include, but are not limited to, Re, Sm, Ho, Lu, Pm, Y, Bi, Pd, Gd, La, Au, Au, Yb, Dy, Cu, Rh, Ag, and Ir.

In some embodiments, the radionuclide is bound to a chelating agent or chelating agent-linker attached to the heme-binding molecule and/or composition. Suitable radionuclides for direct conjugation include, without limitation, ¹⁸F, ¹²⁴I, ¹²⁵I, ¹³¹I and mixtures thereof. Suitable radionuclides for use with a chelating agent include, without limitation, ⁴⁷Sc, ⁶⁴Cu, ⁶⁷C u, ⁸⁹Sr, ⁸⁶Y, ⁸⁷Y, ⁹⁰Y, ¹⁰⁵Rh, Ag, ^{m}In, ¹¹⁷mSn, ¹⁴⁹Pm, ¹⁵³Sm, ¹⁶⁶Ho, ¹⁷⁷Lu, ¹⁸⁶Re, ¹⁸⁸Re, ²¹¹At, ²¹²Bi, and mixtures thereof.

The conjugates according to the present invention containing detectable moieties can be used for the in vitro labelling of dendritic cells as well as for the in vivo detection of dendritic cells if the conjugates are administered to a patient.

The terms "therapeutic agent", "compound with therapeutic activity" or "drug" refer to refers to a pharmacologically active substance or substances that are used to treat, or prevent diseases or conditions. Drugs act by altering the physiology of a living organism, tissue, cell, or in vitro system that they are exposed to.

As mentioned above, the term "antibody-drug conjugate" or "ADC" refers to antibodies, or antigen-binding domains as the ones previously defined, also including antibody derivatives, that bind to CD169/Siglec-1 and are conjugated to a drug such as a cytotoxic, cytostatic, and/or therapeutic agent.

Therefore, the antibody conjugates of the invention relate to an antibody or antibody fragment thereof recombinantly fused or chemically conjugated (covalent bonds and chemically) to heterologous agents to create fusion proteins. The heterologous agent can be a polypeptide (or portion thereof, preferably a polypeptide of at least 10, at least 20, at least 30, at least 40, at least 50, at least 60, at least 70, at least 80, at least 90 or at least 100 amino acids), nucleic acid, small It may be a molecule (less than 1000 daltons) or an inorganic or organic compound. The fusion need not be direct and may occur through a linker sequence.

In an embodiment, the agent with therapeutic interest is a cytotoxic agent.

The term "cytotoxic agent" as used herein refers to a substance that inhibits or prevents the function of cells and/or causes destruction of cells. The term is intended to include radioactive isotopes (e.g., At, I, Y, Re, Re, Sm, TBi, -P, C, and radioactive isotopes of Lu), chemotherapeutic agents; and toxins such as small molecule toxins or enzymatically active toxins of bacterial, fungal, plant or animal origin, including synthetic analogs and derivatives thereof.

In another particular embodiment, the cytotoxic agent is a chemotherapeutic agent.

A "chemotherapeutic agent" is a chemical agent (e.g., compound or drug) useful in the treatment of cancer, regardless of mechanism of action. Classes of chemotherapeutic agents include, but are not limited to: alkylating agents, antimetabolites, spindle poison plant alkaloids, cytotoxic/antitumor antibiotics, topoisomerase inhibitors, antibodies, photosensitizers, and kinase inhibitors.

Chemotherapeutic agents include compounds used in targeted therapy and conventional chemotherapy. Examples of chemotherapeutic agents include, but are not limited to, Erlotinib (TARCEVA^{®}, Genentech/OSI Pharm.), Bortezomib (VELCADE^{®}, Millenium Pharm.), Fulvestrant (FASLODEX^{®}, Astrazeneca), Sutent (SU11248, Pfizer), Letrozole (FEMARA^{®}, Novartis), Imatinib mesylate (GLEEVEC^{®}, Novartis), PTK787/Z 222584 (Novartis), Oxaliplatin (Eloxatin^{®}, Sanofi), 5-FU (5-fluorouracil), Leucovorin, Rapamycin (Sirolimus, RAPAMUNE^{®}, Wyeth), Lapatinib (GSK572016, GlaxoSmithKline), Lonafarnib (SCH 66336), Sorafenib (BAY43-9006, Bayer Labs.), and Gefitinib (IRESSA^{®}, Astrazeneca), AG1478, AG1571 (SU 5271; Sugen), alkylating agents such as thiotepa and CYTOXAN^{®} cyclosphosphamide; alkyl sulfonates such as busulfan, improsulfan and piposulfan; aziridines such as benzodopa, carboquone, meturedopa, and uredopa; ethylenimines and methylamelamines including altretamine, triethylenemelamine, triethylenephosphoramide, triethylenethiophosphorarnide and trimethylomelarnine; acetogenins (especially bullatacin and bullatacinone); a camptothecin (including the synthetic analogue topotecan); bryostatin; callystatin; CC-1065 (including its adozelesin, carzelesin and bizelesin synthetic analogues); cryptophycins (particularly cryptophycin 1 and cryptophycin 8); dolastatin; duocarmycin (including the synthetic analogues, -2189 and CB1-TM1); eleutherobin; pancratistatin; a sarcodictyin; spongistatin; nitrogen mustards such as chlorambucil, chlornaphazine, cholophosphamide, estramustine, ifosfamide, mechlorethamine, mechlorethamine oxide hydrochloride, melphalan, novembichin, phenesterine, prednimustine, trofosfamide, uracil mustard; nitrosureas such as carmustine, chlorozotocin, fotemustine, lomustine, nimustine, and ranimnustine; antibiotics such as the enediyne antibiotics (e. g calicheamicin, especially calicheamicin gammall and calicheamicin omegall (Angew Chem Intl. Ed. Engl. (1994) 33:183-186); dynemicin, including dynemicin A; bisphosphonates, such as clodronate; an esperamicin; as well as neocarzinostatin chromophore and related chromoprotein enediyne antibiotic chromophores), aclacinoraysins, actinomycin, authramycin, azaserine, bleomycins, cactinomycin, carabicin, canninomycin, carzinophilin, chromomycinis, dactinomycin, daunorubicin, detorubicin, 6-diazo-5-oxo-L-norleucine, ADRIAMYCIN^{®} doxorubicin (including morpholino-doxorubicin, cyanomorpholino-doxorubicin, 2-pyrrolino-doxorubicin and deoxydoxorubicin), epirubicin, esorubicin, idarubicin, marcellomycin, mitomycins such as mitomycin C, mycophenolic acid, nogalamycin, olivomycins, peplomycin, potfiromycin, puromycin, quelamycin, rodorubicin, streptpnigrin, streptozocin, tubercidin, ubenimex, zinostatin, zorubicin; antimetabolites such as methotrexate and 5-fluorouracil (5-FU); folic acid analogues such as denopterin, methotrexate, pteropterin, trimetrexate; purine analogs such as fludarabine, 6-mercaptopurine, thiamiprine, thioguanine; pyrimidine analogs such as ancitabine, azacitidine, 6-azauridine, carmofur, cytarabine, dideoxyuridine, doxifluridine, enocitabine, floxuridine; androgens such as calusterone, dromostanolone propionate, epitiostanol, mepitiostane, testolactone; anti- adrenals such as anunoglutetWmide, mitotane, trilostane; folic acid replenisher such as frolinic acid; aceglatone; aldophosphamide glycoside; aminolevulinic acid; eniluracil; amsacrine; bestrabucil; bisanrrene; edatraxate; defofamine; demecolcine; diaziquone; elfornithine; elliptinium acetate; an epothilone; etoglucid; gallium nitrate; hydroxyurea; lentinan; lonidainine; maytansinoids such as maytansine and ansamitocins; mitoguazone; mitoxantrone; mopidanmol; nitraerine; pentostatin; phenamet; pirarubicin; losoxantrone; podophyllinic acid; 2- ethylhydrazide; procarbazine; PSK^{®} polysaccharide complex (JHS Natural Products, Eugene, OR); razoxane; rhizoxin; sizofiran; spirogermanium; tenuazonic acid; triaziquone; 2,2^2"-trichlorotriethylamine; trichothecenes (especially T-2 toxin, verracurin A, roridin A and anguidine); urethan; vindesine; dacarbazine; mannomustine; mitobronitol; mitolactol; pipobroman; gacytosine; arabinoside ("Ara-C"); cyclophosphamide; thiotepa; taxoids, e.g., TAXOL^{®} paclitaxel (Bristol- Myers Squibb Oncology, Princeton, N.J.), ABRAXANE^{™}C remophor-free, albumin-engineered nanoparticle formulation of paclitaxel (American Pharmaceutical Partners, Schaumberg, Illinois), and TAXOTERE^{®} doxetaxel (Rhone- Poulenc Rorer, Antony, France); chloranbucil; GEMZAR^{®} gemcitabine; 6-thioguanine; mercaptopurine; methotrexate; platinum analogs such as cisplatin and carboplatin; vinblastine; platinum; etoposide (VP-16); ifosfamide; mitoxantrone; vincristine; NAVELBINE^{®} vinorelbine; novantrone; teniposide; edatrexate; daunomycin; aminopterin; xeioda; ibandronate; CPT-11; topoisomerase inhibitor RFS 2000; difluorometlhylornithine (DMFO); retinoids such as retinoic acid; capecitabine; and pharmaceutically acceptable salts, acids or derivatives of any of the above.

Also included in the definition of "chemotherapeutic agent" are: (i) anti-hormonal agents that act to regulate or inhibit hormone action on tumors such as anti-estrogens and selective estrogen receptor modulators (SERMs), including, for example, tamoxifen (including NOLVADEX^{®}; tamoxifen citrate), raloxifene, droloxifene, 4-hydroxytamoxifen, trioxifene, keoxifene, LY 1 17018, onapristone, and FARESTON^{®} (toremifine citrate); (ii) aromatase inhibitors that inhibit the enzyme aromatase, which regulates estrogen production in the adrenal glands, such as, for example, 4(5)-imidazoles, aminoglutethimide, MEGASE^{®} (megestrol acetate), AROMASIN^{®} (exemestane; Pfizer), formestanie, fadrozole, RI VISor^{®} (vorozole), FEMARA^{®} (letrozole; Novartis), and ARIMIDEX^{®} (anastrozole; AstraZeneca); (iii) anti-androgens such as flutamide, nilutamide, bicalutamide, leuprolide, and goserelin; as well as troxacitabine (a 1 ,3-dioxolane nucleoside cytosine analog); (iv) protein kinase inhibitors such as ME inhibitors (WO 2007/044515); (v) lipid kinase inhibitors; (vi) antisense oligonucleotides, particularly those which inhibit expression of genes in signaling pathways implicated in aberrant cell proliferation, for example, PKC-alpha, Raf and H-Ras, such as oblimersen (GENASENSE^{®}, Genta Inc.); (vii) ribozymes such as VEGF expression inhibitors (e.g., ANGIOZYME^{®}) and HER2 expression inhibitors; (viii) vaccines such as gene therapy vaccines, for example, ALLOVECTIN^{®}, LEUVECTIN^{®}, and VAX1 D^{®}; PROLEUKIN^{®} rIL-2; topoisomerase 1 inhibitors such as LURTOTECAN^{®}; ABARELIX^{®} rmRH; (ix) antiangiogenic agents such as bevacizumab (AVASTIN^{®}, Genentech); and pharmaceutically acceptable salts, acids and derivatives of any of the above.

Protein kinase inhibitors include tyrosine kinase inhibitors which inhibit to some extent tyrosine kinase activity of a tyrosine kinase such as an ErbB receptor. Examples of tyrosine kinase inhibitors include EGFR- 10 targeted drugs such as: (i) antibodies which bind to EGFR, including MAb 579 (ATCC CRL HB 8506), MAb 455 (ATCC CRL HB8507), MAb 225 (ATCC CRL 8508), MAb 528 (ATCC CRL 8509) (see, US 4943533, Mendelsohn et al.) and variants thereof, such as chimerized 225 (C225 or Cetuximab; ERBITUX^{®}, Imclone) and reshaped human 225 (H225) (WO 96/40210, Imclone Systems Inc.); antibodies that bind type Π mutant EGFR (US 5212290); humanized and chimeric antibodies that bind EGFR (US 5891996); and human antibodies that bind EGFR, such as ABX-EGF (WO 98/50433); (ii) anti-EGFR antibody conjugated with a cyotoxic agent (EP 659439A2); and small molecules that bind to EGFR including ZD1839 or Gefitinib QRESSA^{™}; Astra Zeneca), Erlotinib HC1 (CP-358774, TARCEVA^{™}; Genentech/OSI) and AG1478, AG1571 (SU 5271; Sugen), quinazolines such as PD 153035,4-(3-chloroanilino) quinazoline, pyridopyrimidines, pyrirnidopyrirnidines, pyrrolopyrimidines, such as CGP 59326, CGP 60261 and CGP 62706, and pyrazolopyrimidines, 4- 20 (phenylamino)-7H-pyrrolo[2,3-dJ pyrimidines, curcumin (diferuloyl methane, 4,5-bis (4- fluoroanilino)phthaiimide), tyrphostines containing nitrothiophene moieties; PD-0183805 (Warner-Lambert) and antisense molecules (e.g., those that bind to ErbB-encoding nucleic acid).

In yet another embodiment, the ADC cytotoxic or chemotherapeutic agent of the present invention is an anti-tubulin agent. In more specific embodiments, the cytotoxic agent is selected from the group consisting of vinca alkaloids, podophyllotoxins, taxanes, baccatin derivatives, cryptophycin, maytansinoids, combretastatins, and dolastatin. In a more specific embodiment, the cytotoxic agent is vincristine, vinblastine, vindesine, vinorelbine, VP-16, camptothecin, paclitaxel, docetaxel, episilon A, episilon B, nocodazole, colchicine, cortisimide, estramustine, semadotine, discodermolide, maytansine DM-1, auristatin or other dolastatin derivatives, such as auristatin E or auristatin F, AEB, AEVB, AEFP, MMAE (monomethyl auristatin E), MMAF (monomethyl auristatin F), eruterobin or netropsin.

In addition, powerful chemotherapeutic agents such as CC-1065 analogs, calikiamycin, maytansine, dolastatin 10 analogs, lysoxin, and palytoxin can be linked to ADCs using conditionally stable linkers to form potent immunoconjugates.

In a particular embodiment, the chemotherapeutic agent is selected from lapatinib, tucatinib and neratinib.

In other embodiments, an antibody of the invention or fragment or variant thereof is conjugated to a therapeutic agent or drug moiety that modifies a given biological response. The therapeutic agent or drug moiety is not limited to classic chemotherapeutic agents. For example, the drug moiety may be a protein or polypeptide that possesses the desired biological activity. Such proteins include, for example, toxins such as abrin, ricin A, Pseudomonas exotoxin, cholera toxin, or diphtheria toxin; proteins such as tumor necrosis factor, α-interferon, β-interferon, nerve growth factor, platelet-derived growth factor Tissue plasminogen activator, apoptotic agents such as TNF-α, TNF-β, AIMI, AIMII, Fas ligand, and VEGf, thrombotic or antiangiogenic agents such as angiostatin or endostatin; or biological response modifiers For example, lymphokines (eg, interleukin-1 (IL-1), interleukin-2 (IL-2), interleukin-4 (IL-4), interleukin Kin-6 (IL-6), Interleukin-7 (IL-7), Interleukin-9 (IL-9), Interleukin-15 (IL-15), Interleukin-12 (IL-12), Granules Sphere macrophage colony stimulating factor (GM-CSF), and granulocyte colony stimulating factor (G-CSF)), or growth factors (eg, growth hormone (GH)).

Techniques for conjugating therapeutic moieties to antibodies are well known. The therapeutic moiety may be conjugated to the antibody by any method known in the art including, but not limited to, aldehyde / Schiff bond, sulfhydryl bond, acid labile bond, cis-aconityl (Aconityl) bond, hydrazone bond, and enzyme-degradable bond. The fusion with the therapeutic portion of the antibody need not be direct, but may occur through a linker sequence. Such linker molecules are generally known in the art.

In some embodiments, the conjugation of the drug to the antibody is performed by a linker that is sensitive to the environment (eg, the lysosomal environment due to the endosomal environment or pH or protease sensitivity).

In one embodiment, the linker is an acid labile hydrazone or hydrazide group that is hydrolyzed within the lysosome. In other embodiments, the drug can be conjugated to the antibody via other acid labile linkers such as cis-aconitic amides, orthoesters, acetals and ketals. Such linkers are relatively stable under neutral pH conditions, such as blood conditions, but are unstable at pH 5 or lower, ie, at a pH close to lysosomes.

In other embodiments, the drug binds to the antibody of the invention using a peptide spacer that is cleaved by intracellular proteases. Target enzymes include cathepsins B and D and plasmin, all of which are known to hydrolyze dipeptide drug derivatives resulting in the release of active drugs inside the target cell

In certain non-limiting embodiments of the present invention, the linker region between the drug and antibody portions of the ADC can be cleaved under certain conditions, with linker cleavage or hydrolysis. The drug moiety is released from the antibody moiety. Preferably, this linker is sensitive to cleavage or hydrolysis under intracellular conditions.

In one embodiment, the linker region between the drug and antibody portions of the ADC can be hydrolyzed if the pH changes by a certain value or exceeds a certain value.

In yet another specific embodiment, the linker is a disulfide linker. Various disulfide linkers are known in the art, including but not limited to SATA (N-succinimidyl-S-acetylthioacetate), SPDP (N-succinimidyl-3- (2-pyridyldithio) propionate), SPDB (N-succinimidyl-3- (2-pyridyldithio) butyrate) and SMPT (N-succinimidyl-oxycarbonyl-α-methyl-α- (2-pyridyldithio) toluene) Is included. SPDB and SMPT.

Conjugates comprising the antibodies or antigen-binding fragments thereo Antibodies Antibodies or fragments thereof can be produced by any method known in the art for antibody synthesis, particularly chemical synthesis, or preferably by recombinant expression techniques.

In another aspect, the invention relates to the use of the conjugates of the invention for the delivery of the agent of interest to a target cell that expresses Siglec-1. In a preferred embodiment, the target cell that expresses Siglec-1 is a dendritic cell.

The term "dendritic cell" or "DC" refers to any member of a diverse population of morphologically similar cell types found in lymphoid or non-lymphoid tissues. These cells are characterized by their distinctive morphology and high levels of surface MHC-class II expression. DCs can be isolated from a number of tissue sources. DCs have a high capacity for sensitizing MHC-restricted T cells, and are the only antigen-presenting cells (APCs) that can activate naive T-cells. The antigens may be self-antigens that are expressed during T cell development and tolerance, and foreign antigens that are present during normal immune processes. As used herein, an "activated DC" is a DC that has been pulsed with an antigen and is capable of activating an immune cell. The term "mature DC," as used herein, is defined as a dendritic cell that expresses high levels of MHC class II, CD80 (B7.1) and CD86 (B7.2) molecules. In contrast, immature dendritic cells express low levels of MHC class II, CD80 (B7.1) and CD86 (B7.2) molecules but have a great capacity to take up an antigen.

In another embodiment, the invention relates to a conjugate according to the invention for use in medicine.

In another embodiment, the invention relates to a conjugate according to the invention wherein the compound with therapeutic activity is a cytotoxic compound for use in a method of treatment of a disease which is associated with increased dendritic cell activity.

In one embodiment, the disease which is associated with increased dendritic cell activity is an autoimmune disease.

The term "autoimmune disease" as used herein is defined as a disorder that results from an autoimmune response. An autoimmune disease is the result of an inappropriate and excessive response to a self-antigen. Examples of autoimmune diseases include but are not limited to, Addision's disease, alopecia areata, ankylosing spondylitis, autoimmune hepatitis, autoimmune parotitis, Crohn's disease, diabetes (Type I), dystrophic epidermolysis bullosa, epididymitis, glomerulonephritis, Graves' disease, Guillain-Barr syndrome, Hashimoto's disease, hemolytic anemia, systemic lupus erythematosus, multiple sclerosis, myasthenia gravis, pemphigus vulgaris, psoriasis, rheumatic fever, rheumatoid arthritis, sarcoidosis, scleroderma, Sjogren's syndrome, spondyloarthropathies, thyroiditis, vasculitis, vitiligo, myxedema, pernicious anemia, and ulcerative colitis, among others.

In another embodiment, the invention relates to a conjugate according to the invention wherein the compound with therapeutic activity is an antigen for use in a method of treatment of a disease which requires an immune response against said antigen.

The term "antigen" (Ag) as used herein is defined as a molecule that provokes an immune response. This immune response may involve either antibody production, or the activation of specific immunologically-competent cells, or both. The skilled artisan will understand that any macromolecule, including virtually all proteins or peptides, can serve as an antigen. It is readily apparent that an antigen can be generated, synthesized or derived from a biological sample. Such a biological sample can include, but is not limited to, a tissue sample, a tumor sample, a cell or a biological fluid.

The present invention includes vaccine compositions for "pulsing" APCs (e.g., DCs) with an antigen, thereby activating the DC to stimulate an immune response. For example, an APC may be pulsed in vitro, e.g., by culture ex vivo in the presence of a nanoparticle conjugated to an antigen, or in vivo by exposure to a nanoparticle conjugated to an antigen. An APC can be "pulsed" in a manner that exposes the APC to an antigen for a time sufficient to promote presentation of that antigen on the surface of the APC. For example, an APC can be exposed to an antigen in a form of small peptide fragments, known as antigenic peptides; or APCs can be incubated with whole proteins or protein particles which are then ingested by the APCs. These whole proteins are digested into small peptide fragments by the APC and eventually carried to and presented on the APC surface. Antigen in peptide form may be exposed to the cell by "pulsing" techniques described herein. In one embodiment, carbon nanoparticles are used to facilitate the uptake of antigens by APCs.

Without wishing to be bound by theory, it is believed that the antigen in the form of a foreign or an autoantigen may be processed by an APC in order to retain the immunogenic form of the antigen. The immunogenic form of the antigen implies processing of the antigen through fragmentation to produce a form of the antigen that can be recognized by and stimulate immune cells, for example T cells. Preferably, such a foreign or an autoantigen is a protein which is processed into a peptide by the APC. The relevant peptide which is produced by the APC may be extracted and purified for use as an immunogenic composition. Peptides processed by the APC may also be used to induce tolerance to the proteins processed by the APC.

The antigen-activated APC, otherwise known as a "pulsed APC," is produced by exposure of the APC to an antigen either in vitro or in vivo. In the case where the APC is pulsed in vitro, the APC is plated on a culture dish and exposed to a nanoparticle conjugated to the antigen in a sufficient amount and for a sufficient period of time to allow the antigen to bind to the APC. The amount and time necessary to achieve binding of the antigen to the APC may be determined by using methods known in the art or otherwise disclosed herein. Other methods known to those of skill in the art (for example immunoassays or binding assays) may be used to detect the presence of antigen on the APC following exposure to the antigen.

Antigens useful in the vaccine compositions of the present invention may come from a variety of sources. The antigen may be derived from a virus, a fungus, or a bacterium.

The antigen may be a self-antigen or an antigen associated with a disease selected from the group consisting of an infectious disease, a cancer, or an autoimmune disease.

For an antigenic composition to be useful as a vaccine, the antigenic composition must induce an immune response to the antigen in a cell, tissue or mammal (e.g., a human). As used herein, an "immunological composition" may comprise an antigen (e.g., a peptide or polypeptide), a nucleic acid encoding an antigen (e.g., an antigen expression vector), or a cell expressing or presenting an antigen or cellular component. In particular embodiments, the antigenic composition comprises or encodes all or part of any antigen described herein, or an immunologically functional equivalent thereof. In other embodiments, the antigenic composition is in a mixture that comprises an additional immunostimulatory agent or nucleic acids encoding such an agent. Immunostimulatory agents include but are not limited to an additional antigen, an immunomodulator, an antigen-presenting cell or an adjuvant. In other embodiments, one or more of the additional agent(s) is covalently bonded to the antigen or an immunostimulatory agent, in any combination. In certain embodiments, the antigenic composition is conjugated to or comprises HLA anchor motif amino acids.

It is understood that an antigenic composition of the present invention may be made by a method that is well known in the field, including but not limited to chemical synthesis by solid-phase synthesis and purification away from the other products of the chemical reactions by HPLC, or production by the expression of a nucleic acid sequence (e.g., a DNA sequence) encoding a peptide or polypeptide comprising an antigen of the present invention in an in vitro translation system or in a living cell. In addition, an antigenic composition can comprise a cellular component isolated from a biological sample. Preferably, the antigenic composition is isolated and extensively dialyzed to remove one or more undesired small molecular weight molecules and/or lyophilized for more ready formulation into a desired vehicle. It is further understood that additional amino acids, mutations, chemical modification and the like, if any, that are made in a vaccine component will preferably not interfere substantially with the antibody recognition of the epitopic sequence.

A peptide or polypeptide corresponding to one or more antigenic determinants of the present invention should generally be at least five or six amino acid residues in length, and may contain up to about 10, about 15, about 20, about 25, about 30, about 35, about 40, about 45 or about 50 residues. A peptide sequence may be synthesized by methods known to those of ordinary skill in the art, such as, for example, peptide synthesis using automated peptide synthesis machines, such as those available from Applied Biosystems, Inc. (Foster City, Calif.). Longer peptides or polypeptides also may be prepared, e.g., by recombinant means.

In certain embodiments, a nucleic acid encoding an antigenic composition and/or a component described herein may be used, for example, to produce an antigenic composition in vitro or in vivo for the various compositions and methods of the present invention. For example, in certain embodiments, a nucleic acid encoding an antigen is carried by a vector in a recombinant cell. The nucleic acid may be expressed to produce a peptide or polypeptide comprising an antigenic sequence. The peptide or polypeptide may be secreted from the cell, or may be part of or within the cell. DNA coupled to CMNP can be detected by PCR to trace the presence of minute amounts of CMNP in ex vivo samples.

Tumor Associated Antigens. In one embodiment, a tumor antigen of the present invention comprises one or more antigenic cancer epitopes immunologically recognized by tumor-infiltrating lymphocytes (TIL) derived from a cancer tumor of a mammal. Malignant tumors express a number of proteins that can serve as target antigens for an immune attack. These molecules include, but are not limited to, tissue-specific antigens such as MART-1, tyrosinase and GP 100 in melanoma; and prostatic acid phosphatase (PAP) and prostate-specific antigen (PSA) in prostate cancer. Other target molecules belong to the group of transformation-related molecules such as the oncogene HER-2/Neu/ErbB-2. Yet another group of target antigens are onco-fetal antigens such as carcinoembryonic antigen (CEA). In B-cell lymphoma, the tumor-specific idiotype immunoglobulin constitutes a truly tumor-specific immunoglobulin antigen that is unique to the individual tumor. B-cell differentiation antigens such as CD 19, CD20 and CD37 are other candidates for target antigens in B-cell lymphoma. Some of these antigens (CEA, HER-2, CD 19, CD20, idiotype) have also been used as targets for passive immunotherapy with monoclonal antibodies.

Microbial Antigens. Microbial antigens may be viral, bacterial, or fungal in origin. Examples of infectious viruses include: Retroviridae (e.g., human immunodeficiency viruses, such as HIV-1 (also referred to as HTLV-III, LAV or-HTLV-III/LAV, or HIV-III); and other isolates, such as HIV-LP); Picornaviridae (e.g., polio viruses, hepatitis A virus; enteroviruses, human coxsackie viruses, rhinoviruses, echoviruses); Calciviridae (e.g., strains that cause gastroenteritis); Togaviridae (e.g., equine encephalitis viruses, rubella viruses); Flaviridae (e.g., dengue viruses, encephalitis viruses, yellow fever viruses); Coronaviridae (e.g., coronaviruses); Rhabdoviridae (e.g., vesicular stomatitis viruses, rabies viruses); Filoviridae (e.g., ebola viruses); Paramyxoviridae (e.g., parainfluenza viruses, mumps virus, measles virus, respiratory syncytial virus); Orthomyxoviridae (e.g., influenza viruses); Bungaviridae (e.g., Hantaan viruses, bunga viruses, phleboviruses and Nairo viruses); Arena viridae (hemorrhagic fever viruses); Reoviridae (e.g., reoviruses, orbiviruses and rotaviruses); Birnaviridae; Hepadnaviridae (Hepatitis B virus); Parvovirida (parvoviruses); Papovaviridae (papilloma viruses, polyoma viruses); Adenoviridae (most adenoviruses); Herpesviridae (herpes simplex virus (HSV) 1 and 2, varicella zoster virus, cytomegalovirus (CMV), herpes virus); Poxviridae (variola viruses, vaccinia viruses, pox viruses); and Iridoviridae (e.g., African swine fever virus); and unclassified viruses (e.g., the etiological agents of Spongiform encephalopathies, the agent of delta hepatitis (thought to be a defective satellite of hepatitis B virus), the agents of non-A, non-B hepatitis (class 1=internally transmitted; class 2=parenterally transmitted (i.e., Hepatitis C); Norwalk and related viruses, and astroviruses.

Examples of infectious bacteria include: Helicobacter pyloris, Borelia burgdorferi, Legionella pneumophilia, Mycobacteria sps (e.g., M. tuberculosis, M. avium, M. intracellulare, M. kansasii, M. gordonae ), Staphylococcus aureus, Neisseria gonorrhoeae, Neisseria meningitidis, Listeria monocytogenes, Streptococcus pyogenes (Group A Streptococcus ), Streptococcus agalactiae (Group B Streptococcus ), Streptococcus (viridans group), Streptococcus faecalis, Streptococcus bovis, Streptococcus (anaerobic sps.), Streptococcus pneumoniae, pathogenic Campylobacter sp., Enterococcus sp., Haemophilus influenzae, Bacillus anthracis, corynebacterium diphtheriae, corynebacterium sp., Erysipelothrix rhusiopathiae, Clostridium perfringens, Clostridium tetani, Enterobacter aerogenes, Klebsiella pneumoniae, Pasturella multocida, Bacteroides sp., Fusobacterium nucleatum, Streptobacillus moniliformis, Treponema Treponema pertenue, Leptospira, and Actinomyces israelli.

Examples of infectious fungi include: Cryptococcus neoformans, Histoplasma capsulatum, Coccidioides immitis, Blastomyces dermatitidis, Chlamydia trachomatis, Candida albicans. Other infectious organisms (i.e., protists) including: Plasmodium falciparum and Toxoplasma gondii.

In another embodiment, the invention relates to a conjugate according to the invention wherein the compound with therapeutic activity is a compound which promotes maturation of dendritic cells.

Suitable compounds which are capable of inducing maturation of dendritic cells include, without limitation, CD40 ligation, CpG oligodeoxyribonucleotides, IL-1, TNFalpha or TOLL-like receptor, bacterial lipoglycans and polysaccharides or agents which leado to the activation of the TRAF-6 or NF-kappaB intracellular pathways.

The above methods for the targeting of dendritic cells using the conjugates of the invention may be carried out ex vivo or in vivo, and independently with regard to antigen targeting, manipulation of DC maturation or inhibiting DC activity. For fully ex vivo methods, dendritic cells may be isolated from whole blood of an individual, and exposed ex vivo to the conjugates of the invention, before the dendritic cells are optionally isolated and then readministered to the individual. In vivo methods are also included, wherein the conjugate according to the invention is administered to the individual, such as in the form of a vaccine. Administration of a the conjugate may also be performed systemically or locally, and via any suitable route of administration.

### Methods for the capture of particles containing syalyllactose-containing gangliosides

In a ninth aspect, the invention relates to an in vitro method for the capture of particles containing a syalyllactose-containing ganglioside present in a sample which comprises:
(i) contacting the sample with a Siglec-1 protein variant that comprises the Siglec-1 V-set plus domain under conditions adequate for the binding of the particles containing a syalyllactose-containing ganglioside to the Siglec-1 protein variant and
(ii) recovering the Siglec-1 protein variant from the sample.

The term "sialyllactose", as used herein, refers to a molecule comprising a lactose moiety (P-D-galactopyranosyl-(I→4)-D-glucose) bound to a sialic acid moiety. The sialic acid may be coupled to the lactose by any possible position in the sialic acid and to any possible position in the lactose molecule. In one embodiment, the sialic acid is linked to the lactose by the hydroxyl group at position 2 of the sialic acid (the numbering of the sialic acid structure begins at the carboxylate carbon and continues around the chain). In another embodiment, the sialic acid is linked to the lactose by the hydroxyl groups at positions 3 or 6 in the lactose molecule. In a further embodiment, the "sialyllactose" is a 2,3-sialyl-lactose or a 2,6-sialyl-lactose.

The term "ganglioside" or "sialogangliosides", as used herein, refers to glycosphingolipids which contain several monosaccharide units per molecule. Examples of suitable monosaccharide units which can be contained in the gangliosides or ganglioside derivatives are D-galactose, N-acetyl D-galactosamine, glucose and N-acetylneuraminic acid. Particular preference is given to gangliosides which are derivatives of sphingosine (2-amino-4-octadecene-1,3-diol, sphing-4-enine), with, in particular, sugar residues being bonded on by way of the oxygen on the C-1 and a short (in particular C2-C18) fatty acid, which can be saturated or unsaturated, being bonded by way of the nitrogen on the C-2.

In an embodiment, the molecule containing sialic acid is a glycoprotein or a ganglioside containing α2,3-linked sialic acid.

According to some embodiments, the biological sample comprises a body fluid. Examples of body fluid include, without limitation, blood, urine, semen (seminal fluid), vaginal secretions, cerebrospinal fluid (CSF), synovial fluid, pleural fluid (pleural lavage), pericardial fluid, peritoneal fluid, amniotic fluid, saliva, nasal fluid, otic fluid, gastric fluid, and breast milk, feces, sweat, tears, nipple aspirates, or other secreted biological fluids. According to some embodiments, the body fluid is a circulating/secreted body fluid, e.g., blood (whole blood, serum, or plasma), CSF, or lymph. According to some embodiments, the biological sample comprises a body tissue (e.g., diseased tissue).

In a first step, the method for the capture of particles containing a syalyllactose-containing gangliosides present in a sample according to the invention comprises contacting the sample with a Siglec-1 protein variant that comprises the Siglec-1 V-set plus domain under conditions adequate for the binding of the particles containing a syalyllactose-containing ganglioside to the Siglec-1 protein variant.

The Siglec-1 protein variant for use in the capture method according to the present invention is characterized in that it comprises the Siglec-1 V-set plus domain under conditions adequate for the binding of the particles containing a syalyllactose-containing ganglioside to the Siglec-1 protein variant.

The term "V-set plus domain" from the Siglec-1 protein refers to a domain present in the N-terminal region of the Siglec-1 protein that that holds the sialic acid binding pocket. In one embodiment, the V-set plus domain comprises the amino acid sequence as defined in SEQ ID NO:153.

In another embodiment, the Siglec-1 protein variant further comprises at least one Ig-like C2 set domain derived from the human Siglec-1 protein. In another embodiment, the Ig-like C2 set domain comprises amino acids 139-233, amino acids 238-320, amino acids 326-405, amino acids 411-507, amino acids 511-593, amino acids 601-705, amino acids 708-785, amino acids 799-894, amino acids 898-977, amino acids 984-1083, amino acids 1085-1165, amino acids 1176-1248, amino acids 1259-1341, amino acids 1350-1442, amino acids 1445-1528 and/or amino acids 1536-1631 of the polypeptide shown in the UniProt database under accession number Q9BZZ2 (release date 14 December 2022).

In yet another embodiment, the Ig-like C2 set domain comprises the sequence of SEQ ID NO:154, 155 or 156. In yet another embodiment, the Siglec-1 protein variant comprises three Ig-like C2 set domains, which can be identical or different. In a still more preferred embodiment, the the Siglec-1 protein variant contains, in said order, the Ig-like C2 set domains defined as SEQ ID NO:154, 155 or 156.

In another embodiment, the Siglec-1 protein variant is provided as a fusion protein comprising a second moiety. In a preferred embodiment, the second moiety is an Fc.

The term "Fc" has been defined in the context of the antibodies of the invention and is equally applicable to the Siglec-1 protein variants used in the capture method according to the invention. In some embodiments, the Fc is formed by a first Fc domain, such as the domain comprising the sequence of SEQ ID NO: 157 and/or a second Fc domain, such as the domain comprising the sequence defined in SEQ ID NO:158. In some embodiments, the Fc comprises the first Fc domain as defined in SEQ ID NO: 157 and the second Fc domain as defined in SEQ ID NO:158. In some embodiments, the Fc region has the sequence of SEQ ID NO;166.

In another embodiment, the fusion protein comprising the Siglec-1 protein variant and a Fc comprises the following regions in this order from N- to C-terminus:
V-set domain-Ig-like domain 1-Ig like domain 2-Ig like domain 3- Fc 1st domain-Fc 2nd domain.

In another embodiment, the fusion protein comprising the Siglec-1 protein variant and a Fc comprises the amino acid sequence of SEQ ID NO:152 or 165.

It is understood that the part of the Siglec-1 protein variant comprising the V-set plus domain and, as the case may be, the one or more the one or more Ig-like C2 set domain and the Fc region can be linked or fused directly or, can be covalently bound to the Fc region by a linker. The linker may include one or more unnatural amino acids. The linker may be a cleavable linker or a non-cleavable linker. Optionally or in addition, the linker may be a flexible linker or an inflexible linker. The linker should be a length sufficiently long to allow the part of the Siglec-1 protein variant comprising the V-set plus domain and, as the case may be, the one or more the one or more Ig-like C2 set domain to be linked to the second moiety without steric hindrance from one another and sufficiently short to retain the intended activity of the fusion protein. The linker preferably is sufficiently hydrophilic to avoid or minimize instability of the fusion protein. The linker preferably is sufficiently hydrophilic to avoid or minimize insolubility of the fusion protein. In certain embodiments, the linker comprises a polypeptide linker that connects or fuses the part of the Siglec-1 protein variant comprising the V-set plus domain and, as the case may be, the one or more the one or more Ig-like C2 set domain and the Fc region. When a linker is employed, the linker may comprise hydrophilic amino acid residues, such as Gin, Ser, Gly, Glu, Pro, His and Arg. In certain embodiments, the linker is a peptide containing 1-25 amino acid residues, 1-20 amino acid residues, 2-15 amino acid residues, 3-10 amino acid residues, 3-7 amino acid residues, 4-25 amino acid residues, 4-20 amino acid residues, 4-15 amino acid residues, 4-10 amino acid residues, 5-25 amino acid residues, 5-20 amino acid residues, 5-15 amino acid residues, or 5-10 amino acid residues. Exemplary linkers include glycine and serine-rich linkers, e.g., (GGP)n or (GGGGS)n, where n is 1-5. In certain embodiments, the linker comprises, consists, or consists essentially of GGGGS (SEQ ID NO: 159) In certain embodiments, the linker comprises, consists, or consists essentially of GGGGSGGGGS (SEQ ID NO: 160). In certain embodiments, the linker comprises, consists, or consists essentially of GGGGS GGGGS GGGGS (SEQ ID NO: 161). In certain embodiments, the linker comprises, consists, or consists essentially of EPKSS (SEQ ID NO: 162).

In one embodiment, the Siglec-1 protein or the fusion protein containing the Siglec-1 protein variant is coupled to a support.

In some embodiments, the Siglec-1 protein or the fusion protein containing the Siglec-1 protein variant is attached to a support. Suitable supports or substrates include, but are not limited to, silica (e.g. glass beads, sand, diatomaceous earth) polysaccharides (e.g. dextran, cellulose, agarose), proteins (e.g. gelatin) and plastics (e.g. polystyrenes, polysulfones, polyethersulfones, polyesters, polyurethanes, polyacrylates and their activated and native amino and carboxyl derivatives). The protein can be bound to the substrates through standard chemical means, either directly, or through linkers such as C2 to C>20 linear and branched carbon chains, as well as the plastics, other proteins and polysaccharides listed above. For most synthetic purposes, C18 is the preferred upper limit but the chains can be added together for solubility reasons. Preferred linkers include: C2 to C18 dicarboxylates, diamines, dialdehydes, dihalides, and mixtures thereof (e.g. aminocarboxylates) in both native and activated form (e.g. disuccinimidyl suberimidate (DSS)). In some embodiments, one or more substrates can be used as linkers, alone or in combination with the substances listed as linkers. For example, dextran can be attached to sand, and additional linkers can then optionally be added to the dextran.

In some embodiments, the particles that are captured by the Siglec-1 protein variant are selected from the group consisting of a population of enveloped viruses, a e nveloped VLP populations and an exosome population.

The term "enveloped virus" has been described above in the context of the conjugates of the invention and applies equally to the methods for the capture using the claimed conjugates.

As used herein, the term "exosome" refers to an extracellular vesicle with a diameter between 20-300 nm (e.g, between 40-200 nm, 40-150nm). Exosomes comprise a membrane that encloses an internal space (i.e., lumen), and, in some instance, can be generated from a cell (e.g, producer cell) by direct plasma membrane budding or by fusion of the late endosome with the plasma membrane. In certain instances, an exosome comprises a scaffold/transmembrane moiety.

In an embodiment, the capture method according to the invention is carried out in a sample from a SARS-Cov2-infected individual. In yet another embodiment, the method is carried out on a an exosome-enriched sample.

Exosomes can be enriched using any suitable method including, for example, ultracentrifugation, pellet-down by centrifugation, fractionation by particle size, immunoprecipitation methods and the like.

In ultracentrifugation methods, exosomes are precipitated and isolated by ultracentrifugation of the sample (e.g., centrifugation at 100000 c g for 70 minutes for 2-3 minutes). In fractionation methods, a density gradient solution such as sucrose is used to fractionate on the basis of size or density (density gradient fractionation method).

Another method for isolation of exosomes is polymer precipitation, which is a simple method based on changing the solubility of the exosomes. See Bunggulawa et al. J. Nanobiotechnol. (2018) 16:81. Antimisiaris et al., Pharmaceutics. (2018) 10:218. Pellet-down by centrifugation comprises adding a reagent (polymer) to a sample and concentrating it by precipitating exosomes using a centrifuge.

Fractionation by particle size is a technique of putting samples through a plurality of filters (usually 2 to 3) to capture exosomes. In one example, where two filters are used, large particles can be removed using a first filter of about 200 nm pore size and exosomes can be captured using a second about 20 nm pore size filter.

Immunoprecipitation can recover exosomes comprising a specific protein, using magnetic beads on which an antibody against the protein is immobilized. In immunoprecipitation, it is possible to capture exosomes in which specific exosomal antigens (e.g., CD9, CD63, CD81, etc.) are present on the surface, and by using different antibodies, various exosomal membrane surface antigens are targeted.

In a second step, the method for the capture of particles containing a syalyllactose-containing gangliosides present in a sample according to the invention comprises recovering the Siglec-1 protein variant from the sample, which will carry over the particles containing a syalyllactose-containing gangliosides present in the sample.

The invention is described below by way of the following aspects
1. An antibody or an antigen-binding fragment thereof that specifically binds to sialic acid-binding Ig-like lectin 1 (CD169/Siglec-1), wherein the antibody comprises one VH region and one VL region and wherein the antibody is selected from the group consisting of:
   1) the 1F5 antibody characterized in that:
      (i) the CDR1 of the VH region comprises the sequence of SEQ ID NO:1 or functionally equivalent variant thereof, the CDR2 of the VH region comprises the sequence of SEQ ID NO:2 or a functionally equivalent variant thereof and the CDR3 of the VH region comprises the sequence of SEQ ID NO: 3 or 4 or a functionally equivalent variant thereof, and
      (ii) the CDR1 of the VL region comprises the sequence of SEQ ID NO:5 or functionally equivalent variant thereof, the CDR2 of the VL region comprises the sequence XAS, wherein X Is Trp of Phe, or a functionally equivalent variant thereof and the CDR3 of the VL region comprises the sequence of SEQ ID NO: 8 or functionally equivalent variant thereof or a functionally equivalent variant thereof; and
   2) the 3F1 antibody characterized in that:
      (i) the CDR1 of the VH region comprises the sequence of SEQ ID NO:40 or functionally equivalent variant thereof, the CDR2 of the VH region comprises the sequence of SEQ ID NO:41, the sequence of SEQ ID NO:42, the sequence of SEQ ID NO:43 or a functionally equivalent variant thereof and the CDR3 of the VH region comprises the sequence of SEQ ID NO: 44, the sequence of SEQ ID NO:45 or functionally equivalent variants thereof, and
      (ii) the CDR1 of the VL region comprises the sequence of SEQ ID NO:46 or a functionally equivalent variant thereof, the CDR2 of the VL region comprises the sequence XAS, wherein X Is Trp of Phe or a functionally equivalent variant thereof and the CDR3 of the VL region comprises the sequence of SEQ ID NO: 50 or functionally equivalent variants thereof, and
   3) the 5B10 antibody comprising
      (i) the CDR1 of the VH region comprises the sequence of SEQ ID NO:88 or functionally equivalent variant thereof, the CDR2 of the VH region comprises the sequence of SEQ ID NO:89, and the CDR3 of the VH region comprises the sequence of SEQ ID NO: 90 or functionally equivalent variants thereof, and
      (ii) the CDR1 of the VL region comprises the sequence of SEQ ID NO:91, or a functionally equivalent variant thereof, the CDR2 of the VL region comprises the sequence FAS or a functionally equivalent variant thereof and the CDR3 of the VL region comprises the sequence of SEQ ID NO: 93 or a functionally equivalent variant thereof,
   4) the 6G5 antibody comprising
      (i) the CDR1 of the VH region comprises the sequence of SEQ ID NO:116 or a functionally equivalent variant thereof, the CDR2 of the VH region comprises the sequence of SEQ ID NO:117 or a functionally equivalent variant thereof and the CDR3 of the VH region comprises the sequence of SEQ ID NO: 118 or a functionally equivalent variant thereof, and
      (ii) the CDR1 of the VL region comprises the sequence of SEQ ID NO:123, or a functionally equivalent variant thereof, the CDR2 of the VL region comprises the sequence of SEQ ID NO: 124 or a functionally equivalent variant thereof and the CDR3 of the VL region comprises the sequence of SEQ ID NO: 125 or a functionally equivalent variant thereof and
   5) the 4E8 antibody is further characterized in that:
      (i) the CDR1 of the VH region comprises the sequence of SEQ ID NO:133 or a functionally equivalent variant thereof, the CDR2 of the VH region comprises the sequence of SEQ ID NO:134 or a functionally equivalent variant thereof and the CDR3 of the VH region comprises the sequence of SEQ ID NO: 135 or a functionally equivalent variant thereof, and
      (ii) the CDR1 of the VL region comprises the sequence of SEQ ID NO:140, or a functionally equivalent variant thereof, the CDR2 of the VL region comprises the sequence of SEQ ID NO: 141 or a functionally equivalent variant thereof and the CDR3 of the VL region comprises the sequence of SEQ ID NO: 142 or a functionally equivalent variant thereof.
2. The antibody or an antigen-binding fragment thereof according to aspect 1, wherein:
   1) the 1F5 antibody is further characterized in that:
      (i) the FR1 region of the VH region comprises the sequence of SEQ ID NO:9 or functionally equivalent variant thereof, the FR2 region of the VH region comprises the sequence of SEQ ID NO:10 or functionally equivalent variant thereof, the FR3 region of the VH region comprises the sequence of SEQ ID NO:11 or functionally equivalent variant thereof and the FR4 of the VH region comprises the sequence of SEQ ID NO: 12 or functionally equivalent variants thereof, and
      (ii) the FR1 region of the VL region comprises the sequence of SEQ ID NO:18 or functionally equivalent variant thereof, the FR2 region of the VH region comprises the sequence of SEQ ID NO:19 or functionally equivalent variant thereof, the FR3 region of the VH region comprises the sequence of SEQ ID NO:20 or functionally equivalent variant thereof and the FR4 of the VH region comprises the sequence of SEQ ID NO: 21 or functionally equivalent variants thereof; and
   2) the 3F1 antibody is further characterized in that:
      (i) the FR1 region of the VH region comprises the sequence of SEQ ID NO:51 or functionally equivalent variant thereof, the FR2 region of the VH region comprises the sequence of SEQ ID NO:52 or functionally equivalent variant thereof, the FR3 region of the VH region comprises the sequence of SEQ ID NO:53 or functionally equivalent variant thereof and the FR4 of the VH region comprises the sequence of SEQ ID NO: 54 or a functionally equivalent variants thereof, and
      (ii) the FR1 region of the VL region comprises the sequence of SEQ ID NO:60 or functionally equivalent variant thereof, the FR2 region of the VH region comprises the sequence of SEQ ID NO:61 or functionally equivalent variant thereof, the FR3 region of the VH region comprises the sequence of SEQ ID NO:62 or functionally equivalent variant thereof and the FR4 of the VH region comprises the sequence of SEQ ID NO: 63 or functionally equivalent variants thereof; and
   3) the 5B10 antibody is further characterized in that:
      (i) the FR1 region of the VH region comprises the sequence of SEQ ID NO:94 or functionally equivalent variant thereof, the FR2 region of the VH region comprises the sequence of SEQ ID NO:95 or functionally equivalent variant thereof, the FR3 region of the VH region comprises the sequence of SEQ ID NO:96 or functionally equivalent variant thereof and the FR4 of the VH region comprises the sequence of SEQ ID NO: 97 or functionally equivalent variants thereof, and
      (ii) the FR1 region of the VL region comprises the sequence of SEQ ID NO:102 or functionally equivalent variant thereof, the FR2 region of the VH region comprises the sequence of SEQ ID NO:103 or functionally equivalent variant thereof, the FR3 region of the VH region comprises the sequence of SEQ ID NO:1 or functionally equivalent variant thereof and the FR4 of the VH region comprises the sequence of and SEQ ID NO: 105 or functionally equivalent variants thereof,
   4) the 6G5 antibody is further characterized in that:
      (i) the FR1 region of the VH region comprises the sequence of SEQ ID NO:119 or functionally equivalent variant thereof, the FR2 region of the VH region comprises the sequence of SEQ ID NO:120 or functionally equivalent variant thereof, the FR3 region of the VH region comprises the sequence of SEQ ID NO:121 or functionally equivalent variant thereof and the FR4 of the VH region comprises the sequence of SEQ ID NO: 122 or a functionally equivalent variants thereof, and
      (ii) the FR1 region of the VL region comprises the sequence of SEQ ID NO:126 or functionally equivalent variant thereof, the FR2 region of the VH region comprises the sequence of SEQ ID NO:127 or functionally equivalent variant thereof, the FR3 region of the VH region comprises the sequence of SEQ ID NO:128 or functionally equivalent variant thereof and the FR4 of the VH region comprises the sequence of and SEQ ID NO: 129 or a functionally equivalent variant thereof or
   5) the 4E8 antibody is further characterized in that:
      (i) the FR1 region of the VH region comprises the sequence of SEQ ID NO:136 or functionally equivalent variant thereof, the FR2 region of the VH region comprises the sequence of SEQ ID NO:137 or functionally equivalent variant thereof, the FR3 region of the VH region comprises the sequence of SEQ ID NO:138 or functionally equivalent variant thereof and the FR4 of the VH region comprises the sequence of SEQ ID NO: 139 or a functionally equivalent variants thereof, and
      (ii) the FR1 region of the VL region comprises the sequence of SEQ ID NO:143 or functionally equivalent variant thereof, the FR2 region of the VH region comprises the sequence of SEQ ID NO:144 or functionally equivalent variant thereof, the FR3 region of the VH region comprises the sequence of SEQ ID NO:145 or functionally equivalent variant thereof and the FR4 of the VH region comprises the sequence of and SEQ ID NO: 146 or a functionally equivalent variant thereof.
3. The antibody or an antigen-binding fragment thereof according to aspect 1 wherein the antibody of the antibody fragment is humanized.
4. The antibody or an antigen-binding fragment thereof according to aspect 3, wherein:
   1) the 1F5 antibody is further characterized in that:
      (i) the FR1 region of the VH region comprises the sequence of SEQ ID NO:13 or functionally equivalent variant thereof, the FR2 region of the VH region comprises the sequence of SEQ ID NO:14 or functionally equivalent variant thereof, the FR3 region of the VH region comprises the sequence of SEQ ID NO:15, the sequence of SEQ ID NO:16 or functionally equivalent variant thereof and the FR4 of the VH region comprises the sequence of SEQ ID NO: 17 or a functionally equivalent variant thereof, and
      (ii) the FR1 region of the VL region comprises the sequence of SEQ ID NO:22 or functionally equivalent variant thereof, the FR2 region of the VH region comprises the sequence of SEQ ID NO:23 or functionally equivalent variant thereof, the FR3 region of the VH region comprises the sequence of SEQ ID NO:24 or functionally equivalent variant thereof and the FR4 of the VH region comprises the sequence of SEQ ID NO: 25 or functionally equivalent variants thereof; and
   2) the 3F1 antibody is further characterized in that:
      (i) the FR1 region of the VH region comprises the sequence of SEQ ID NO:55 or functionally equivalent variant thereof, the FR2 region of the VH region comprises the sequence of SEQ ID NO:56 or functionally equivalent variant thereof, the FR3 region of the VH region comprises the sequence of SEQ ID NO:57, the sequence of SEQ ID NO:58 or a functionally equivalent variant thereof and the FR4 of the VH region comprises the sequence of SEQ ID NO: 59 or a functionally equivalent variant thereof, and
      (ii) the FR1 region of the VL region comprises the sequence of SEQ ID NO:64 or functionally equivalent variant thereof, the FR2 region of the VH region comprises the sequence of SEQ ID NO:65 or functionally equivalent variant thereof, the FR3 region of the VH region comprises the sequence of SEQ ID NO:66 or functionally equivalent variant thereof and the FR4 of the VH region comprises the sequence of SEQ ID NO: 67 or a functionally equivalent variant thereof; and
   3) the 5B10 antibody is further characterized in that:
      (i) the FR1 region of the VH region comprises the sequence of SEQ ID NO:98 or functionally equivalent variant thereof, the FR2 region of the VH region comprises the sequence of SEQ ID NO:99 or functionally equivalent variant thereof, the FR3 region of the VH region comprises the sequence of SEQ ID NO:100 or functionally equivalent variant thereof and the FR4 of the VH region comprises the sequence of SEQ ID NO: 101 or a functionally equivalent variant thereof, and
      (ii) the FR1 region of the VL region comprises the sequence of SEQ ID NO:106 or functionally equivalent variant thereof, the FR2 region of the VH region comprises the sequence of SEQ ID NO:107 or functionally equivalent variant thereof, the FR3 region of the VH region comprises the sequence of SEQ ID NO:108 or functionally equivalent variant thereof and the FR4 of the VH region comprises the sequence of SEQ ID NO: 109 or a functionally equivalent variant thereof.
5. The antibody or an antigen-binding fragment thereof according to any preceding aspect wherein:
   1) the 1F5 antibody comprises a VH region as defined in SEQ ID NO:31, 26, 27 or 28 or a VL region as defined in SEQ ID NO:29 or 30,
   2) the 13F1 antibody comprises a VH region as defined in SEQ ID NO:86, 68, 69 or 70 or a VL region as defined in SEQ ID NO:87, 77, 78, 80, 84 or 85,
   3) the 5B10 antibody comprises a VH region as defined in SEQ ID NO:112 or 110 or a a VL region as defined in SEQ ID NO:113 or 111
   4) the 6G5 antibody comprises a VH region as defined in SEQ ID NO:130 or a VL region as defined in SEQ ID NO:131 or
   5) the 4E8 antibody comprises a VH region as defined in SEQ ID NO:147 or a VL region as defined in SEQ ID NO:148.
6. The antibody or an antigen-binding fragment thereof according to any preceding aspect wherein:
   1) the 1F5 antibody is selected from the group consisting of:
      (i) the antibody in which the VH region comprises the sequence of SEQ ID NO:26 and the VL region comprises the sequence of SEQ ID NO:29,
      (ii) the antibody in which the VH region comprises the sequence of SEQ ID NO:31 and the VL region comprises the sequence of SEQ ID NO:32,
      (iii) the antibody in which the VH region comprises the sequence of SEQ ID NO:27 and the VL region comprises the sequence of SEQ ID NO:30,
      (iv) the antibody in which the VH region comprises the sequence of SEQ ID NO:28 and the VL region comprises the sequence of SEQ ID NO:30,
      (v) the antibody in which the VH region comprises the sequence of SEQ ID NO:27 and the VL region comprises the sequence of SEQ ID NO:29
      (vi) the antibody in which the VH region comprises the sequence of SEQ ID NO:28 and the VL region comprises the sequence of SEQ ID NO:29
      (vii) the antibody in which the VH region comprises the sequence of SEQ ID NO:26 and the VL region comprises the sequence of SEQ ID NO:30,
   2) the 3F1 antibody is selected from the group consisting of:
      (i) the antibody in which the VH region comprises the sequence of SEQ ID NO:68 and the VL region comprises the sequence of SEQ ID NO:77
      (ii) the antibody in which the VH region comprises the sequence of SEQ ID NO:86 and the VL region comprises the sequence of SEQ ID NO:87
      (iii) the antibody in which the VH region comprises the sequence of SEQ ID NO:69 and the VL region comprises the sequence of SEQ ID NO:78
      (iv) the antibody in which the VH region comprises the sequence of SEQ ID NO:70 and the VL region comprises the sequence of SEQ ID NO:78
      (v) the antibody in which the VH region comprises the sequence of SEQ ID NO:69 and the VL region comprises the sequence of SEQ ID NO:80
      (vi) the antibody in which the VH region comprises the sequence of SEQ ID NO:70 and the VL region comprises the sequence of SEQ ID NO:80
      (vii) the antibody in which the VH region comprises the sequence of SEQ ID NO:69 and the VL region comprises the sequence of SEQ ID NO:77
      (viii) the antibody in which the VH region comprises the sequence of SEQ ID NO:70 and the VL region comprises the sequence of SEQ ID NO:77
      (ix) the antibody in which the VH region comprises the sequence of SEQ ID NO:68 and the VL region comprises the sequence of SEQ ID NO:84
      (x) the antibody in which the VH region comprises the sequence of SEQ ID NO:68 and the VL region comprises the sequence of SEQ ID NO:85 or
   3) the 5B10 antibody is selected from the group consisting of:
      (i) the antibody wherein the VH region comprises the sequence SEQ ID NO:112 and the VL region comprises the sequence of SEQ ID NO: 113 or
      (ii) the antibody wherein the VH region comprises the sequence SEQ ID NO: 110 or a VL region as defined in SEQ ID NO:113 or 111
   4) the 6G5 antibody comprises a VH region as defined in SEQ ID NO:130 and a VL region as defined in SEQ ID NO:131 or
   5) the 4E8 antibody comprises a VH region as defined in SEQ IDN O:147 and a VL region as defined in SEQ ID NO:148.
7. The antibody or antigen-binding fragment thereof of any one of aspects 1 to 6, wherein the antibody or antigen-binding fragment thereof is an IgG, a Fab, a F(ab'), a F(ab')₂, a diabody or an ScFv.
8. A nucleic acid encoding antibody or an antigen-binding fragment thereof according to any of aspects 1 to 5.
9. The nucleic acid according to aspect 8 wherein the antibody or the antigen-binding fragment thereof is encoded as a fusion protein which comprises a signal sequence located N-terminally with respect to the antibody or the antigen-binding fragment thereof.
10. An expression vector comprising the nucleic acid of aspect 9.
11. A host cell comprising the nucleic acid of aspect 9 or the expression vector of aspect 10.
12. A pharmaceutical composition comprising an antibody or an antigen-binding fragment thereof according to aspects 1 to 7 and a pharmaceutically acceptable excipient, carrier, or diluent.
13. The antibody or an antigen-binding fragment thereof according to any of aspects 1 to 7, the nucleic acid according to aspects 8 or 9, the expression vector according to aspect 10 or the host cell according to aspect 11 for use in medicine.
14. The antibody or an antigen-binding fragment thereof according to any of aspects 1 to 7, the nucleic acid according to aspects 8 or 9, the expression vector according to aspect 10 or the host cell according to aspect 11 for use in the prevention or in the treatment of an infection by an enveloped virus.
15. The antibody, antigen-binding fragment thereof, nucleic acid, expression vector host cell for use according to aspect 14 wherein the enveloped virus is a lentivirus, a virus of the filoviridae family, an Orthopneumovirus, a coronavirus or an arenavirus.
16. The antibody, antigen-binding fragment thereof, nucleic acid, expression vector host cell for use according to aspect 15 wherein the lentivirus is the human immunodeficiency virus (HIV-1), the virus of the filoviridae family is the Ebola virus (EBOV), the orthopneumovirus is the Respiratory syncytial virus (RSV) or the coronavirus is SARS-CoV-2.
17. A conjugate comprising:
   - an antibody or antigen-binding domain as defined in any of aspects 1 to 7 and
   - an agent of interest,
   wherein the agent of interest is covalently coupled to the antibody or to the antigen-binding fragment thereof.
18. The conjugate according to aspect 17 wherein the compound of interest is a detectable moiety or a compound with therapeutic activity.
19. The conjugate according to aspect 18 wherein the agent of interest is a compound with therapeutic activity and wherein the compound with therapeutic activity is a cytotoxic compound, an antigen or a compound which induces maturation of dendritic cells.
20. Use of the conjugate according to aspects 17 to 19 for the delivery of the agent of interest to a target cell that expresses Siglec-1.
21. Use according to aspect 20 wherein the target cell is a dendritic cell.
22. A conjugate according to aspect 21 for use in medicine.
23. A conjugate according to aspect 21 wherein the compound with therapeutic activity is a cytotoxic compound for use in a method of treatment of a disease which is associated with increased dendritic cell activity.
24. A conjugate according to aspect 21 wherein the compound with therapeutic activity is an antigen for use in a method of treatment of a disease which requires an immune response against said antigen.
25. A conjugate according to aspect 21 wherein the compound with therapeutic activity is compound which induces maturation of dendritic cells for use as activator of the immune system or in a method of treatment of a disease which requires an immune response.
26. An in vitro method for the capture of particles containing sialic acid present in a sample which comprises:
   (i) contacting the sample with a Siglec-1 protein variant that comprises the Siglec-1 protein Ig-like V-set domain under conditions adequate for the binding of the particles containing sialic acid to the Siglec-1 protein variant and
   (ii) recovering the Siglec-1 protein variants from the sample.
27. The method according to aspect 25 wherein the Siglec-1 protein variant further comprises at least one Ig-like C2 set domains.
28. The method according to aspect 26 wherein the Siglec-1 protein comprises at least three Ig-like C2 set domains.
29. The method according to aspect 27 wherein the three Ig-like C2 set domains are the three Ig-like C2 set domains 1, 2 and 3.
30. The method according to aspects 25 to 28 wherein the molecule containing sialic acid is a glycoprotein or a ganglioside containing α2,3-linked sialic acid.
31. The method according to aspects 25 to 29 wherein the Siglec-1 protein variant is provided as a fusion protein comprising a second moiety.
32. The method according to aspect 30 wherein the second moiety is an Fc.
33. The method according to any of aspects 25 to 31 wherein the Siglec-1 protein or the fusion protein containing the Siglec-1 protein variant is coupled to a support.
34. The method according to any of aspects 25 to 32 wherein the particles are selected from the group consisting of enveloped virus, an enveloped VLP and an exosome.
35. The method according to aspect 33 wherein the sample is a sample from a SARS-Cov2-infected individual.
36. The method according to aspect 34 wherein the sample is an exosome-enriched sample.

The invention is described below by virtue of the following examples which are to be construed as merely illustrative and not limitative of the scope of the invention.

### EXAMPLES

### Material and methods

2825200

### Cell Cultures

Vero E6 cells (ATCC CRL-1586) were cultured in Dulbecco's modified Eagle medium, (DMEM; Lonza) supplemented with 5% fetal calf serum (FCS; EuroClone), 100 U/mL penicillin, 100 µg/mL streptomycin, and 2 mM glutamine (all ThermoFisher Scientific). HEK-293T (ATCC repository) were maintained in DMEM with 10% fetal bovine serum, 100 IU/mL penicillin and 100 µg/mL streptomycin (all from Invitrogen). HEK-293T overexpressing the human ACE2 were kindly provided by Integral Molecular Company and maintained in DMEM (Invitrogen) with 10% fetal bovine serum, 100 IU/mL penicillin and 100 µg/mL streptomycin, and 1 µg/mL of puromycin (all from Invitrogen). TMPRSS2 human plasmid (Origene) was transfected using X-tremeGENE HP Transfection Reagent (Merck) on HEK-293T overexpressing the human ACE2 and maintained in the previously described media containing 1 mg/mL of geneticin (Invitrogen) to obtain TMPRSS2/ACE2 HEK-293T cells.

### Primary cell cultures

Peripheral blood mononuclear cells were obtained with a Ficoll-Hypaque gradient (Alere Technologies AS) from HIV-1-seronegative donors and monocyte populations (>97% CD14+) were isolated with CD14-positive selection magnetic beads (Miltenyi Biotec). DCs were obtained culturing these cells in the presence of 1,000 IU/mL of granulocyte-macrophage colony-stimulating factor (GM-CSF) and interleukin-4 (IL-4; both from R&D) for seven days and replacing media and cytokines every 2 days. Activated DCs were differentiated by culturing iDCs at day five for two more days in the presence of 100 ng/mL of lipopolysaccharide (LPS; Sigma-Aldrich) or 1,000 IU/mL of interferon-alfa (IFNα; Sigma-Aldrich). DCs were blocked with 1 mg/mL of human IgG (Baxter, Hyland Immuno) and stained with α-Siglec-1-PE 7-239 mAb (AbD Serotec), α-DC-SIGN-PE DCN46 mAb, α-HLA-DR-PerCP L243 mAb, α-CD86-FITC 2331 (FUN-1) mAb, α-CD83-FITC HB15e mAb and α- CD14-PerCP M⊐P9 mAb (all from BD Biosciences) at 4°C for 30 min. Mouse IgG1-PE (AbD Serotec) and IgG2b-PE (BD Biosciences) were included as isotype controls. Samples were analyzed with FACSCalibur (Becton-Dickinson) using CellOuest and FlowJo software to evaluate collected data. Adequate differentiation from monocytes to DCs was monitored by the loss of CD14 and the acquisition of DC-SIGN.

Monocytes were obtained following the same procedure, and cultured with 1,000 IU/mL of IFNα for 24-48 h.

Human tonsils were removed during tonsillectomies of individuals undergoing prescribed surgery at the HUGTiP. After mechanical disruption, mononuclear tonsillar cells were isolated by Ficoll-Hypaque gradient centrifugation. B-lymphocytes were subsequently depleted with magnetic beads against CD19 while blocking Fc receptors (Miltenyi Biotec). Myeloid cells were further enriched by BDCA1 positive selection (Miltenyi Biotec). Cells were stimulated with 1,000 IU/mL IFNα for 24-48 h as described as previously described. See Pino M, et al., Retrovirology 2015; 12:37. A II primary cells were maintained in RPMI with 10% FBS, 100 IU/mL of penicillin, and 100 ug/mL of streptomycin (all from Invitrogen).

### Cell lines, plasmids and viral stocks

HEK-293T (ATCC repository) and TZM-bl (U.S. National Institutes of Health [NIH] AIDS Research and Reference Reagent Program and Tranzyme Inc.) were maintained in DMEM (Invitrogen). Vero E6 cells (ATCC repository) were maintained in EMEM (ATCC). Raji B-lymphocyte and Raji DC-SIGN cell lines were maintained in RPMI (Invitrogen) or RPMI plus 1 mg/ml of geneticin (Invitrogen). All media contained 10% FBS, 100 IU/ml of penicillin, and 100 ug/ml of streptomycin.

Siglec-1 expressing HEK-293T cells were transfected with X-tremeGENE 9 DNA Transfection Reagent (Merck) in T75 flasks using 30 µg of vector backbone pCMV6-Entry (Origene) comprising the coding region of human Siglec-1 or a point mutation generated by changing (Arg→Ala) at 116 (R116A) by Blue Heron Biotech. Raji cells were used to develop a stable cell line expressing Siglec-1 (Raji Siglec-1) or Siglec-5 (Raji Siglec-5). Cells were transfected with pCMV6-Entry comprising the coding region of human Siglec-1 or Siglec-5 (Origene) by using Amaxa nucleofector (Lonza) following manufacturer's instructions. Transfected cells were sorted and cloned in a FACSVantage SE and maintained in RPMI with 1 mg/ml of geneticin for selection of a stable clone with high Siglec expression. Murine cells stably expressing human Siglec-1 were generated by electroporation of Siglec-1 plasmid and selection in blasticidine-containing media (InvivoGen). Cells were stained with mAbs α-Siglec-1-PE 7-239 (BioLegend), α-DC-SIGN-PE DCN46 (BD Biosciences), and α-Siglec-5/14-PE 1A5 (BioLegend) to assess surface expression of all receptors. Of note, Siglec-14 shares 100% of amino acid homology with Siglec-5 in the V-set domain, so mAbs recognizing Siglec-5 cross-react with Siglec-14.

EboVP40-eGFP VLPs were obtained transfecting the molecular clone CAGGS-eGFP-VP40 EboVP40-NanoLuc VLPs32 were obtained transfecting the molecular clone VP40-NanoLuciferase. Ebo-GPVP40-eGFP or Ebo-GPVP40-NanoLuc were obtained transfecting the corresponding plasmid along with pcDNA3.1-Zaire GP (BEI Resources). Ebo-GPVP40-BlaM VLPs were obtained transfecting the molecular clones pcDNA3.1-BlaM-VP40, pcDNA3-Zaire NP and pcDNA3.1-Zaire GP or pCAGGS-Marburg Musoke GP (from BEI Resources). HEK-293T cells were transfected with calcium phosphate (Clontech) or X-tremeGENE 9 DNA Transfection Reagent (Merck) in T75 flasks using a total of 20-30 µg of plasmid DNAs at equimolar ratios. Primary monocytes (2x106) were isolated by CD14-negative selection (Miltenyi Biotec) and labeled with rabbit polyclonal α-GM1 Ab (Abcam), nucleofected with Amaxa and the recommended kit (Lonza) using 2 µg of EboVP40-NanoLuc and 2 µg of pcDNA3.1-Zaire GP to counteract tetherin activity58 to produce monocyte-derived Ebo-GPVP40-NanoLuc. 72h post-transfection, supernatants containing VLPs were cleared of cellular debris by centrifugation and frozen at -80°C until use. The VP40 content of VLP stocks was determined by a homemade sandwich ELISA using IgG1 mouse anti-VP40 mAbs to coat Nunc MaxiSorp plates (Invitrogen) and a mouse anti-VP40 IgG2a mAb to detect bound protein (both from Fitzgerald). Goat anti-mouse IgG2a HRP (Jackson Immuno Research) was used to reveal the assay. Purified VP40 protein (IBT Bioservices) was used as a standard for ELISA quantification. Alternatively, monocyte-derived Ebo-GPVP40-NanoLuc was quantified using Nano-Glo^{®} Luciferase Assay System (Promega) and assessed on an EnSight Multimode Plate Reader (Perkin Elmer).

HIV-1Gag-eGFP VLP, HIV-1mCherry, HIV-1NFN-SX and HIV-1NL4.3 were obtained, quantified and titrated as described in the art. See Izquierdo-Useros N, et al., J Virol 2007; 81:7559-7570 and Pino M, et al., Retrovirology 2015; 12:37.

### Ganglioside detection on Ebola VLP

EboVP40-eGFP VLPs were adhered to poly-L coated coverslips, fixed in 3 % PFA, permeabilized and blocked using 0.1 % saponin and 0.5 % BSA. VLPs were immunostained with rabbit α-GM1 pAb and detected by α-rabbit IgG Fab fragments (Jackson ImmunoResearch) coupled to Star Red dye (KK114 from Abberior) by NHS-chemistry. Samples were acquired in a Zeiss LSM 710 confocal microscope with a 63×/1.4 NA oil objective and processed using Fiji software.

### Siglec-1 mini-protein based ELISA

Siglec-1 mini-protein was constructed by inserting the Xbal restriction fragment from the Siglec-1 Origene plasmid (including the V-set domain and 3 Ig-like domains of the receptor) into the Xbal restriction fragment of the pcDNA3-human IgG Fc plasmid. The plasmid was sequenced and transfected into HEK-293T cells with calcium phosphate or X-tremeGENE 9. Secreted protein was harvested 48-72 h post-transfection and detected by western blot using α-Siglec-1 7D2 mAb.

Protein was incubated with 10 µg/ml of mAbs α-Siglec-1 7-239 or IgG1 isotype control for 30 min at RT. Mixes were then added to Ebo-GPVP40-NanoLuc produced on HEK-293T or primary monocytes for 1 h at RT. Combinations were applied for 1.5 h to a Nunc Maxisorp plate coated with 5 µg/ml of goat α-human-Fc (Jackson Immunoresearch) and blocked with BSA, extensively washed, lysed with Nano-Glo^{®} Luciferase Assay System and assessed on a EnSight luminometer.

### Uptake of Ebola VLP with glycoproteins

Uptake experiments with Ebo-GPVP40-eGFP were performed pulsing 1x105 DCs with 600 ng of VP40 for 3 h at 37°C. Alternatively, 2x105 Raji cell lines were pulsed with 600-900 ng of VP40 for 3 h at 37°C. For blockade, cells were pre-incubated as previously described. Uptake of two different monocyte-derived Ebo-GPVP40-NanoLuc VLP stocks was tested pulsing 5x105 Raji Siglec-1 cells for 12 h at 37°C. Cells were extensively washed, lysed with Nano-Glo^{®} Luciferase Assay System and assessed on an EnSight luminometer.

### Transduction of DCs

Lentiviral particles coding for different shRNAs were co-infected along a vpx-expressing lentivirus to counteract the restriction factor SAMHD1 and facilitate monocyte infection. Briefly, VSV-G-Pseudotyped SIV3 lentivector was produced. Isolated monocytes (5×106) were infected with SIV3 particles and transduced with two different SIGLEC1-specific or one non-target shRNA control MISSION Lentiviral Transduction Particles (Sigma-Aldrich) at a MOI= 50. Transduced monocytes were differentiated into LPS mDCs and labeled with mAbs α-Siglec-1-PE 7-239 and assessed for Ebo-GPVP40-eGFP VLP capture as described above.

### Microscopy analysis

2.5x105 DCs were pulsed with 780 ng of VP40 from Ebo-GPVP40-eGFP for four h at 37°C. After extensive washing, cells were fixed and permeabilized (Fix & Perm; Invitrogen), and stained with α-Siglec-1 7-239 PE mAb. Alternatively, cells were cultured for 12 h with 390 ng of VP40 Ebo-GPVP40-eGFP, washed, exposed to 220 ng of p24 from HIV-1mCherry for 4 additional hours, washed, fixed, permeabilized and stained with α-Siglec-1 7-239 Alexa 647 mAb (BioLegend). Cells were cytospun into coverslips, covered with DAPI-containing Fluoroshield mounting media (Sigma-Aldrich) and analyzed with an Ultraview ERS Spinning Disk System (Perkin-Elmer) mounted on a Zeiss Axiovert 200 M inverted microscope. For deconvolved images we used a confocal LSM Zeiss780 equipped with an apochromatic 63x oil (NA = 1.4) objective. Pinhole aperture was set to 1 and sampling conditions adjusted to Nyquist criterion (voxel size = 54.9x54.9x200 nm). X-y sections were collected throughout the whole cell z-volume every 0.2 µm. Stacks were deconvolved using Huygens Pro and applying a Point Spread Function obtained by recording Tetraspeck Microspheres of 0.1 µm (Molecular Probes) with the same microscopic parameters as our samples. Volocity software (Perkin-Elmer) was used to analyze microscopy images as previously described. For electron microscopy analysis, 5x106 LPS DCs were pulsed for 12 h with 3,700 ng of VP40 from Ebo-GPVP40-eGFP, incubated an additional 4 h with 1,150 ng of p24 from HIV-1NFN-SX, extensively washed in PBS, and fixed in 2.5% glutaraldehyde for 3 h at 4°C. Cells were then processed by the Electron Microscopy Platform of HUGTiP, for analysis of ultra-thin sections with a JEOL JEM 1010 electron microscope.

### Super-resolution microscopy analysis of VCCs

1x106 LPS DCs were pulsed with 400 ng of VP40 from Ebo-GPVP40-NanoLuc and 200 ng of p24 from a 1:1 mixture of HIV-1Gag-eGFP and HIV-1NL4.3 for 12 h at 37°C. This mixture was used to reduce the amount of fluorescent signal in VCCs and aid in distinguishing individual virus particles. Cells were washed, adhered to poly-L coated coverslips, fixed in 3% PFA, and permeabilised and blocked using 0.1 % saponin and 0.5 % BSA plus 100 µg/mL of human IgGs (Privigen, Behring CSL). Cells were stained with mouse IgG2a α-VP40 mAb (Fitzgerald) and α-mouse Fab fragments (Jackson ImmunoResearch) coupled to STAR RED dye (Abberior) followed by staining with α-Siglec-1 6H9 mAb directly coupled to STAR 580 dye (Abberior). Of note, 6H9 mAb was selected throughout our mAb screening for its capacity to recognize Siglec-1 by FACS while not blocking HIV-1Gag-eGFP uptake. Following immunostaining, samples were post-fixed, overlaid with SlowFade Diamond mounting medium (ThermoFisher Scientific) and imaged using confocal or STED microscopy.

Super-resolution analysis was performed using Leica SP8 STED 3X microscope equipped with a 100×/1.4 NA oil STED objective. 3D STED images were acquired sequentially using 637 nm, 587 nm and 498 nm lines from the white light laser. STAR dye signals were depleted with a donut-shaped 775-nm pulsed STED laser, whereas eGFP signal was depleted with a donut-shaped 592-nm pulsed STED laser. Approximately 120 nm lateral and 300 nm axial resolution (full-width-at-half-maximum) was achieved in all three-acquisition channels representing 2-fold resolution improvement with respect to confocal images in all directions (estimated from fluorescent bead and single fluorescent antibody molecule measurements). 3D STED images were acquired with following parameters: pinhole size: 1 Airy; dwell time: 1.2 µs/pixel, XY pixel size: 40 nm, Z-step size: 150 nm. 3D STED images were deconvoluted using Huygens Professional software using theoretical PSF parameters corresponding to the system's effective observation spot or point-spread-function. Image analyses were performed using Fiji and Imaris software.

### Ebola VLP Cytoplasmic Entry Assays

DCs or distinct Raji cells were pre-incubated or not with blocking reagents as previously described. An equivalent fusogenic amount of Ebo-GPVP40-BlaM VLP (ranging from 15-55 ng of VP40) was added to 2.5x105 cells and incubated 12 h at 37°C. The CCF2-AM substrate (Invitrogen) was added to cells following manufacturer's instructions to identify cells in which Ebo-GPVP40-BlaM cytoplasmic entry occurred. Cells were acquired with a LSRII (BD) and the percentage of positive cells was determined with FlowJo software. DCs derived from blood donors were regularly stained with an α-Siglec-1 7-239 PE, allowing us to identify Siglec-1 null individuals by FACS. Direct genotyping of rs150358287G4T by Taqman allelic discrimination from Applied Biosystems (AHKAY5K) confirmed the null status of the indicated donor. To use equivalent numbers of fusogenic viral particles in all the entry assays, Ebo-GPVP40-BlaM stocks were titrated in duplicate by serial ½ dilutions in 3x104 Vero E6 cells/well seeded in 96-well plates, loaded with CCF2-AM substrate and assessed by FACS. Fusogenicity was compared in the highest non-saturated well, where cytoplasmic entry on Vero cells ranged between 26 to 47% of the cells.

### Mice immunization and generation of novel mAbs

Three groups of five female mice BALB/c (Envigo) were immunized 3 times with either: a) 30 µg of 3 distinct Siglec-1 linear peptides; b) 8 ×106 IFN-α treated human DCs or c) 20 ×106 300.19 murine cells stably expressing human Siglec-1. One group of five female mice C57BL/6J (Envigo) were immunized 3 times with 20 x106 L1.2 C57BL/6J murine cells stably expressing human Siglec-1 with or without 10 µg of CpG ODN 1826 adjuvant (InvivoGen). Mice received three dorsal subcutaneous injections in a 28-day interval with immunogens diluted in Freund's adjuvant (300µl). At day 68 -72, blood was extracted to analyze serum responses using HIV-1 VLP uptake assays with Raji Siglec-1 cells as described in the competition section. All sera coming from immunizations with Siglec-1 positive cells had blocking activity and were further tested by ELISA and FACS. ELISA plates covered with 1 µg/ml of hSiglec-1 recombinant protein (R&D Systems) or synthesized Siglec-1 linear peptides (LifeTein, bioNova) were used to detect sera containing specific mAbs employing the α-Siglec-1 commercial mAbs 7D2 and 7-239 as positive controls (1 µg/ml). Sera were also used to label Raji Siglec-1 cells with secondary anti-mouse IgGs labeled in PE (Invitrogen) by FACS. Serum from mice immunized with a non-related peptide was used as a negative control. Positive sera at lower dilutions for ELISA and FACS analysis were used to identify those mice producing the best mAbs. Splenocytes from those mice were employed to generate hybridomas that were tested by FACS using Raji Siglec-1 cells. Best hybridomas were cloned twice and further analyzed in HIV-1 VLP uptake assays.

Five hybridomas generating five novel α-Siglec-1 mAbs (3F1, 5B10, 1F5, 6G5, and 4E8) were selected based on their ability to recognize Siglec-1 and block HIV-1 VLP uptake. mAbs were purified by protein A or protein G affinity chromatography with HiTrap columns (GE Healthcare). DNA from hybridomas was sequenced following the standard operating procedure of GenScript. Siglec-1 mini-protein was bound to Nunc Maxisorp plates coated with 5 µg/ml of goat anti h-Fc and used for detection of novel and commercial mAbs with a HRP labeled goat anti-mouse IgG F(ab')2 (Jackson Immunoresearch). A murine IgG2b (eBioscience) or IgG1 (BD) isotype mAbs were used as negative controls. Specificity of novel α-Siglec-1 mAbs was tested against HEK-293T cells transfected with 30 µg of plasmids coding for Siglec-1, 5 and 7 (all from Origen) and revealed with DyLight 649 secondary IgG mAb (Invitrogen). Transfection efficiency was measured by FACS staining HEK-293T with mAbs α-Siglec-1-PE 7-239, α-Siglec-7-PE 5-386 (AbD Serotec), and α-Siglec-5/14-PE 1A5. Ab isotypes were determined by ELISA using peroxidase-conjugated antibodies specific for the heavy chain of mouse IgG1, IgG2a, IgG2b, IgG3 or IgM (Southern Biotech). Of note, none of the commercial or novel anti-Siglec-1 mAbs displayed unspecific antiviral effects, as none of them were able to reduce Ebo-GPVP40-BlaM VLP entry on Vero cells as compared to an isotype mAb control.

### Surface Plasmon Resonance (SPR) analysis of α-Siglec-1 mAbs

SPR analyses were carried out with a Biacore 3000 (GE Healthcare). Human Siglec-1 recombinant protein was immobilized at 10 µg/ml in sodium acetate 10 mM pH 5.0 onto a CM5 sensor chip using an amine coupling method as indicated by the supplier. HBS-EP (Biacore, GE Healthcare) was used as running buffer. Kinetic assays to estimate KD1 were performed using different concentrations of mAbs (1,23 nM-100 nM) at a flow rate of 30 µl/min, an association time of 2 min and a dissociation time of 5 min. Competition binding assays were performed with α-Siglec-1 specific mAbs (100 nM) injected at a flow rate of 30 µl/min. Two mAbs were sequentially injected in a single cycle (co-inject option) for 2 min. Three control analyses were used: a) injection of first mAb followed by running buffer, b) co-injection of first mAb and c) injection of running buffer followed by second mAb. The chip surface was regenerated with a single pulse of 10 mM Glycine-HCl pH 1.7 for 10 sec at the same flow rate. Sensograms were analyzed using the BIAevaluation software 4.1 and resonance data were overlaid, aligned and fitted to a bivalent model. All data set were processed using a double-referencing method. Percentage of binding inhibition to Siglec-1 of the second mAb by the first mAb was carried out by the following calculation: 100 - [(second mAb binding in the presence of first mAb)/(second mAb binding in the absence of first mAb)] × 100. See Myszka D, et al., Methods Enzymol 2000; 323:325-340.

### Competition between novel α-Siglec-1 mAbs and HIV-1 or Ebola VLPs in Raji Siglec-1 cells

A constant concentration of 150 ng of HIV-1Gag-eGFP Gag was mixed in parallel with increasing concentrations of commercial mAbs α-Siglec-1 (clones 7-239 and 7D2), novel α-Siglec-1 mAbs (clones 3F1, 5B10, 1F5, 6G5 and 4E8) and an IgG1 isotype control. 2x105 Raji-Siglec-1 cells were pulsed with these mixes for 1 h at 37°C and assessed by FACS. Equivalent quantities of two mAbs were mixed to achieve the same concentrations used for single mAb experiments. Of note, concentration of all mAbs used (including commercial ones) was confirmed in a sandwich ELISA using a goat HRP-conjugated polyclonal α-mouse Igs Ab (Dako) and an IgG2b mAb (BD Biosciences) as a standard for quantification. Ebo-GPVP40-eGFP VLP competing assays were performed as described for HIV-1, but pulsing 2x105 Raji Siglec-1 cells for 3 h at 37° C with 200-500 ng of VP40 Ebola VLP mixed with the indicated mAbs.

### HIV-1 uptake and trans-infection assays

DCs were pre-incubated with mAbs as previously described and pulsed with 80 ng of HIV-1NL4.3 p24 per 2.5x105 cells for 3 h at 37° C. After extensive washing, part of the cells was lysed with 0.5% Triton X-100 to measure p24 Gag antigen content by an ELISA (Perkin Elmer). Remaining DCs were co-cultured with the reporter cell line TZM-bl at a ratio 1:1 to measure trans-infection. Co-cultures were assayed for luciferase activity 48 h later (BrightGlo luciferase system; Promega) in a Fluoroskan Ascent FL luminometer (Thermo Labsystems). Background values consisting of non-HIV-1 pulsed co-cultures were subtracted for each experiment.

### Ebola VLP uptake and cytoplasmic entry assays using novel α-Siglec-1 mAbs

Ebo-GPVP40-eGFP VLP uptake assays were performed as previously described using distinct DCs and including the indicated novel α-Siglec-1 mAbs at 10 µg/ml. IFNα-treated BDCA1-positive myeloid cells directly isolated from lymphoid tissues were pulsed with 770 ng of VP40 for 12 h at 37°C per 1.5-3x106 cells and treated as described for DCs.

Ebo-GPVP40-BlaM VLP entry assays were performed as previously described using distinct DCs, IFNα-treated monocytes and IFNα-treated BDCA1-positive myeloid cells directly isolated from lymphoid tissues. Cytoplasmic entry assays with Ebo-Marburg GPVP40-BlaM VLPs containing the Marburg glycoprotein were equally done on activated DCs and IFNα-treated monocytes, but entry was only detected on activated monocytes. Cells were pulsed with 30 ng of VP40 per 1.5-3×105 cells with equivalent fusogenic units for 12 h at 37°C but including novel α-Siglec-1 mAbs at 10 µg/ml and the Cathepsin B CA-074 Me inhibitor at 50 µM (Enzo Life Sciences) when indicated.

### Statistical analysis

Mean changes were analyzed using a paired t-test or a Mann-Whitney test considered significant at P <0.05. Significant mean changes from 100% of the data normalized to percentages were assessed with a one sample t-test, considered significant at P <0.05. Response curves of mAbs were adjusted to a non-linear fit regression model (calculated with a four-parameter logistic curve with variable slope) and the associated extra sum-of-squares F tests were used to compare significant differences between the Log IC50 of mAbs. All analyses and figures were generated with GraphPad Prism v7.0d software.

### Virus Isolation, Titration and Sequencing

SARS-CoV-2 was isolated from a nasopharyngeal swab collected from an 89-year-old male patient giving informed consent and treated with Betaferon and hydroxychloroquine for 2 days before sample collection. The swab was collected in 3 mL medium (Deltaswab VICUM) to reduce viscosity and stored at -80°C until use. Vero E6 cells were cultured on a cell culture flask (25 cm2) at 1.5 × 106 cells overnight prior to inoculation with 1 mL of the processed sample, for 1 h at 37°C and 5% CO2. Afterwards, 4 mL of 2% FCS-supplemented DMEM were supplied and cells were incubated for 48 h. Supernatant was harvested, centrifuged at 200 x g for 10 min to remove cell debris and stored at -80°C. Cells were assessed daily for cytopathic effect and the supernatant was subjected to viral RNA extraction and specific RT-qPCR using the SARS-CoV-2 UpE, RdRp and N assays (Corman V, et al., Eurosurveillance 2020; 25). The virus was propagated for two passages and a virus stock was prepared collecting the

supernatant from Vero E6. Viral RNA was extracted directly from the virus stock using the Indimag Pathogen kit (Indical Biosciences) and transcribed to cDNA using the PrimeScript^{™} RT reagent Kit (Takara) using oligo-dT and random hexamers, according to the manufacturer's protocol. DNA library preparation was performed using SWIFT amplicon SARS-CoV-2 panel (Swift Biosciences). Sequencing ready libraries where then loaded onto Illumina MiSeq platform and a 300bp paired-end sequencing kit. Sequence reads were quality filtered and adapter primer sequences were trimmed using trimmomatic. Amplification primer sequences were removed using cutadapt (Martin M, et al., EMBnet.Journal 2011; 17:310). Sequencing reads were then mapped against coronavirus reference (NC_045512.2) using bowtie2 tool (Langmead B, et al., Nat Methods 2012; 9:357-359). Consensus genomic sequence was called from the resulting alignment at a 18x1800x879 average coverage using samtools (Li H, et al., Bioinformatics 2009; 25:2078-2079. Genomic sequence was deposited 5 at GISAID repository (https://www.gisaid.org/) with accession ID EPI_ISL_510689.

### SARS-CoV-2 Uptake Assays

Uptake experiments with SARS-CoV-2 were performed pulsing 0.5x106 Raji or myeloid cells at a constant rate of X ng of Nucleocapsid at 37°C. For blockade, cells were preincubated for 15 min at RT with 10 µg/ml of mAbs α-Siglec-1 7-239, or IgG1 isotype control (BD Biosciences), or left untreated before viral exposure. After extensive washing, cells were lysed at a constant concentration of 1x106 cells/mL, centrifuged to remove cellular debris and assayed with a SARS-CoV-2 Nucleocapsid protein (NP) High-sensitivity Quantitative ELISA (bioVendor).

### Pseudovirus Production

HIV-1 reporter pseudoviruses expressing SARS-CoV-2 Spike protein and luciferase were generated using two plasmids. pNL4-3.Luc.R-.E- was obtained from the NIH AIDS repository. SARS-CoV-2.SctΔ19 was generated (Geneart) from the full protein sequence of SARS-CoV-2 spike with a deletion of the last 19 amino acids in C-terminal, human-codon optimized and inserted into pcDNA3.4-TOPO (Ou X, et al., Nat. Commun. 2020; 11:1620). Spike plasmid was transfected with X-tremeGENE HP Transfection Reagent (Merck) into HEK-293T cells, and 24 hours post-transfection, cells were transfected with pNL4-3.Luc.R-.E-. Supernatants were harvested 48 hours later, filtered with 0.45 µM (Millex Millipore) and stored at -80°C until use. The p24gag content of all viruses was quantified using an ELISA (Perkin Elmer) and viruses were titrated in HEK-293T overexpressing the human ACE2.

### Trans-infection Assay

HEK-293T overexpressing the human ACE2, or both ACE2 and TMPRSS2 were used to test if SARS-CoV-2 pesudoviruses can fuse with these receptors via trans-infection. A constant pseudoviral titer was used to pulse cells in the presence of the indicated mAbs for 2h at 37°C. Cells were extensively washed and co-cultured with or without target cells at a ratio 1:1. 48h post-infection, cells were lysed with the Glo Luciferase system (Promega). Luminescence was measured with an EnSight Multimode Plate Reader (Perkin Elmer).

### Antibody cloning

Total RNA isolated from hybridomes was reverse transcribed into cDNA using isotype-specific anti-sense primers or universal primers following the technical manual of PrimeScriptTM 1st Strand cDNA Synthesis Kit. The antibody fragments of VH, VL, CH and CL were amplified according to the standard operating procedure of rapid amplification of cDNA ends (RACE) of GenScript and followed by sequencing analysis.

Mouse VH and VL of murine antibodies were aligned by Ig Blast-NCBI (https://www.ncbi.nlm.nih.gov/igblast) to get the closest human related V gene sequences and to identify the framework residues (FRs) and complementarity-determining regions (CDRs). For the humanization process, mouse residues forming the CDRs were retained while the FRs residues not matching between mouse and human germline were changed to the residue present in the human V gene.

Constructs with the murine VH and VL sequences of #1F5, #3F1 and #5B10 antibodies were ordered at Invitrogen to produce each antibody as both mouse/human chimeric hIgG1 and humanized CDR-engrafted hIgG1. For the light chain, constructs encoding for the whole light chain (VL + CL) were obtained directly from Invitrogen cloned into the pcDNA3.1 mammalian expression vector. For the heavy chain, constructs encoding for the VH flanked by the HindIII and Nhel restriction sites were ordered at Invitrogen cloned into the pMA-RQ vector. Each VH was excised from the pMA-RQ vector by Hindlll and Nhel digestion and cloned into a pcDNA3.1 vector that already contained the human IgG1 constant domains (ref Dekkers et al 2017 Front immunol). DNA encoding for anti-CD169 variants to remove potential sequence liabilities was ordered at Invitrogen or generated by site directed mutagenesis (Missing: method description). All constructs were amplified by maxiprep and sequenced for validation.

### Antibody production

Suspension growing HEK FreeStyle^{™}2 93-F Cells (Ref. R790-07, ThermoFisher Scientific) were cultured in FreeStyle^{™}2 93 Expression Medium (Ref. 12338026, ThermoFisher Scientific) at 37°C with 8% CO2. The day of the transfection cells were centrifuged and seeded at density of 1E6cell/mL in fresh Freestyle medium. Plasmids encoding for the heavy and kappa chain of each antibody were transiently PEI mediated transfected (Polyethylenimine; Ref. 24765-1, Polysciences Inc.) together with 3 helper plasmids (pORF21, pORF27 and p33-SV40LT). Transfection mixtures per 100 mL of cell culture contained: 31.35 µg of heavy chain construct, 37.65 µg of light chain construct, 31 µg of helper vectors mix, 300 µg PEI and 6 mL of optiMEM (Ref. 51985026, ThermoFisher Scientific). Transiently transfected HEK293FS cells were cultured at 100 mL scale for 6 days at 37°C, 8% CO2. The culture medium was harvested by centrifugation and the antibodies were purified from the culture medium using Protein A HiTrap columns (ThermoFisher Scientific) and eluted using citrate buffer (20 mM citrate, 150 mM NaCl, pH=3.5) followed by neutralization with phosphate buffer (KH2PO4/K2HPO4 pH=8). Buffer was changed into 5% Glucose, 5 mM Sodium Acetate, pH4.5 by Pierce Protein Concentrator PES, 10 kDa (Ref. 88517, Pierce). Antibody concentration was determined by measuring absorbance at 280 nm. Volume was adjusted to have each antibody at 1 mg/ml.

### Functional competition assay with Raji-Siglec-1 cells

Raji-Siglec-1 cell line was generated in the inventors lab to express constitutively CD169 receptor as described previously (Perez-Zsolt et al., 2019b). This cell line was maintained in RPMI media (Ref. 21875091, Gibco, Thermo Invitrogen) supplemented with 1 mg/ml of geneticin (Ref. 10131-027, Gibco, Thermo Invitrogen), 10% FBS (Ref. 10270-106, Gibco, Thermo), 100 IU/ml of penicillin and 100 ug/ml of streptomycin (Ref. 15070063, Gibco, Thermo).

HEK-293T (ATCC repository) were cultured in DMEM (Ref. 41966-029, Gibco, Thermo Invitrogen) supplemented with 10% FBS, 100 U/ml of penicillin and 100µg/ml of streptomycin at 37°C with 8% CO2. HIVGag-eGFP-VLPs stocks were generated by transfecting 1E7 HEK-293T cells with 15 µg of pHIV-Gag eGFP plasmid using 15 µl of LipoD293 (Ver. II) reagent (Ref. SL100668, SignaGen). Supernatants containing VLPs were filtered (Millex HV, 0.45µm; Millipore) and frozen at -80°C until use. Similarly, to generate HIV-1NL4.3 Gag-iGFP VLPs stocks, 1E7 HEK-293T cells (cultured as previously described) were transfected with 20 µg of pHIV-Gag iGFP plasmid using XtremeGene9 reagent (Ref. 6365809001, MERCK) and following manufacturer's instructions. Supernatants containing VLPs were collected 48 hours post-transfection, filtered (Millex HV, 0.45µm; Millipore) and frozen at -80°C until use. By using HIVGag-eGFP-VLPs and HIVGag-iGFP-VLPs, the inventors can mimic the native virion structure in the absence of genetic material or the native replicating virus only during the first infection cycle respectively, making them non-infectious and safe to manipulate.

Serial dilutions from 20µg/ml to 0.2 µg/ml of anti-CD169 antibodies or IgG1 isotype control (Ref. 554721, BD Bioscience) were prepared in supplemented RPMI media. Then, HIVGag-eGFP-VLPs at non-saturating concentration were added, followed by Raji-Siglec-1 cells. Assay was performed for 60 min at 37°C. After washing with PBS, cells were suspended in PBS supplemented with 0.5% FBS and analyzed by FACS (FACSCantoll, BD) to determine VLP uptake (analyzed with FlowJo software). As non-inhibiting controls the inventors used the corresponding mAb isotype controls, while as negative control, the inventors used cells not exposed to the virus particles. All experiments have been performed in duplicates.

### Viral entry into primary myeloid cells

Peripheral blood mononuclear cells were obtained with a Ficoll-Hypaque gradient (Missing REF Alere Technologies AS / ATOM, Cat No. 1114547) from blood donors. Then, monocyte populations (>97% CD14+) were isolated with CD14 positive selection magnetic beads (130-050-201 Miltenyi Biotec). Dendritic cells (DCs) were obtained by culturing these cells in the presence of 1,000 IU/ml granulocyte-macrophage colony-stimulating factor and interleukin-4 (Missing REF and Missing REF, R&D) for seven days and replacing media and cytokines every two days. Activated DCs were differentiated by culturing immature DCs at day five for two more days in the presence of 100 ng/ml lipopolysaccharide (LPS, Missing REF Sigma-Aldrich). CD169 expression in mature DCs was validated by flow cytometry upon blocking with 1 mg/ml human IgG (Missing REF Privigen, Behring CSL) for 30 minutes at room temperature, followed by staining with anti-Siglec-1-PE 7-239 mAb (Missing REF AbD Serotec) at 4 °C for 30 min. Mouse IgG1-PE (Missing REF AbD Serotec) was included as isotype control for this staining. Samples were analyzed with FACSCalibur (BD Biosciences) using CellQuest and FlowJo (v10.8.1) software to evaluate collected data.

To generate HIV-1NL4.3, 1E7 HEK-293T cells (cultured as previously described) were transfected with 30 µg of pNL4.3 plasmid, obtained from the US National Institute of Health (NIH) AIDS Research and Reference Reagent Program (Missing REF), using calcium phosphate kit (Missing REF Calphos; Clontech) and following manufacturer's instructions. Supernatants containing VLPs were collected 48 hours post-transfection, filtered (Millex HV, 0.45µm; Millipore) and frozen at -80°C until use. The p24Gag content of HIVGag-iGFP-VLPs and viral stocks was determined by enzyme-linked immunosorbent assay (ELISA) (Missing REF Perkin-Elmer) for titration of viral preparations.

EboVP40-eGFP and Ebo-GPVP40-BlaM VLPs were generated as described (Perez-Zsolt et al., 2019a). Missing transfection method. EboVP40-eGFP VLPs were generated transfecting HEK-293T cells with the molecular clone CAGGS-eGFP-VP40 (kindly provided by Dr. Bieniasz). For Ebo-GPVP40-BlaM VLPs, cells were transfected with molecular clones pcDNA3.1-BlaM-VP40, pcDNA3-Zaire NP and pcDNA3.1-Zaire GP (all from BEI Resources Missing REF). Supernatants containing VLPs were collected 72 hours post-transfection, filtered (Millex HV, 0.45µm; Millipore) and frozen at -80°C until use.

SARS-CoV-2 used was isolated in March 2020 from a nasopharyngeal swab as described (Rodon, 2021). The virus was propagated upon two passages in Vero E6 cells. Genomic sequence was deposited at GISAID repository with accession ID EPI_ISL_510689 (http://gisaid.org). Vero E6 cells (ATCC CRL-1586) were cultured in DMEM medium supplemented with 5% FBS, 100 U/mL penicillin, 100 µg/mL streptomycin, and 2 mM glutamine (Missing REF ThermoFisher Scientific). TMPRSS2/ACE2 HEK-293T cells used were generated by transiently transfecting TMPRSS2 human plasmid (Missing REF Origene) with X tremeGENE HP Transfection Reagent (Ref 6365809001 Merck) into ACE2 HEK-293T cell line. This cell line that overexpresses human ACE2 protein constitutively was kindly provided by Integral Molecular Company (Missing REF). TMPRSS2/ACE2 HEK-293T cells were maintained in DMEM supplemented with 10% FBS, 100 IU/mL penicillin, 100 µg/mL streptomycin, 1 µg/mL of puromycin (Missing REF Invitrogen) and 1 mg/mL of geneticin (Missing REF Invitrogen).

HIV-1 and EboVP40-eGFP uptake experiments were performed pulsing 0.25E6 DCs with a constant number of viral particles per condition at 37°C for 4 hours. Cells were pre-incubated for 15 min at RT with 10ug/ml of anti-CD169 mAbs, an IgG1 isotype control (Missing REF BD Biosciences) or left untreated before viral exposure. After extensive washing with PBS buffer, cells were acquired by flow cytometry (FACSCantoll, BD) and the frequency of positive cells was determined using FlowJo software (v10.8.1 TreeStar). Forward-angle and side-scatter light gating were employed to exclude dead cells and debris from all analysis.

For HIV-1 trans-infection assays 0.25E6 LPS-matured DCs were incubated with a constant amount of HIV-1NL4-3 for 4 h at 37°C. Cells were pre incubated for 15 min at RT with 10 µg/ml of anti-CD169 mAbs, an IgG1 isotype control (BD Biosciences) or left untreated before viral exposure. After extensive washing, cells were co-cultured with the reporter cell line TZM-bl (Missing REF) at a 1:1 ratio to measure trans-infection. Co cultures were assayed for luciferase activity 48 h later (Missing REF BrightGlo luciferase system; Promega) using an EnSight Multimode Plate Reader (Perkin Elmer). Background values from non-HIV-1 pulsed co-cultures were subtracted for each experiment.

Fusion assays were performed as described previously (Perez-Zsolt et al., 2019). When non-infectious Ebo-GPVP40-BlaM VLPs fuse with cellular membranes, they release β-lactamase that can then cleave a CCF2-AM dye loaded into the DC cytoplasm and change its fluorescence emission from fluorescein to coumarin. As opposed to the system previously used to detect viral uptake, this assay selectively detects Ebola virions entering the cytoplasm of the cell by fusion. DCs were preincubated or not with anti-CD169 mAbs as before. A constant fusogenic amount of Ebo-GPVP40 BlaM VLPs was added to 0.25E6 cells and incubated overnight at 37°C. The CCF2-AM substrate (Ref K1032 Invitrogen) was added to cells following the manufacturer's instructions, to identify cells in which Ebo-GPVP40-BlaM cytoplasmic entry had occurred. Cells were assessed by flow cytometry (FACSCantoll, BD), and the percentage of positive cells was determined with FlowJo software. To use equivalent numbers of fusogenic viral particles in all entry assays, Ebo-GPVP40-BlaM VLP stocks were titrated in duplicate by serial 50% dilutions in Vero E6 cells (3E4per well) seeded in 96-well plates, loaded with CCF2-AM substrate, and assessed by flow cytometry (FACSCantoll, BD).

For SARS CoV-2 uptake experiments, the followed procedure was as previously described (Perez-Zsolt et al., 2021). 0.25E6 DCs were pulsed with a constant number of viral particles per condition at 37oC for 3 hours. Cells were pre-incubated for 15 min at RT with 10ug/ml of anti-CD169 mAbs, an IgG1 isotype control (Missing REF BD Biosciences) or left untreated before viral exposure. After extensive washing with PBS buffer, SARS-CoV-2 Nucleocapsid protein (NP) in the cells was quantified by High-sensitivity Quantitative ELISA (Ref 41A228R BioVendor) to determine virus uptake.

According to previously described method SARS-CoV-2 for trans-infection experiments (Perez-Zsolt et al., 2021), HEK-293T over-expressing the human ACE2 or lacking this molecule were used to test if SARS-CoV-2 replication competent virus was trans-infected. Uptake experiments with SARS-CoV-2 were performed pulsing 0.25E6 LPS activated DCs with a MOI of 0.75 for 3 h at 37°C. After extensive washing, cells were co cultured at a ratio 3:1 with HEK-293T cells expressing or not ACE2. Six days later, supernatant was assayed with a SARS-CoV-2 nucleocapsid protein (NP) High-sensitivity Quantitative ELISA (Missing REF ImmunoDiagnostics).

### Oxidative stress induction and SEC-MALS analysis

Oxidative stress to investigated antibodies was induced by incubating them with 0.1% hydrogen peroxide solution for 20 min at 37°C and quenched with 80 mm Met (Met? Missing REF) aqueous solution as described (Luo et al., 2011). Resulting protein solution was analysed by Size-Exclusion-Chromatography and Multi-Angle-Light-Scattering (SEC-MALS) for estimation of size distribution and relative aggregate formation. Thus, 0.050 mg of each antibody at 1mg/ml was injected into a Superdex200 10/300 GL, id0026 column (Missing REF) attached to Agilent 1260 infinity II HPLC system (Missing REF), equipped with UV7VIS detector SPD-20A S(Shimadzu), miniDAWN (Wyatt), Optilab (Wyatt) using PBS as running buffer with flow at 0,75ml/min. Graphs made were made using the UV signal. Further analysis and estimations of the molecular weight were calculated via multi-angle and dynamic light scattering using the Astra software (version 7) of Wyatt Technologies.

### Surface Plasmon Resonance analysis

Analysis of the binding curves and calculation of the affinity of the humanized mAb variants for CD169 was performed by Surface Plasmon Resonance in the IBIS MX96 (IBIS Technologies) platform by using equilibrium analysis as described before (Dekkers et al., 2017). In brief, anti-CD169 antibodies, and controls, were spotted on a single Easy2Spot G-type sensor (Ssens) at 60nM, 20nM and 6nM concentrations in 10mM sodium acetate buffer, pH4.5 using a continuous flow microspotter (Wasatch). The CD169 protein (Missing REF R&D Systems) was injected over the sensor in increasing 12 concentrations, ranging from 0.049 to 100nM, allowing to measure binding by Surface Plasmon Resonance (SPR). Regeneration after every sample was carried out with 10mM Glycine-HCl, pH2.0. Association and dissociation were monitored to calculate dissociation constants (KD) in Scrubber version 2 software Scrubber version 2 software (Biologic Software), and then interpolated to one specific Rmax = 1000 RU using Excel.

### Mini-Siqlec-1 protein (mS1) capture of EVs from plasma

The plasmid pC-mS1-Fc encodes for a truncated form of Siglec-1 containing the sialylated ligand-binding domain, the mS1 recombinant protein consists of the V-set domain and three Ig-like domains of Siglec-1/CD169 cell receptor fused to the human IgG Fc domain. HEK293T cells were transfected using LipoD293 (Ver. II) Transfection Reagent (SignaGen Ref: SL100668) with 15 µg of that plasmid for 107 cells in T75 flasks. Secreted mS1 protein was harvested 72 hours post-transfection, filtered at 0.22 µm and immediately frozen in 20% glycerol.

mS1-beads were prepared in a two-step procedure. First, 2.4-108 of Aldehyde/Sulfate Latex beads (Thermo #A37304) were incubated with 0.32mg of recombinant protein G (Sigma-Aldrich # P4689) for 15 min in agitation at 20°C. Then, beads were saturated in 1% BSA. After two washing steps with cold PBS, beads were incubated with mS1 protein in saturating conditions for 30 min at room temperature in agitation. Then, beads were centrifuged for 3 min at 15000 xg and washed three times with cold PBS.

For capturing, one ml aliquots of human plasma samples were thawed on ice, immediately diluted 1:10 in cold PBS and ultracentrifuged at 120,000 xg for 4 hours in a TH-641 Swinging Bucket Rotor. Resulting pellets (P120 fraction) were resuspended in cold PBS and protein concentration determined by BCA (Biorad). From P120 fractions described above, 1mg of total protein was incubated with 1.6·107 mS1-B (total volume of 300 µl) at 20°C with agitation for 1 hour. Then, beads were centrifuged for 3 min at 15000 xg. Flow-through was collected and stored. EVs-mS1-B complexes were then washed with 0.5 ml and 0.3 ml of ice-cold PBS. Immediately after the last wash, half of the sample (150 µl) was processed for proteomic analysis as described below.

### Example 1: Activation of DCs enhances Ebola viral binding and uptake via Siglec-1 recognition of sialylated gangliosides

Upon Ebola virus invasion, therapies targeting host factors that modulate viral entry should work on primary target cells like DCs. These potent antigen-presenting cells respond to immune activating signals triggered by infection to initiate antiviral responses but are also preferential targets of filovirus infection. Activating immune signals such as the bacterial cell wall component lipopolysaccharide (LPS) or the type I interferon alpha (IFNα) are augmented throughout the course of filovirus infection. LPS was found increased due to bacterial translocation, which could account for reported septicemia associated to Ebola virus disease, while IFNα was detected in blood samples from infected patients in particular Ebola outbreaks. Thus, the effect of these activating factors present during the course of Ebola virus infection on DCs was assessed. LPS triggered up-regulation of HLA-DR and co-stimulatory molecules such as CD86 and CD83, while IFNα-treated DCs or non-activated immature DCs (iDCs) had higher levels of the C-type lectin DC-SIGN. See Fig. 1. Noteworthy, LPS or IFNα-treated DCs up-regulated Siglec-1 expression compared to iDCs. See Fig. 2A.

Distinctly activated DCs were next pulsed with equal amounts of fluorescent Ebola viral-like particles lacking the envelope glycoprotein, which were produced by transfection of a plasmid coding for the Ebola matrix VP40 protein fused to eGFP (EboVP40-eGFP VLP). After extensive washing, the fold change in geometric mean fluorescence intensity was measured over time by flow cytometry, comparing cells exposed to the virus versus non-pulsed DCs. Active viral uptake at physiological temperature was higher than when temperature was arrested for endocytosis to only allow viral binding, and these differences increased over time on activated DCs. See Fig. 2B. Moreover, when the percentage of eGFP-positive cells was analyzed at 6 hours, activated DCs showed a higher EboVP40-eGFP VLP binding capacity at 4°C (P≤0.0040; Fig. 2C) and enhanced uptake ability at 37°C than iDCs (P≤0.0009; Fig. 2D). Hence, although inflammatory stimuli trigger an activation and maturation program assumed to restrict DC ability for antigen uptake, Ebola viruses were preferentially bound and up taken by activated DCs.

These results suggested the activity of a viral endocytic receptor elicited upon DC activation. EboVP40-eGFP VLP employed lacked the viral glycoprotein required for C-type lectin recognition but carried sialyllactose-containing gangliosides such as GM1 that were detected with specific antibodies on the viral membrane of Ebo VLPs by confocal microscopy. See Fig. 2E. As these gangliosides are recognized by Siglec-1 receptor the specificity of this interaction with Ebo VLPs was confirmed.

A recombinant Siglec-1 mini-protein expressing the V-set domain plus three Ig-like domains fused to a human IgG Fc fragment was produced. This mini-protein was pre-incubated with or without an isotype control or a murine commercial α-Siglec-1 mAb (clone 7-239) that prevents HIV-1 binding. These mixes were further incubated with an Ebo VLP stock produced by transfection of a plasmid coding for the VP40 protein fused to NanoLuciferase (EboVP40-NanoLuc VLP). When these mixtures were applied to an α-human Fc fragment-coated ELISA plate, only the untreated mini-protein or that treated with an isotype mAb effectively bound EboVP40-NanoLuc VLP as detected by luminescence. See Fig. 2F. Next, two Siglec-1 plasmids with or without a mutation on a key residue (critical for sialic acid recognition and for HIV-1 binding to Siglec-1) were transfected into a cell line devoid of this receptor and achieved similar transfection efficiency. See Fig. 2G, left graph). Compared to the wild type receptor, expression of the mutated protein led to the loss of EboVP40-eGFP VLP recognition via Siglec-1 on transfected cells. See Fig. 2G, right graph). These data demonstrate that Siglec-1 directly interacts with Ebo VLPs bearing gangliosides but lacking the viral glycoprotein, and that recognition of sialylated viral residues is critical for this interaction.

Then, whether the observed Ebola viral binding and uptake on activated DCs was mediated by Siglec-1 was investigated. DCs were pre-treated with two commercial α-Siglec-1 mAbs (clones 7-239 and 7D2). While isotype control mAb had no inhibitory effect, pre-treatment with α-Siglec-1 mAbs led to a reduction of EboVP40-eGFP VLP binding (Fig. 2H) or uptake on DCs. See Fig. 2I and Fig. 3. As Ebola VLP had no viral glycoprotein, pre-treatment with the α-DC-SIGN MR1 mAb or with the broader competitive C-type lectin inhibitor mannan had no effect on viral binding or uptake. See Fig. 2H-I. Thus, Siglec-1 binds Ebola VLP through the recognition of sialylated gangliosides anchored on viral membranes.

### Example 2: Siglec-1 facilitates the uptake of Ebola viruses displaying envelope glycoproteins and stores these particles in the same virus-containing compartment as HIV-1

Next, the Siglec-1 capacity for ganglioside binding in the presence of the native glycoproteins exposed by infectious Ebola viruses, which are recognized by C-type lectins, was tested. Ebola VLPs were pseudotyped with the native Zaire envelope glycoprotein (GP), a method that recapitulates wild-type Ebola virus results without the need for high-level biosafety procedures. DCs were pulsed with equal amounts of these Ebo-GPVP40-eGFP VLPs, washed and assessed by FACS. Again, activated DCs showed a higher Ebo-GPVP40-eGFP VLP uptake capacity than iDCs (P≤0.0026; Fig. 4A). Moreover, pre-treatment with α-DC-SIGN MR1 mAb or mannan had no significant effect on viral uptake in activated DCs. See Fig. 4B. In sharp contrast, treatment with α-Siglec-1 mAbs inhibited retention of viruses containing the Zaire envelope glycoprotein. See Fig. 4B. On iDCs, however, where DC-SIGN is highly expressed (Fig. 1), pre-treatment with MR1 or mannan had a similar inhibitory effect as α-Siglec-1 mAbs. See Fig. 4B. These results strongly suggest that on activated DCs, alternative receptors beyond DC-SIGN operate in Ebola viral uptake, as previously reported for HIV-1.

To verify the essential role of Siglec-1 during Ebola viral uptake, two complementary strategies were applied. First, Siglec-1 and other lectins into a cellular model devoid of these receptors were transfected. A Raji B cell line was selected because it lacks endogenous expression of these lectins and can be transfected without unspecific up-regulation of Siglec-1, as opposed to what happens when monocytic cell lines are used. Raji cells assessed by FACS expressed Siglec-1, DC-SIGN, Siglec-5, or none of these receptors (Fig 5) and were compared on their ability for Ebo-GPVP40-eGFP VLP uptake. See Fig. 4C. Raji Siglec-1 efficiently captured Ebo-GPVP40-eGFP VLP, and this effect was abolished by pretreatment with α-Siglec-1 7-239 mAb (P<0.0001; Fig. 2C). Moreover, Ebo-GPVP40-NanoLuc VLP produced on primary target cells, such as monocytes, were also efficiently uptaken via Siglec-1. See Fig. 6. Raji DC-SIGN also bound Ebo-GPVP40-eGFP VLP and this was blocked by α-DC-SIGN MR1 mAb (P=0.0023; Fig. 4C). No increased uptake was seen in Raji Siglec-5 or the parental Raji cells (Fig. 4C). In contrast to Siglec-1 receptor, Siglec-5 is a shorter molecule that binds to cell-surface sialylated molecules in cis. This masks Siglec-5 potential for viral binding despite displaying a high homology with the V-set domain of Siglec-1. Next, LPS-activated DCs were also transduced with two lentiviruses coding for different SIGLEC1-specific shRNAs20, which decreased Siglec-1 surface expression and led to a concurrent loss of Ebo-GPVP40-eGFP VLP uptake capacity (orange lines, Fig. 4D), while no effect was seen using a non-targeted shRNA control lentivirus (blue line, Fig. 4D). The complementary approaches of Siglec-1 de novo expression on heterologous cells and silencing on DCs, combined with the blocking effect of specific mAbs, strongly supports our conclusion that Siglec-1 is a central molecule mediating Ebola virus uptake.

Siglec-1 interacts with Ebola viral-like particles but also binds to other sialyllactose-containing viruses such as HIV-1. Once HIV-1 is bound to the DC surface via Siglec-1, the receptor polarizes bound viruses towards one cellular pole and traps them within a sac-like virus-containing compartment (VCC) that is continuous with the plasma membrane and remains connected to the extracellular space. To elucidate whether Siglec-1 receptor also recruits Ebo-GPVP40-eGFP VLP to these compartments, viral uptake by confocal microscopy was assayed. DCs were pulsed with fluorescent Ebo-GPVP40-eGFP VLP and subsequently stained with an α-Siglec-1 mAb labeled in PE and DAPI to detect the cellular nucleus. See Fig. 4E. In agreement to prior HIV-1 observations, LPS DCs mainly accumulated Ebo-GPVP40-eGFP within a sac-like VCC enriched in Siglec-1; IFNα-treated DCs mostly displayed viruses polarized towards one cellular pole, and the majority of iDCs had Ebo-GPVP40-eGFP randomly attached throughout the cell surface. See Fig. 4E. When red fluorescent HIV-1 was added to LPS-activated DCs that had previously accumulated Ebo-GPVP40-eGFP in sac-like VCC, most of these virus-containing compartments enriched in Siglec-1 also trapped HIV-1. See Fig. 4F. This indicates that Siglec-1 uptakes Ebola viruses in the same VCC as HIV-1. Ultrastructure analysis by transmission electron microscopy (Fig. 4G) and super-resolution microscopy (Fig. 4H) also corroborated the presence of filoviral particles in sac-like compartments containing HIV-1. Moreover, super-resolution microscopy verified the proximity between Siglec-1 and Ebo-GPVP40-eGFP. See Fig. 4H and Fig. 7). Thus, experiments of confocal, electron and super-resolution microscopy on activated DCs further confirmed that Siglec-1 is critical for Ebola and HIV-1 uptake in virus-containing compartments.

### Example 3: Ebola virus uptake by Siglec-1 facilitates viral cytoplasmic entry into activated DCs

As DCs are primary targets of Ebola virus infection, it was also assessed if Ebola uptake via Siglec-1 enhanced the capacity of Ebola virus to fuse and enter the cytoplasm of activated DCs. This mechanism may resemble the one triggered by phosphatidylserine-binding receptors, which also display a V-set domain as Siglec-1. A viral cytoplasmic entry assay that relies on the incorporation of a β-lactamase-VP40 chimera into Ebola VLPs bearing the Zaire envelope glycoprotein (Ebo-GPVP40-BlaM VLP) to recapitulate Ebola virus entry was employed. When non-infectious Ebo-GPVP40-BlaM VLP fuse with cellular membranes, they release β-lactamase that can then cleave a CCF2-AM dye loaded into the cytoplasm of DCs and change its fluorescence emission. As opposed to the system previously used to detect viral uptake, this assay selectively detects Ebola virions entering the cytoplasm of the cells by fusion. Distinctly treated DCs were pulsed with equal amounts of Ebo-GPVP40-BlaM VLP. After extensive washing, the percentage of cells cleaving CCF2-AM dye as a result of Ebola VLP cytoplasmic entry was analyzed by flow cytometry. Entry of Ebo-GPVP40-BlaM VLPs was lower on LPS-treated DCs than in iDCs, while stimulation with IFNα did not reduce fusion (P≤0.0005; Fig. 8A). Thus, as opposed to that observed for viral binding and uptake (Fig. 2C-D), cytoplasmic viral entry did not correlate with the levels of Siglec-1 expression on DCs. See Fig. 2A.

To investigate the relative contribution of Siglec-1 and C-type lectin receptors to Ebola VLP cytoplasmic entry, cells were pre-treated with specific inhibitors. See Fig. 8B and Fig. 9. Isotype control mAb had no inhibitory effect, while addition of 7D2 or 7-239 α-Siglec-1 mAbs led to a reduction of Ebo-GPVP40-BlaM VLP cytoplasmic entry on activated DCs. See Fig. 8B. Pre-treatment with α-DC-SIGN MR1 mAb had no significant effect on cytoplasmic entry in any of the DCs tested, even though these Ebola VLP displayed the envelope glycoprotein required for C-type lectin receptor recognition. Combination of α-DC-SIGN and α-Siglec-1 mAbs had no further inhibitory capacity than the one displayed by the α-Siglec-1 mAb. See Fig. 8B. Mannan, an inhibitor blocking DC-SIGN and other C-type lectin receptors, had no effect on cytoplasmic entry in LPS DCs, but significantly reduced fusion on IFNα-treated DCs and most prominently on iDCs. See Fig. 8B. Combination of mannan and α-Siglec-1 mAbs had no further inhibitory capacity than mannan on iDCs but reached a higher inhibitory capacity on IFNα-treated DCs. See Fig. 8B. Thus, while C-type lectins partially mediate Ebola VLP cytoplasmic entry on iDCs and IFNα-treated DCs, blockade of Siglec-1 is able to significantly reduce viral entry on LPS DCs (62.6 % ±17.5) and on IFNα-treated DCs (42% ±18.4). Yet, this reduction was lower to that observed on viral uptake. See Fig. 4B. Therefore, Siglec-1 independent viral binding still allows for cytoplasmic entry, highlighting the need to combine distinct inhibitors to fully achieve blockade.

When cytoplasmic viral entry was assessed on Raji cells, no fusion was detected (Fig. 8C) regardless of the levels of Siglec-1 expression. See Fig. 5. Thus, resistance to cytoplasmic Ebola viral entry already described in Raji cells is not overcome by the capacity of Siglec-1 to enhance viral uptake. However, cytoplasmic viral entry was reduced on permissive primary DCs derived from a Siglec-1 null individual which naturally lack the expression of Siglec-1 receptor, especially upon DC activation. See Fig. 8D. Overall, these data indicate that on activated DCs, Siglec-1 is a host attachment factor that modulates the binding, uptake and trafficking of Ebola viruses to a sac-like virus-containing compartment, where viruses are stored and facilitate viral entry into the cytoplasm. See Fig. 10.

### Example 4: Generation and characterization of novel mAbs against Siglec-1

The previous results show that, beyond their already known effect against HIV-1, α-Siglec-1 mAbs offer protection against Ebola viral uptake and fusion. Thus, the screening for novel α-Siglec-1 mAbs with broad antiviral activity was undertaken. First, the generation of α-Siglec-1 murine mAbs recognizing the viral binding epitope localized in the V-set domain of Siglec-1, which interacts directly with sialylated ligands (i.e. such as the ones displayed by gangliosides exposed on Ebola and HIV-1 membranes), was attempted. When mice were immunized with linear Siglec-1 peptides from the V-set domain, the generated polyclonal Abs recognized those peptides, but failed to bind to the native receptor. See Fig. 11. This suggested the requirement of a native three-dimensional immunogen to generate receptor-binding mAbs. Thus, mice were immunized with murine and human cells expressing high levels of the native human Siglec-1 receptor. After screening over a thousand hybridomas from these immunizations, five novel clones were selected for their capacity to produce mAbs that recognize both the cellular Siglec-1 receptor and a recombinant Siglec-1 protein. Upon purification, mAb specificity was further confirmed by mAb binding to cells transfected with a plasmid coding for human Siglec-1, that was absent in Siglec-7 or Siglec-5 transfected cells. See Fig. 12.

To further define the Siglec-1 binding region of novel mAbs 3F1, 5B10, 1F5, 4E8 and 6G5, a recombinant Siglec-1 mini-protein that expresses the V-set domain plus three IgG-like domains fused to an Fc domain bound to an ELISA plate was employed. Siglec-1 mini-protein was effectively detected by novel and commercial mAbs indicating that all binding epitopes located in the four terminal domains. See Fig. 13A. Sequence analysis revealed that novel mAbs displayed 93 to 98 % germline identity for heavy chains and 95 to 98 % for light chains, with a distinctive germline gene usage indicative of their unique B cell lineage origin. See Fig. 13B. Novel mAbs displayed two distinct isotypes. See Fig. 13B. Apparent affinity of novel and commercial α-Siglec-1 mAbs was estimated by surface plasmon resonance, where a full human Siglec-1 protein was covalently immobilized to a sensor chip. Measurement of the equilibrium dissociation constant (KD1) for Siglec-1 of each mAb revealed that all displayed high affinities below the nanomolar range. See Fig. 13C.

Relative competition for binding to Siglec-1 between distinct mAbs was also analyzed using surface plasmon resonance, and two representative experiments are shown in Figure 13D. Two mAbs were injected sequentially in a single cycle. See Fig. 13D, blue lines. Competition of the second mAb with the first mAb (Fig. 13D, top graph) was observed when a similar signal was retrieved when either a buffer followed by the first mAb or co-injected with the first mAb (grey lines) were used as controls. No competition was observed (Fig. 13D, bottom graph) when signal was similar to that obtained with buffer followed by the second mAb (red lines). All cross-competition analyses (Fig. 14) allowed calculation of the percentage of binding inhibition of the second mAb in the presence of the first mAb. See Fig. 13E. Novel mAbs 5B10, 1 F5, on the one hand, and 4E8 on the other competed with commercial mAbs 7-239 or 7D2, respectively, while 3F1 competed with both 5B10 and 1F5. See Fig. 13E and Fig. 14. This is more likely due to the steric hindrance caused by mAbs binding to the same epitope or in close proximity, although conformational changes induced by the first mAb could also hide the epitope recognized by the second mAb. By these combinatorial competitions a relative antigenic binding map was defined, where overlap represents high degree of competition between tested mAbs. See Fig. 13F. It was found that some mAbs bound to Siglec-1 in the presence of others, thus suggesting that they recognize distinct epitopes and that, therefore, could be used as a combination therapy to maximize efficacy.

### Example 5: Novel mAbs against Siglec-1 inhibit HIV-1 and Ebola viral uptake

Subsequently, it was assessed if the novel α-Siglec-1 mAbs could stop HIV-1 and Ebola viral uptake. First, fluorescent HIV-1 VLPs lacking the viral envelope glycoprotein (HIV-1Gag-eGFP VLP) were employed. A constant concentration of HIV-1Gag-eGFP VLP was mixed with increasing concentrations of the novel or commercial α-Siglec-1 mAbs and later added in combination to Raji Siglec-1 cells. In this competition, the commercial mAb 7-239 and the novel mAbs 1F5, 5B10 and 3F1 were able to block 100% of the HIV-1 VLP uptake. See Fig. 15A. Moreover, the concentration of the novel mAb 3F1 that inhibited viral uptake a 50% (IC50 value) was significantly lower than that of the commercial mAb 7-239 (P<0.0001). See Fig. 15B. Combinations of mAbs 3F1, 5B10 and 1F5 were not able to further improve the blocking capacity of each single mAb. See Fig. 15C, top graph. These results concur with the capacity of these particular mAbs to compete with each other (Fig. 13E) and suggest that they bind to the same viral binding epitope or in close proximity. However, novel mAbs 4E8 and 6G5 showed an incomplete viral blocking capacity (Fig. 15A) that was overcome when added in combination. See Fig. 15C, bottom graph. This data agrees with the inability of these mAbs to compete with each other or with other novel mAbs (Fig. 13E) and support the hypothesis that they bind further away from the viral binding epitope.

However, in competition experiments performed with Ebo-GPVP40-eGFP VLP, all the novel α-Siglec-1 mAbs inhibited 100% of the Ebo-GPVP40-eGFP uptake in Raji Siglec-1 cells. See Fig. 15D. Since Ebola virus is approximately six-fold larger in length than HIV-1, in this case the distant binding of 4E8 and 6G5 mAbs may be sufficient to cause total blocking via steric hindrance. Again, the IC50 values of the novel mAb 3F1 for Ebo-GPVP40-eGFP were significantly lower than those of commercial mAbs (P<0.0001). See Fig. 15E.

Next, the capacity of the novel α-Siglec-1 mAbs to block viral uptake on primary DCs was assessed. IFNα and LPS levels are not only augmented throughout the course of Ebola virus infection but are also triggered by HIV-1 infection due to type I interferon responses and to an increased translocation of bacteria from the intestinal lumen. When the uptake of wild type HIV-1NL4.3 was compared, LPS and IFNα-DCs showed a higher viral capture capacity than iDCs (P≤0.0004; Fig. 15F), as previously shown for Ebo-GPVP40-eGFP. See Fig. 4D). Pre-treatment with novel α-Siglec-1 mAbs also inhibited DC uptake of HIV-1NL4.3, especially on activated cells (P≤0.0006). See Fig. 15G. Accordingly, pre-treatment with novel α-Siglec-1 mAbs significantly blocked Ebo-GPVP40-eGFP VLP capture on activated DCs (P<0.0001; Fig. 15H) and on IFNα-treated BDCA1-positive myeloid cells directly isolated from lymphoid tissues (P=0.0167; Fig. 15l), which also rely on Siglec-1 for HIV-1 uptake. In consequence, the novel α-Siglec-1 mAbs could be used alone or in combination to block HIV-1 and Ebola viral uptake in activated DCs and primary myeloid cells.

### Example 6: Novel mAbs against Siglec-1 inhibit HIV-1 trans-infection and Ebola viral fusion

As opposed to their high susceptibility to Ebola virus infection, DCs are largely resistant to HIV-1 infection. However, DCs can mediate HIV-1 transfer to susceptible target cells via trans-infection. This process is potently boosted upon DC activation and relies on HIV-1 binding to Siglec-1, allowing for viral transmission and infection of target CD4+ T cells. Thus, the capacity of the novel α-Siglec-1 mAbs to block HIV-1 trans-infection was verified. Distinct DCs mock-treated or pre-incubated with α-Siglec-1 or isotype mAbs were pulsed with equal amounts of HIV-1NL4.3 and co-cultured with a CD4+ reporter cell line. Luciferase induced by HIV-1NL4.3 infectivity on target cells revealed that activated DCs had the highest capacity to trans-infect HIV-1NL4.3 (P≤0.0042). See Fig. 16A. As expected by their viral blocking capacity on activated DCs (Fig. 15G), the novel α-Siglec-1 mAbs effectively blocked HIV-1 trans-infection. See Fig. 16B.

Finally, the capacity of the novel mAbs α-Siglec-1 to block Ebola viral fusion was also assayed. Ebola viral fusion on activated DCs was effectively reduced by novel α-Siglec-1 mAbs (P≤0.0067; Fig. 16C), where cathepsin B inhibitor CA-074 Me was included as a potent blocking control of fusion. This effect extended to other myeloid cells that are also targets of Ebola virus infection and express Siglec-143, such as IFNα-treated monocytes (Fig. 16D), or to IFNα-treated BDCA1-positive myeloid cells directly isolated from lymphoid tissues. See Fig. 16E. Fusion of Ebola virus containing the Marburg glycoprotein was also inhibited by novel α-Siglec-1 mAbs as expected by the envelope glycoprotein independent mode of viral recognition of this receptor, which relies on binding to viral membrane gangliosides. See Fig. 16F. Overall, the novel α-Siglec-1 mAbs acting on activated antigen presenting cells cross-protect against Ebola virus fusion and HIV-1 trans-infection.

### Example 7: Wild-type virus assays

The capacity of the anti-Siglec-1 mAbs for interfering with filovirus productive infection was assayed. The following human cell models were employed: i) LPS and IFNa-treated monocyte-derived DCs; ii) IFNa-treated monocytes; and iii) IFNa-treated BDCA-1-positive myeloid cells derived from lymphoid tissues (tonsils).

The assay was conducted using the wild-type viruses, Zaire Ebola virus H. sapiens-tc/GIN/2014/Makona-C05 (ZEBOV) and Marburg virus strain Musoke (MARV) (both from EVAg). The virus were handled in BSL4 facilities and were grown in Vero E6 cells (ATCC). Viral titers were determined by using a plaque-forming assay as described previously. See Duehr J, et al., J Virol. 2017; 91:e00652-17. Briefly, distinct amounts of ZEBOV and MARV were added to confluent Vero E6 cell monolayers in 12-well plates. Cells were overlaid with minimum essential medium (MEM) containing 0.64% agarose (Oxoid), and after 4 days of incubation at 37°C, cells were fixed with 3.7% paraformaldehyde. Plaques were stained using a polyclonal anti-EBOV mouse serum (1:1,000), an anti-mouse secondary antibody conjugated to horseradish peroxidase (HRP) (Sigma), and Trueblue reagent (KPL) to visualize and count the plaques.

Monocyte-derived DCs, IFNa-treated monocytes or IFNa-treated BDCA-1-positive cells directly isolated from lymphoid tissues (tonsils) were pre-incubated with 20 ug/ml of commercial anti-Siglec-1 mAbs 7-239 7D2 (both from Abcam), novel anti-Siglec-1 mAbs, an IgG1 isotype control (BD Biosciences), other relevant mAbs (such as TIM-4, TIM-1, DC-SIGN, Mannose Receptor, GAS6, Protein S) and 50 uM of the cathepsin B inhibitor CA-074 Me (Enzo Life Sciences) as a fusion control. Cells were pulsed with saturating titers of ZEBOV or MARV for several days at 37°C in a kinetic experiment. Supernatants were harvested, and productive replication was be assessed by a plaque forming unit assay or a PCR-based viral detection method.

### Example 8: Animal models

The therapeutic potential of the anti-Siglec-1 mAbs during filovirus infection was evaluated in an in vivo model. The protective effect of the anti-Siglec-1 mAbs was assayed in a macaque model by making an EBOV challenge. Other mAbs that block known targets of EBOV interaction (i.e. TIM-4, TIM-1, DC-SIGN, Mannose Receptor, GAS6, Protein S) were used for comparison. See Baseler L, et al., Annu Rev Pathol Mech Dis. 2017; 12:387-418 and Wong G, et al., Expert Opin Biol Ther. 2018; 18(2):159-173.

As all these mAbs recognize human targets, their cross-reactivity with their homologues in macaques would be confirmed. This cross-reactivity was positive fors ome of the clones tested (e.g. mAb 7D2). See Jaroenpool J, et al., Cell Immunol. 2007; 250(1-2):91-104. Monocytes and peritoneal macrophages from macaques were tested for cross-reactivity against macaque homologues with anti-human mAbs. The applicable guidelines for animal welfare were followed during the assays.

The mAbs displaying cross-reactivity in macaques were further tested for their capacity to protect macaques from an EBOV challenge. Rhesus macaques received combinations of one, two or three mAbs (including a mAb against Siglec-1 and other relevant mAbs such as TIM-4, TIM-1, DC-SIGN, Mannose Receptor, GAS6 and Protein S) or an isotype control. Animals were then challenged in a BSL4 facility with a uniformly lethal dose of EBOV and monitored daily for plasma viremia, Ebola virus disease-related markers and survival. The monitoring included (a) the determination of transaminases and creatinine in plasma, and (b) as platelet and D-dimer measurement, since Ebola virus disease in macaques is characterized by hepatic and renal dysfunction, as well as coagulopathy. See Geisbert T, et al., Am J Pathol. 2003; 163(6):2347-2370. Viral challenge was performed before and after mAb infusions, for testing the post-exposure and pre-exposure potential of these combinations.

In addition, the combinations of anti-Siglec-1 mAbs and mAbs directed towards the envelope glycoprotein of specific EBOV strains, such as Zaire Ebola virus H. sapiens-tc/GIN/2014/Makona-C05 were also assayed. Viral challenge was performed as described previously, before and after mAb infusions, for testing the post-exposure and pre-exposure potential of these combinations.

### Example 9: Anti-Siglec-1 mAbs inhibit Arenavirus uptake

The capacity of anti-Siglec-1 mAbs for inhibiting the viral uptake of Arenavirus was assayed. Non-infectious Junin VLPs tagged with fluorescent eGFP (JUNV eGFP-VLP) were generated by transfecting HEK-293Tcells with a plasmid encoding for the structural Junin Z protein. The Junin VLPs were added to Raji Siglec-1 cells stably transfected or to monocyte-derived DCs activated with lipopolysaccharide (which upregulate Siglec-1 expression). Viral uptake was analyzed by FACS or confocal microscopy in the presence of an α-Siglec-1 monoclonal antibody (i.e. 7-239 mAb) or an isotype control.

The experimental results show that Raji Siglec-1 cells captured a higher amount of Junin VLPs than Raji cells. Moreover, the anti-Siglec-1 7-239 mAb was able to block VLP uptake significantly (p=0.0159; Mann-Whitney). See Fig. 17. Pre-incubation of activated DCs did not affect the uptake of Junin VLP. However, the presence of the anti-Siglec-1 7-239 mAb blocked efficiently the capture of Junin VLPs by DCs (p≤ 0.0002; one sample t-test). See Fig. 18. Thus, anti-Siglec-1 mAbs could be used for blocking Arenavirus uptake in activated DCs and primary myeloid cells.

### Example 10: Generation of antigen-binding fragments (Fabs) and their blocking effect against viral uptake by DCs mediated by Siglec-1

Fabs were generated from the 1F5, 5B10, 3F1 and 4E8 mAbs using Pierce^{™} Fab Micro Preparation Kit (Cat. No. 44685; ThermoFisher Scientific) or Pierce^{™} Mouse IgG1 Fab and F(ab')2 Micro Preparation Kit (Cat. No. 44680; ThermoFisher Scientific) according to the manufacturer's instructions. S ee Fig. 19. The yield for the 1F5 and 5B10 Fabs was around 10%, thus these Fabs were selected for further testing. The yield for the 3F1 and 4E8 Fabs was lower. The Fc region labelling assay for the 1F5 and 5B10 Fabs showed that no Fc region was present. See Fig. 20.

The purified the 1F5 and 5B10 Fabs were then tested for determining their blocking effect on the Siglec-1 receptor. To that purpose, DCs were incubated for 15 minutes with the Fabs, original mAbs or control mAbs at 40 µg/ml and then fluorescent HIV VLPs or fluorescent WT HIV was added to be incubated for 1 hour at a concentration of 20 µg/ml at 37oC and 5% CO2. The results demonstrate how in both cases the 1F5 and 5B10 Fabs showed the same efficacy blocking Siglec-1 in DCs as their original mAbs or control mAbs. See Fig. 21 and 22.

### Example 11: Siglec-1 facilitates SARS-CoV-2 recognition and cellular uptake and capture

The Siglec-1 capacity for SARS-CoV-2 recognition was tested by assessing viral uptake in two complementary cellular models. First, Raji B cells transfected with different lectins were used. The capacity of these cells for SARS-CoV-2 uptake was measured. Raji cells were pulsed with equal amounts of SARS-CoV-2, extensively washed, lysed, and assessed by ELISA to measure the cell-associated amount of viral nucleocapsid protein (NP). While Raji Siglec-1 cells effectively captured SARS-CoV-2, Raji cells transfected with Siglec-5, Siglec-7, DC-SIGN or devoid of any of these lectins did not. See Fig. 23A. Siglec-1 uptake of SARS-15 CoV-2 relied on the recognition of sialylated ligands, which most likely are gangliosides exposed on viral membranes, as previously described for HIV-1 and Ebola virus (Izquierdo-Useros N, et al., PLoS Biol. 2012; 10:e1001315, Izquierdo-Useros N, et al., PLoS Biol. 2012; 10:e1001315, Pérez-Zsolt D, et al., Nat Microbiol 2019; 4:1558-1570, Puryear W,e t al., Proc. Natl. Acad. Sci. USA 2012; 109:7475-7480). This was confirmed using the Raji cell line transfected with the Siglec-1 mutant R116, which contains a mutation critical for sialic recognition (Hartnell A, et al., Blood 2001; 97:288-296, Puryear W, et al., PLoS Pathog 2013; 9:e1003291) and could not trap SARS-CoV-2 (Fig. 23A), as previously shown for HIV-1 and Ebola virus (Pérez-Zsolt, 2019, Puryear, 2019, supra). Finally, and to further confirm if SARS-CoV-2 interaction was mediated by Siglec-1, Raji cells were pre-treated with an α-Siglec-1 mAb 7-239 previously shown to decrease HIV-1 and Ebola virus uptake (Izquierdo-Useros, 2012b, Pérez-Zsolt, 2019, Puryear, 2013, supra). While isotype mAb had no inhibitory effect, pre-treatment with 7-239 mAb clearly reduced SARS-CoV-2 uptake. See Fig. 23B.

Second, monocyte-derived macrophages and dendritic cells (DCs) were used to verify the role of Siglec-1 receptor on primary myeloid cells. Siglec-1 is upregulated on myeloid cells upon SARS-CoV-2 infection (Bedin A, et al., Infectious Diseases (except HIV/AIDS) 2020). This is due to the presence of activating immune signals such as type I interferons (e.g., interferon alpha or IFNα), which are augmented throughout viral infections, and increase Siglec-1 expression on macrophages and DCs (Pino M, et al., Retrovirology 2015; 12:37, Puryear, 2013, supra). Myeloid cells previously treated or not with IFNα were pulsed with equal amounts of SARS-CoV-2, washed, and assayed with an ELISA as described for Raji cells. See Fig. 23C. Of note, all myeloid cells trapped SARS-CoV-2, but macrophages had a higher capacity (Fig. 23C), which correlates with the higher Siglec-1 basal expression compared to DCs. Moreover, IFNα activated macrophages, which display higher amounts of Siglec-1 (Pino, 2015, supra), were the cells with higher uptake capacity. See Fig. 23C.

To investigate whether SARS-CoV-2 viral interaction was mediated by Siglec-1, an α-Siglec-1 mAb 7-239 was used. While isotype mAb had no inhibitory effect, pre-treatment with 7-239 blocked SARS-CoV-2 uptake specially in macrophages. See Fig. 23C. In contrast, only IFNα activated DCs trapped SARS-CoV-2 in a Siglec-1 dependent manner, while immature DCs, which express higher levels of DC-SIGN (Pérez-Zsolt, 2019, supra) did not effectively capture SARS-CoV-2. The complementary approaches of Siglec-1 de novo expression, combined with the blocking effect of specific mAbs, supports that Siglec-1 is a central molecule mediating SARS-CoV-2 uptake.

### Example 12: Siglec-1 facilitates SARS-CoV-2 trans-infection to target cells

To assess the relevance of Siglec-1 for SARS-CoV-2 trans-infection, distinct Raji cells were pulsed with equal amounts of an HIV-1 construct lacking any viral glycoprotein, but containing a luciferase reporter gene, which were pseudotyped with the SARS-CoV-2 spike protein. Cells were extensively washed and co-cultured with different target cell lines expressing either ACE2 or ACE2 and TMPRSS2 in combination. While Raji Siglec-1 cells effectively transferred SARS-CoV-2 to both cellular targets, Raji cells transfected with DC-SIGN or devoid of any of these lectins did not. See Fig. 24A. Moreover, trans-infection relied on Siglec-1 uptake of SARS-CoV-2 via recognition of sialylated ligands, as the Siglec-1 mutant R116 did not trans-infect SARS-CoV-2. See Fig. 24A. No direct infection of Raji cells was detected, as seen when assessing pulsed cells in the absence of ACE2-expressing cellular targets.

Next, primary myeloid cells activated or not with IFNα to up-regulate Siglec-1 expression were tested. See Fig. 24B. An α-Siglec-1 mAb 7-239 was used to explore whether SARS-CoV-2 trans-infection was mediated by Siglec-1. While isotype mAb had no inhibitory effect, pre-treatment with 7-239 blocked SARS-CoV-2 trans-infection to ACE2-expressing cellular targets by IFNα activated DCs. See Fig. 23C. In contrast, non-activated DCs, which mostly expressed DC-SIGN (Geijtenbeek T, et al., Cell 2000; 100:575-585), did not mediate trans-infection. See Fig. 23C. No direct infection of DCs was detected, as seen when assessing pulsed cells in the absence of ACE2-expressing cellular targets. Thus, SARS-CoV-2 uptake via Siglec-1 is able to allow for trans-infection of cellular targets expressing ACE2 and TMPRSS2 receptor.

### Example 13: Humanization of anti-CD169-mAbs

From the inventors previous study, the inventors selected three anti-CD169 monoclonal antibodies (mAbs) based on their ability to recognize CD169/Siglec-1 cellular receptor and block HIV 1 virus-like particle (VLP) uptake with highest efficiency (Perez-Zsolt et al., 2019b). The genomic sequence of these murine antibodies (clones named #1F5, #3F1 and #5B10) was extracted and sequenced from the corresponding hybridomes following the standard operating procedure of GenScript. Then, the inventors generated two humanized variants for the selected three candidates based on chimeric and complementarity-determining region (CDR)-engrafted technologies (Fig. 25A).

In order to generate CDR-engrafted humanized antibodies (named h0), mouse variable regions from heavy and light chains (VH and VL, respectively) of #1F5, #3F1 and #5B10 antibodies were aligned by Ig Blast-NCBI to get the closest human related V gene sequences and to identify the framework residues (FRs) and complementarity-determining regions (CDRs). For the humanization process, mouse residues forming the CDRs were retained while the FRs residues that were not matching between mouse and human germline were changed to the residue present in the human V gene. The respective six monoclonal antibodies were cloned into the human IgG1 format, the most abundant IgG subclass in human, that also retains both good complement activity and Fc-receptor mediated effector functions, as well long half-life of three weeks (ref/review). IgG1 can also be further engineered to extend the half-life and to avoid interaction with complement and/or Fc-receptor mediated effector functions. Chimeric and humanized CDR-engrafted antibodies were produced by transient transfection in HEK293FS cells and purified using Protein A. The inventors confirmed purity and integrity of the antibodies, as well as that being human IgG1/ kappa by SDS-PAGE and Western blot analysis in both reducing and non-reducing conditions.

To examine a functional consequence of this humanization processes, the inventors measured if these new antibodies maintained their blocking capacity to prevent viral binding of enveloped viruses. Thus, the inventors performed a functional competition assay with Raji-Siglec-1 cells and HIV-1 viral like particles (VLPs) expressing eGFP as reporter gene, so that binding of viral particles is then measured by Flow Cytometry (FACS). In this way, the inventors could compare the effect of newly generated antibodies targeting CD169 to the original murine mAbs, that serve as corresponding positive blocking control (Fig. 25B-D). Thus, the inventors measured the half maximal inhibitory concentration (IC50) to facilitate the comparison (Fig. 25E). As shown, differences in IC50 do not seem substantial.

Although IgG antibodies are relatively stable molecules, they are subject to a variety of degradation reactions that can occur during manufacturing, formulation, and storage. Post-translational modifications have the potential to reduce affinity/potency, stability, and homogeneity of an antibody, resulting in complications to downstream process development. Commonly, the antibody VH and VL sequence contain multiple motifs subjected to chemical instability. In particular, the risks of chemical liabilities that can impact antigen binding, such as glycosylation, deamidation, oxidation, and isomerization in the antibody CDR sequences, need to be controlled through formulation development or eliminated by replacing the amino acid motif displaying the chemical instability. However, only a subset of these motifs results in actual chemical modification, other do almost always occur and may even be required for antigen binding. Therefore, experimental assessment of the effect of potential instabilities is required for optimization. Hence, the inventors decided to further improve humanized CDR-engrafted #1F5 and #3F1 monoclonal antibodies by eliminating potential sequence liabilities present in the VH and VL of these antibodies in the early phase of the antibody engineering process. The inventors used the structure-based antibody prediction server SAbPred for this investigation. Detected potential sequence liabilities are summarized in Table 1.

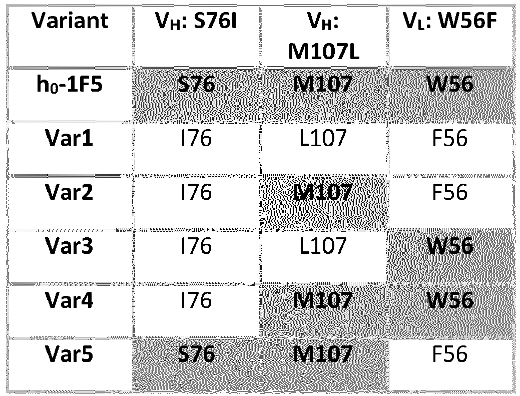

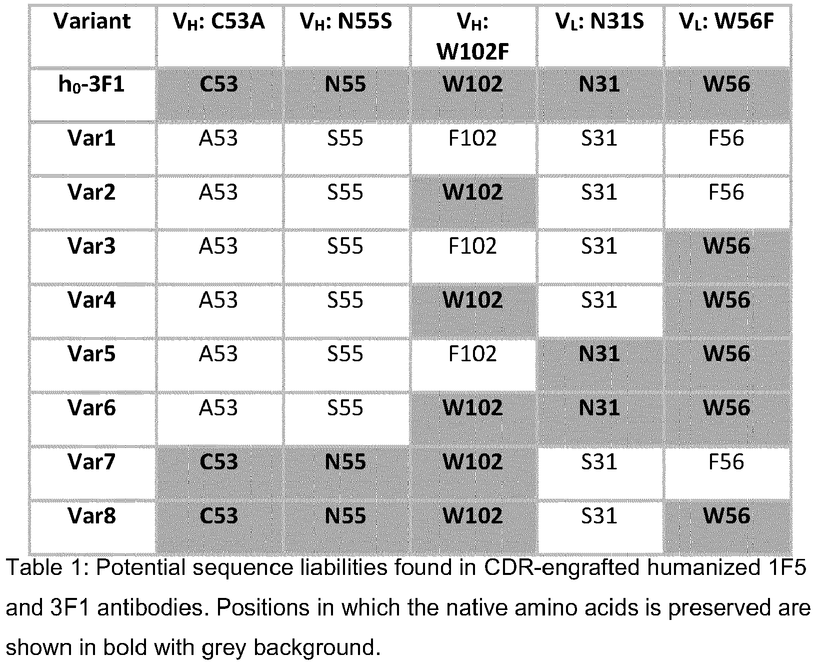

Subsequently, each identified liability was evaluated by looking in scientific publications which potential hotspots have already proven to have a negative impact during the manufacturing process of biological drugs. For each identified liability, the inventors have further evaluated whether these residues form part of the CDRs or of the FRs. Potential liabilities present in the human V region and forming the FRs were retained since those conserved regions are less solvent accessible, and hence less susceptible to chemical instability as they are buried within the 3-D protein structure, moreover they could play an important role for the correct antibody folding. The inventors focused the evaluation by introducing mutations in the potential hotspots mapped in CDRs because of the relatively flexible and solvent accessible nature of CDR loops (Fig. 26A).

A common chemical degradation pathway of protein therapeutics is oxidation that can be induced by light exposure, thermal stress, or impurities such as metal ions and peroxides. Although methionine is the most susceptible residue to oxidation in mAbs, other residues such as tryptophan, cysteine, tyrosine and histidine have also been reported. Tryptophan residues are commonly found in CDRs due to their structural features and hydrophobic properties that confer niche recognition points that facilitate strong and specific binding between mAbs and their respective antigens (Hageman et al., 2018). Other frequent chemical modifications of interest for mAbs are deamidation and isomerization. Deamidation of asparagine residues and aspartate isomerization of biological pharmaceuticals is a major cause of degradation if the pharmaceutical proteins are not formulated and stored appropriately. These chemical mechanisms are pH- and temperature-dependent and have several competing transition states (Lu et al., 2019). On the other hand, IgGs can contain N-linked glycans in the variable domains, the socalled Fab glycans, in addition to the Fc glycans in the CH2 domains. These Fab glycans are acquired following introduction of N-glycosylation sites during somatic hypermutation and thereby constitute part of the physiological repertoire of antibodies. Furthermore, Fab glycans are usually localized close to antigen-binding sites and can influence antigen binding and contribute to affinity maturation. Conversely, some groups reported that glycans in the variable domains enhance antibody solubility and stability, while recent literature indicates that Fab glycans may trigger aggregation (van de Bovenkamp et al., 2018).

The analysis of h0-1F5 antibody showed the presence of a methionine in the CDR3 of the heavy chain and one tryptophan in the CDR2 of the light chain at risk of oxidation that can negatively impact antigen binding. The solutions tested in the inventors study were to replace these residues with a Leucine and a Phenylalanine, respectively. Furthermore, S76 in framework 3 was replaced by a I, normally found in closest human VH germline sequences.

The analysis of h0-3F1 antibody showed that the CDR2 in the heavy chain contains an unpaired cysteine that can trigger folding problems and aggregation, thus it was replaced by an alanine. It also showed a motif at risk of deamidation (N55; G56) in the same region that was exchanged into S55; G56. Moreover, a tryptophan at risk of oxidation in CDR3 of the heavy chain was replaced by the more stable phenylalanine. In the light chain instead, a N-linked glycosylation site is present in CDR1 and since controversial results are reported for Fab glycans, the effect of this modification can only be determined experimentally. This same residue is also included in a motif at risk for deamidation (N31; S32), therefore the inventors removed both liabilities by replacing N31 by a Serine that is a non-glycosylated residue. In addition, tryptophan at position 56 in the light change, as potential target for oxidation, was replaced by phenylaniline.

In order to analyse if these modifications alter the functionality of humanized antibodies, the inventors generated 5 variants of h0-1F5 antibody and 8 variants of h0-3F1 antibody by the combining stabilizing mutations described above (Fig. 26A). The inventors then performed a competition assay between HIV 1Gag eGFP VLPs and those anti-CD169 mAbs variants at a single concentration (Fig. 26B). All mutations introduced in h0-1F5 antibody loose viral entry blocking capacity except for variant 4 with S76I residue exchange in the light chain. On the other hand, mutations introduced in h0-3F1 antibody loose viral entry blocking capacity except for variant 6 that lost C53 unpaired cysteine and the deamination motif in the heavy chain. These two variants that maintained blocking activity similar to the parental antibody (named h1-1F5 and h1-3F1) were selected as lead candidates and were further investigated. First, the inventors performed the same functional competition assay with Raji-Siglec-1 cells and HIV-1Gag-eGFP-VLP with decreasing concentrations of antibody (Fig. 26C) to determine corresponding IC50 (Fig. 26D). Thus, h1-3F1 mAb maintain similar blocking capacity than parental antibodies, while h1-1F5 showed a higher IC50, similarly to h0-1F5. Because measured IC50 are dependent on assay conditions, they cannot be readily compared across experiments not performed at the same time. This explains differences between values in Fig. 25E and Fig. 26D.

### Example 14: Humanized anti-CD169-mAbs block viral entry into primary myeloid cells

The inventors next assessed if humanized anti-CD169 mAbs could block HIV-1 viral uptake and trans-infection. For this, the inventors have used primary monocyte derived dendritic cells (DCs) activated with LPS because it has been previously reported that viral capture is higher compared to iDCs (Izquierdo-Useros et al., 2007; Perez-Zsolt et al., 2019a; Puryear et al., 2013). The inventors used fluorescent HIV-1NL4.3 Gag-iGFP, which is as infectious as native HIV in single-round infectivity assays (Hübner et al., 2007). LPS-activated DCs were pre-incubated with anti-CD169 mAbs or an isotype control before adding a constant amount of HIV-1NL4.3 Gag-iGFP. Pre-treatment with humanized anti-CD169 mAbs inhibited DC capture at the same level as murine anti-CD169 mAbs (Fig. 27A). The inventors also tested the capacity of humanized anti-CD169 mAbs to block HIV-1 trans-infection. For this purpose, the inventors used HIV-1NL4.3 infectious virus. LPS treated DCs were pre-incubated with anti-CD169 mAbs or isotype control and pulsed with equivalent amounts of HIV-1NL4.3. After extensive washing, DCs were co-cultured with CD4+ reporter TZM-bl cell line, and luciferase induction on these cells by HIV 1NL4.3 was measured to assess trans-infection. As previously seen for viral uptake, humanized anti-CD169 mAbs efficiently blocked HIV-1 trans-infection (Fig. 27B).

To assess the capacity of humanized anti-CD169 mAbs to block Ebola uptake in primary DCs the inventors employed fluorescent Ebola VLPs bearing EBOV GP (Ebo-GPVP40-eGFP VLPs). LPS-activated DCs were pre-incubated with anti-CD169 mAbs or an isotype control before adding a constant amount of Ebo-GPVP40-eGFP VLPs. Pre-treatment with humanized anti-CD169 mAbs inhibited DC capture at the same level as murine anti-CD169 mAbs (Fig. 27C). The inventors also tested the capacity of humanized anti-CD169 mAbs to reduce cytoplasmatic entry in activated DCs. To assess if new anti-CD169 mAbs could impact on cytoplasmic viral entry into activated DCs, the inventors employed Ebola VLPs bearing the BlaM-VP40 chimeric protein and EBOV GP (Ebo-GPVP40-BlaM VLPs). LPS treated DCs were pre-incubated with anti-CD169 mAbs, isotype control or with the CTSB inhibitor CA-074 me, a potent cytoplasmatic viral entry inhibitor as control (Martinez et al., 2010) and pulsed with equivalent amounts of Ebo-GPVP40-BlaM VLPs. After extensive washing, cytoplasmatic entry was measured by flow cytometry. As previously seen for viral uptake, humanized anti-CD169 mAbs efficiently blocked EBOV cytoplasmatic entry (Fig. 27D).

Finally, the inventors assessed if humanized anti-CD169 mAbs could block SARS-CoV-2 viral uptake and trans-infection. For this, the inventors have used again LPS activated DCs. To test the blocking of viral uptake, these cells were pre-incubated with anti-CD169 mAbs or an isotype control before adding a constant amount of SARS-CoV-2 virus. Pre-treatment with humanized anti-CD169 mAbs inhibited DC capture at the same level as murine anti CD169 mAbs (Fig. 27E). The inventors also tested the capacity of humanized anti-CD169 mAbs to block SARS-CoV-2 trans-infection. For this purpose, LPS-treated DCs were pre-incubated with anti-CD169 mAbs or isotype control and pulsed with equivalent amounts of SARS-CoV-2. After extensive washing, DCs were co-cultured with HEK-293T over-expressing ACE2 and TMPRSS2, and a viral nucleocapsid detection kit was used to assess trans-infection by luminometry. As previously seen for viral uptake, humanized anti-CD169 mAbs efficiently blocked SARS-CoV 2 trans-infection (Fig. 27F).

### Example 15: Stability of humanized anti-CD169-mAbs

In summary from data shown in Figures 2 and 3, the inventors have produced two stabilized variants of the humanized antibodies anti-CD169 #1F5 and #3F1 with same functional activity compared to the parental non-stabilized antibody. Then, the inventor sanalyzed further these antibodies under oxidative stress evaluating their aggregation propensity and binding affinities.

Antibodies under oxidative stress with hydrogen peroxide treatment were characterized by SEC-MALS for size distribution of resulting protein solution (Fig. 28A). No aggregation or degradation products were detectable after inducing oxidative stress and antibodies looked pure with a monomeric peak. Additionally, the inventors performed the same functional competition assay with Raji-Siglec-1 cells and HIV-1Gag-eGFP-VLP with decreasing concentrations of antibody to assess if the stabilized humanized anti-CD169 mAbs maintain their blocking capacity upon oxidative stress (Fig. 28A). As illustrated, while h1-1F5 and the parental h0-1F5 loose completely their blocking capacity, h1-3F1 mAb maintain similar blocking capacity than parental antibodies even after oxidative stress.

Then, the inventors analyzed the binding affinity of these treated antibodies for their primary target that is CD169 receptor by IBIS MX96 Technology (Fig. 29). Affinity measurement were similar when comparing humanized and stabilized variants after oxidative stress. Interestingly, both h0-1F5 and stabilized h1-1F5 bind weakly to the CD169 target and dissociation constants (KD) could not be determined. This correlates with an increase in the calculated IC50 upon humanization process (Fig. 25E and 26D). On the other hand, both h0-3F1 and stabilized h1-3F1 bind equally well to CD169 target and the calculated KD ranged 4.5E8 M and 7.19E8 M, correlating with a lower and more conserved IC50 upon humanization and stabilization processes (Fig. 26D).

Finally, the inventors performed an epitope binning assay that is a competitive immunoassay to characterize all these antibodies against CD169 receptor. The inventors included to two additional anti-CD169 mAbs (1C3H9E6 and 6H964C2 clones) that are unable to block viral binding to myeloid cells, but recognise CD169 with high affinity (Fig. 29). Based on the low signal for all experiments with 1F5 variant, this antibody is very sensible to the regeneration with 10nM acetate buffer and 100mM ethanolamine required during the procedure (Fig. 30). This correlates with the measured activity for this antibody under oxidative stress. When any of 3F1 variants are spotted in the platform, only 1C3H9E6, 6H964C2 and anti-CD169 (#7-239 commercial clone) can bind to CD169 recombinant protein after binding to the 3F1 antibody (Fig. 31). Interestingly, 1C3H9E6 and 6H964C2 non-blocking mAbs allow binding of any 3F1 variants indicating complementarity and non-competition for epitope recognition (Fig. 32).

### Example 16: Detection of virus and VLPs using a Siglec-1 mini-protein (mS1) bead platform

Siglec-1 mini-proteins (mS1) were prepared as described above. See Sec. 4, General Procedures. The mS1 were bound to 4 µm diameter latex beads (ThermoFisher Scientific) for simplifying the isolation and analysis of VLPs and full viral particles. Protein G was used to ensure the specific mS1-coating of the latex beads, as Protein G binds the Fc region of IgG with high affinity.

### 1. Detection of enveloped viral-like particles

The efficacy of the mS1-beads for detecting HIV-1 and EBOV VLPs was tested by flow cytometry. The VLPs contained GFP for facilitating its detection and measurement. Control beads not containing mS1 were used for comparative purposes.

The specificity of the mS1-bead platform was verified using anti-Siglec-1 antibodies, which served as a negative controls. See Fig. 33A and 33B. The sensitivity of the platform permits discriminating the amounts of VLPs present in the sample, as the mean fluorescence intensity (MFI) of the sample would vary the amount of VLPs present. See Fig. 33C.

### 2. Detection of full viral particles

A staining step with specific antibodies was added to the protocol above for detecting any viral proteins, including antigens inside the viral envelope. To this purpose, the effect of fixation and permeabilization on the mS1 bead platform for detecting the HIV p24 protein was analyzed using recombinant incompetent viral particles (RIVPs) which also included GFP. See Fig. 34A and 4B. Moreover, the detection of full virus such as HIV-1NL4-3 with anti-p24 antibody labeled with a RD1 fluorophore was additionally tested. See Fig. 35A and 8B.

The results above demonstrate that the mS1 bead platform could be useful in the specific detection and quantification of VLPs and full viral particles.

### Example 17: Capture of extracellular vesicles of COVID-19 patients using a Siglec-1 mini-protein (mS1) bead platform

When analyzing the human proteome of EVs isolated by mS1-capture, we did not observe statistically significant differences in the number of total proteins and EV markers identified in severe COVID-19 patients versus non-COVID-19 individuals (Fig. 36A and 36B). However, when we compared the abundance of the proteins detected in both groups, we found 46 differentially expressed and 13 unique proteins present in severe patients (Fig. 36C). Similar to what we found in the CD9+-EVs, gene ontology analysis of the differentially expressed protein in mS1-captured EVs show the enrichment of extracellular exosome terms in the category of cellular components (Fig. 36D). Importantly, 13 out of the 46 differentially enriched proteins in mS1-captured EVs were also detected in CD9+-EVs including SERPINA3, IFITM3, HLA-A and HLA-B. Of note, we observed that a subset of 171 proteins including 26 classical EV markers were detected in negative controls where mS1-latex beads were incubated with PBS indicating a background saturation of mS1 recombinant protein with HEK293T cells secreted EVs in the mS1 production step.

## Claims

1. An antibody or an antigen-binding fragment thereof that specifically binds to sialic acid-binding Ig-like lectin 1 (CD169/Siglec-1), wherein the antibody comprises one VH region and one VL region and wherein the antibody is selected from the group consisting of:
1) the 1F5 antibody **characterized in that**:
(i) the CDR1 of the VH region comprises the sequence of SEQ ID NO:1 or functionally equivalent variant thereof, the CDR2 of the VH region comprises the sequence of SEQ ID NO:2 or a functionally equivalent variant thereof and the CDR3 of the VH region comprises the sequence of SEQ ID NO: 3 or 4 or a functionally equivalent variant thereof, and
(ii) the CDR1 of the VL region comprises the sequence of SEQ ID NO:5 or functionally equivalent variant thereof, the CDR2 of the VL region comprises the sequence XAS, wherein X Is Trp of Phe, or a functionally equivalent variant thereof and the CDR3 of the VL region comprises the sequence of SEQ ID NO: 8 or functionally equivalent variant thereof or a functionally equivalent variant thereof; and
2) the 3F1 antibody **characterized in that**:
(i) the CDR1 of the VH region comprises the sequence of SEQ ID NO:40 or functionally equivalent variant thereof, the CDR2 of the VH region comprises the sequence of SEQ ID NO:41, the sequence of SEQ ID NO:42, the sequence of SEQ ID NO:43 or a functionally equivalent variant thereof and the CDR3 of the VH region comprises the sequence of SEQ ID NO: 44, the sequence of SEQ ID NO:45 or functionally equivalent variants thereof, and
(ii) the CDR1 of the VL region comprises the sequence of SEQ ID NO:46 or a functionally equivalent variant thereof, the CDR2 of the VL region comprises the sequence XAS, wherein X Is Trp of Phe or a functionally equivalent variant thereof and the CDR3 of the VL region comprises the sequence of SEQ ID NO: 50 or functionally equivalent variants thereof, and
3) the 5B10 antibody comprising
(i) the CDR1 of the VH region comprises the sequence of SEQ ID NO:88 or functionally equivalent variant thereof, the CDR2 of the VH region comprises the sequence of SEQ ID NO:89, and the CDR3 of the VH region comprises the sequence of SEQ ID NO: 90 or functionally equivalent variants thereof, and
(ii) the CDR1 of the VL region comprises the sequence of SEQ ID NO:91, or a functionally equivalent variant thereof, the CDR2 of the VL region comprises the sequence FAS or a functionally equivalent variant thereof and the CDR3 of the VL region comprises the sequence of SEQ ID NO: 93 or a functionally equivalent variant thereof,
4) the 6G5 antibody comprising
(i) the CDR1 of the VH region comprises the sequence of SEQ ID NO:116 or a functionally equivalent variant thereof, the CDR2 of the VH region comprises the sequence of SEQ ID NO:117 or a functionally equivalent variant thereof and the CDR3 of the VH region comprises the sequence of SEQ ID NO: 118 or a functionally equivalent variant thereof, and
(ii) the CDR1 of the VL region comprises the sequence of SEQ ID NO:123, or a functionally equivalent variant thereof, the CDR2 of the VL region comprises the sequence of SEQ ID NO: 124 or a functionally equivalent variant thereof and the CDR3 of the VL region comprises the sequence of SEQ ID NO: 125 or a functionally equivalent variant thereof and
5) the 4E8 antibody is further **characterized in that**:
(i) the CDR1 of the VH region comprises the sequence of SEQ ID NO:133 or a functionally equivalent variant thereof, the CDR2 of the VH region comprises the sequence of SEQ ID NO:134 or a functionally equivalent variant thereof and the CDR3 of the VH region comprises the sequence of SEQ ID NO: 135 or a functionally equivalent variant thereof, and
(ii) the CDR1 of the VL region comprises the sequence of SEQ ID NO:140, or a functionally equivalent variant thereof, the CDR2 of the VL region comprises the sequence of SEQ ID NO: 141 or a functionally equivalent variant thereof and the CDR3 of the VL region comprises the sequence of SEQ ID NO: 142 or a functionally equivalent variant thereof.

2. The antibody or an antigen-binding fragment thereof according to claim 1, wherein:
1) the 1F5 antibody is further **characterized in that**:
(i) the FR1 region of the VH region comprises the sequence of SEQ ID NO:9 or functionally equivalent variant thereof, the FR2 region of the VH region comprises the sequence of SEQ ID NO:10 or functionally equivalent variant thereof, the FR3 region of the VH region comprises the sequence of SEQ ID NO:11 or functionally equivalent variant thereof and the FR4 of the VH region comprises the sequence of SEQ ID NO: 12 or functionally equivalent variants thereof, and
(ii) the FR1 region of the VL region comprises the sequence of SEQ ID NO:18 or functionally equivalent variant thereof, the FR2 region of the VH region comprises the sequence of SEQ ID NO:19 or functionally equivalent variant thereof, the FR3 region of the VH region comprises the sequence of SEQ ID NO:20 or functionally equivalent variant thereof and the FR4 of the VH region comprises the sequence of SEQ ID NO: 21 or functionally equivalent variants thereof; and
2) the 3F1 antibody is further **characterized in that**:
(i) the FR1 region of the VH region comprises the sequence of SEQ ID NO:51 or functionally equivalent variant thereof, the FR2 region of the VH region comprises the sequence of SEQ ID NO:52 or functionally equivalent variant thereof, the FR3 region of the VH region comprises the sequence of SEQ ID NO:53 or functionally equivalent variant thereof and the FR4 of the VH region comprises the sequence of SEQ ID NO: 54 or a functionally equivalent variants thereof, and
(ii) the FR1 region of the VL region comprises the sequence of SEQ ID NO:60 or functionally equivalent variant thereof, the FR2 region of the VH region comprises the sequence of SEQ ID NO:61 or functionally equivalent variant thereof, the FR3 region of the VH region comprises the sequence of SEQ ID NO:62 or functionally equivalent variant thereof and the FR4 of the VH region comprises the sequence of SEQ ID NO: 63 or functionally equivalent variants thereof; and
3) the 5B10 antibody is further **characterized in that**:
(i) the FR1 region of the VH region comprises the sequence of SEQ ID NO:94 or functionally equivalent variant thereof, the FR2 region of the VH region comprises the sequence of SEQ ID NO:95 or functionally equivalent variant thereof, the FR3 region of the VH region comprises the sequence of SEQ ID NO:96 or functionally equivalent variant thereof and the FR4 of the VH region comprises the sequence of SEQ ID NO: 97 or functionally equivalent variants thereof, and
(ii) the FR1 region of the VL region comprises the sequence of SEQ ID NO:102 or functionally equivalent variant thereof, the FR2 region of the VH region comprises the sequence of SEQ ID NO:103 or functionally equivalent variant thereof, the FR3 region of the VH region comprises the sequence of SEQ ID NO:1 or functionally equivalent variant thereof and the FR4 of the VH region comprises the sequence of and SEQ ID NO: 105 or functionally equivalent variants thereof,
4) the 6G5 antibody is further **characterized in that**:
(i) the FR1 region of the VH region comprises the sequence of SEQ ID NO:119 or functionally equivalent variant thereof, the FR2 region of the VH region comprises the sequence of SEQ ID NO:120 or functionally equivalent variant thereof, the FR3 region of the VH region comprises the sequence of SEQ ID NO:121 or functionally equivalent variant thereof and the FR4 of the VH region comprises the sequence of SEQ ID NO: 122 or a functionally equivalent variants thereof, and
(ii) the FR1 region of the VL region comprises the sequence of SEQ ID NO:126 or functionally equivalent variant thereof, the FR2 region of the VH region comprises the sequence of SEQ ID NO:127 or functionally equivalent variant thereof, the FR3 region of the VH region comprises the sequence of SEQ ID NO:128 or functionally equivalent variant thereof and the FR4 of the VH region comprises the sequence of and SEQ ID NO: 129 or a functionally equivalent variant thereof or
5) the 4E8 antibody is further **characterized in that**:
(i) the FR1 region of the VH region comprises the sequence of SEQ ID NO:136 or functionally equivalent variant thereof, the FR2 region of the VH region comprises the sequence of SEQ ID NO:137 or functionally equivalent variant thereof, the FR3 region of the VH region comprises the sequence of SEQ ID NO:138 or functionally equivalent variant thereof and the FR4 of the VH region comprises the sequence of SEQ ID NO: 139 or a functionally equivalent variants thereof, and
(ii) the FR1 region of the VL region comprises the sequence of SEQ ID NO:143 or functionally equivalent variant thereof, the FR2 region of the VH region comprises the sequence of SEQ ID NO:144 or functionally equivalent variant thereof, the FR3 region of the VH region comprises the sequence of SEQ ID NO:145 or functionally equivalent variant thereof and the FR4 of the VH region comprises the sequence of and SEQ ID NO: 146 or a functionally equivalent variant thereof.

3. The antibody or an antigen-binding fragment thereof according to claim 1 wherein the antibody of the antibody fragment is humanized.

4. The antibody or an antigen-binding fragment thereof according to claim 3, wherein:
1) the 1F5 antibody is further **characterized in that**:
(i) the FR1 region of the VH region comprises the sequence of SEQ ID NO:13 or functionally equivalent variant thereof, the FR2 region of the VH region comprises the sequence of SEQ ID NO:14 or functionally equivalent variant thereof, the FR3 region of the VH region comprises the sequence of SEQ ID NO:15, the sequence of SEQ ID NO:16 or functionally equivalent variant thereof and the FR4 of the VH region comprises the sequence of SEQ ID NO: 17 or a functionally equivalent variant thereof, and
(ii) the FR1 region of the VL region comprises the sequence of SEQ ID NO:22 or functionally equivalent variant thereof, the FR2 region of the VH region comprises the sequence of SEQ ID NO:23 or functionally equivalent variant thereof, the FR3 region of the VH region comprises the sequence of SEQ ID NO:24 or functionally equivalent variant thereof and the FR4 of the VH region comprises the sequence of SEQ ID NO: 25 or functionally equivalent variants thereof; and
2) the 3F1 antibody is further **characterized in that**:
(i) the FR1 region of the VH region comprises the sequence of SEQ ID NO:55 or functionally equivalent variant thereof, the FR2 region of the VH region comprises the sequence of SEQ ID NO:56 or functionally equivalent variant thereof, the FR3 region of the VH region comprises the sequence of SEQ ID NO:57, the sequence of SEQ ID NO:58 or a functionally equivalent variant thereof and the FR4 of the VH region comprises the sequence of SEQ ID NO: 59 or a functionally equivalent variant thereof, and
(ii) the FR1 region of the VL region comprises the sequence of SEQ ID NO:64 or functionally equivalent variant thereof, the FR2 region of the VH region comprises the sequence of SEQ ID NO:65 or functionally equivalent variant thereof, the FR3 region of the VH region comprises the sequence of SEQ ID NO:66 or functionally equivalent variant thereof and the FR4 of the VH region comprises the sequence of SEQ ID NO: 67 or a functionally equivalent variant thereof; and
3) the 5B10 antibody is further **characterized in that**:
(i) the FR1 region of the VH region comprises the sequence of SEQ ID NO:98 or functionally equivalent variant thereof, the FR2 region of the VH region comprises the sequence of SEQ ID NO:99 or functionally equivalent variant thereof, the FR3 region of the VH region comprises the sequence of SEQ ID NO:100 or functionally equivalent variant thereof and the FR4 of the VH region comprises the sequence of SEQ ID NO: 101 or a functionally equivalent variant thereof, and
(ii) the FR1 region of the VL region comprises the sequence of SEQ ID NO:106 or functionally equivalent variant thereof, the FR2 region of the VH region comprises the sequence of SEQ ID NO:107 or functionally equivalent variant thereof, the FR3 region of the VH region comprises the sequence of SEQ ID NO:108 or functionally equivalent variant thereof and the FR4 of the VH region comprises the sequence of SEQ ID NO: 109 or a functionally equivalent variant thereof.

5. The antibody or an antigen-binding fragment thereof according to any preceding claim wherein:
1) the 1F5 antibody comprises a VH region as defined in SEQ ID NO:31, 26, 27 or 28 or a VL region as defined in SEQ ID NO:29 or 30,
2) the 13F1 antibody comprises a VH region as defined in SEQ ID NO:86, 68, 69 or 70 or a VL region as defined in SEQ ID NO:87, 77, 78, 80, 84 or 85,
3) the 5B10 antibody comprises a VH region as defined in SEQ ID NO:112 or 110 or a a VL region as defined in SEQ ID NO:113 or 111
4) the 6G5 antibody comprises a VH region as defined in SEQ ID NO:130 or a VL region as defined in SEQ ID NO:131 or
5) the 4E8 antibody comprises a VH region as defined in SEQ ID NO:147 or a VL region as defined in SEQ ID NO:148.

6. The antibody or an antigen-binding fragment thereof according to any preceding claim wherein:
1) the 1F5 antibody is selected from the group consisting of:
(i) the antibody in which the VH region comprises the sequence of SEQ ID NO:26 and the VL region comprises the sequence of SEQ ID NO:29,
(ii) the antibody in which the VH region comprises the sequence of SEQ ID NO:31 and the VL region comprises the sequence of SEQ ID NO:32,
(iii) the antibody in which the VH region comprises the sequence of SEQ ID NO:27 and the VL region comprises the sequence of SEQ ID NO:30,
(iv) the antibody in which the VH region comprises the sequence of SEQ ID NO:28 and the VL region comprises the sequence of SEQ ID NO:30,
(v) the antibody in which the VH region comprises the sequence of SEQ ID NO:27 and the VL region comprises the sequence of SEQ ID NO:29
(vi) the antibody in which the VH region comprises the sequence of SEQ ID NO:28 and the VL region comprises the sequence of SEQ ID NO:29
(vii) the antibody in which the VH region comprises the sequence of SEQ ID NO:26 and the VL region comprises the sequence of SEQ ID NO:30,
2) the 3F1 antibody is selected from the group consisting of:
(i) the antibody in which the VH region comprises the sequence of SEQ ID NO:68 and the VL region comprises the sequence of SEQ ID NO:77
(ii) the antibody in which the VH region comprises the sequence of SEQ ID NO:86 and the VL region comprises the sequence of SEQ ID NO:87
(iii) the antibody in which the VH region comprises the sequence of SEQ ID NO:69 and the VL region comprises the sequence of SEQ ID NO:78
(iv) the antibody in which the VH region comprises the sequence of SEQ ID NO:70 and the VL region comprises the sequence of SEQ ID NO:78
(v) the antibody in which the VH region comprises the sequence of SEQ ID NO:69 and the VL region comprises the sequence of SEQ ID NO:80
(vi) the antibody in which the VH region comprises the sequence of SEQ ID NO:70 and the VL region comprises the sequence of SEQ ID NO:80
(vii) the antibody in which the VH region comprises the sequence of SEQ ID NO:69 and the VL region comprises the sequence of SEQ ID NO:77
(viii) the antibody in which the VH region comprises the sequence of SEQ ID NO:70 and the VL region comprises the sequence of SEQ ID NO:77
(ix) the antibody in which the VH region comprises the sequence of SEQ ID NO:68 and the VL region comprises the sequence of SEQ ID NO:84
(x) the antibody in which the VH region comprises the sequence of SEQ ID NO:68 and the VL region comprises the sequence of SEQ ID NO:85 or
3) the 5B10 antibody is selected from the group consisting of:
(i) the antibody wherein the VH region comprises the sequence SEQ ID NO:112 and the VL region comprises the sequence of SEQ ID NO: 113 or
(ii) the antibody wherein the VH region comprises the sequence SEQ ID NO: 110 or a VL region as defined in SEQ ID NO:113 or 111
4) the 6G5 antibody comprises a VH region as defined in SEQ ID NO:130 and a VL region as defined in SEQ ID NO:131 or
5) the 4E8 antibody comprises a VH region as defined in SEQ IDN O:147 and a VL region as defined in SEQ ID NO:148.

7. The antibody or an antigen-binding fragment thereof according to any of claims 1 to 6, for use in the prevention of an infection by an enveloped virus.

8. The antibody or antigen-binding fragment thereof for use according to claim 7 wherein the enveloped virus is a lentivirus, a virus of the filoviridae family, an Orthopneumovirus, a coronavirus or an arenavirus.

9. The antibody or a ntigen-binding fragment thereof host cell for use according to claim 9 wherein the lentivirus is the human immunodeficiency virus (HIV-1), the virus of the filoviridae family is the Ebola virus (EBOV), the orthopneumovirus is the Respiratory syncytial virus (RSV) or the coronavirus is SARS-CoV-2.

10. A conjugate comprising:
- an antibody or antigen-binding domain as defined in any of claims 1 to 6 and
- an agent of interest,
wherein the agent of interest is covalently coupled to the antibody or to the antigen-binding fragment thereof.

11. A conjugate according to claim 10 wherein the agent of interest is a compound with therapeutic activity and wherein the therapeutic compound is a cytotoxic compound for use in a method of treatment of a disease which is associated with increased dendritic cell activity.

12. An in vitro method for the capture of particles containing sialic acid present in a sample which comprises:
(i) contacting the sample with a Siglec-1 protein variant that comprises the Siglec-1 protein Ig-like V-set domain under conditions adequate for the binding of the particles containing sialic acid to the Siglec-1 protein variant and
(ii) recovering the Siglec-1 protein variants from the sample.

13. The method according to claim 12 wherein the Siglec-1 protein variant further comprises at least one Ig-like C2 set domains.

14. The method according to claim 13 wherein the Siglec-1 protein comprises at least three Ig-like C2 set domains.

15. The method according to claim 14 wherein the three Ig-like C2 set domains are the three Ig-like C2 set domains 1, 2 and 3.
